(19) Europäisches Patentamt
European Patent Office
Office européen des brevets

(11) EP 1 683 862 B1

(12) **EUROPEAN PATENT SPECIFICATION**

(45) Date of publication and mention of the grant of the patent:
**01.08.2012 Bulletin 2012/31**

(21) Application number: **04773529.5**

(22) Date of filing: **24.09.2004**

(51) Int Cl.:
*C12Q 1/68* (2006.01)

(86) International application number:
**PCT/JP2004/014441**

(87) International publication number:
**WO 2005/030959 (07.04.2005 Gazette 2005/14)**

(54) **MICROARRAY FOR ASSESSING NEUROBLASTOMA PROGNOSIS AND METHOD OF ASSESSING NEUROBLASTOMA PROGNOSIS**

MIKROARRAY ZUR BEURTEILUNG DER NEUROBLASTOMPROGNOSE UND VERFAHREN ZUR BEURTEILUNG DER NEUROBLASTOMPROGNOSE

MICRORESEAU D'EVALUATION DE PRONOSTIC NEUROBLASTOME ET PROCEDE D'EVALUATION DE PRONOSTIC DE NEUROBLASTOME

(84) Designated Contracting States:
**DE FR GB**

(30) Priority: **25.09.2003 US 505614 P**

(43) Date of publication of application:
**26.07.2006 Bulletin 2006/30**

(73) Proprietors:
- **Chiba-Prefecture**
  **Chiba-shi,**
  **Chiba 260-8667 (JP)**
- **HISAMITSU PHARMACEUTICAL CO., INC.**
  **Saga 841-0017 (JP)**

(72) Inventors:
- **NAKAGAWARA, Akira**
  **Chiba-shi, Chiba 260-8717 (JP)**
- **OHIRA, Miki**
  **Chiba-shi, Chiba 260-8717 (JP)**
- **ISHII, Shin**
  **Ikoma-shi, Nara 630-0101 (JP)**
- **GOTO, Takeshi**
  **Chiyoda-ku,**
  **Tokyo 100-6221 (JP)**
- **KUBO, Hiroyuki**
  **Chiyoda-ku, Tokyo 100-6221 (JP)**
- **HIRATA, Takahiro**
  **Chiyoda-ku, Tokyo 100-6221 (JP)**
- **YOSHIDA, Yasuko**
  **Nagoya-shi, Aichi 467-8530 (JP)**
- **YAMADA, Saichi**
  **Nagoya-shi, Aichi 467-8530 (JP)**

(74) Representative: **Paget, Hugh Charles Edward et al**
**Mewburn Ellis LLP**
**33 Gutter Lane**
**London**
**EC2V 8AS (GB)**

(56) References cited:
**FR-A1- 2 860 518**

- **YAMANAKA Y ET AL: "Maturational sequence of neuroblastoma revealed by molecular analysis of cDNA microarrays" INTERNATIONAL JOURNAL OF ONCOLOGY, EDITORIAL ACADEMY OF THE INTERNATIONAL JOURNAL OF ONCOLOGY,, GR, vol. 21, no. 4, October 2002 (2002-10), pages 803-807, XP008032825 ISSN: 1019-6439**
- **BERWANGER B ET AL.: "Loss of a FYN-regulated differentiation and growth arrest pathway in advanced stage neuroblastoma." CANCER CELL, vol. 2, no. 5, November 2002 (2002-11), pages 377-386, XP002423895 United States**
- **OHIRA M. ET AL.: 'Expression profiling and characterization of 4200 genes cloned from primary neuroblastomas: identification of 305 genes differentially expressed between favorable and unfavorable subsets' ONCOGENE vol. 22, August 2003, pages 5525 - 5536, XP002984256**
- **VAN'T VEER L.J. ET AL.: 'Gene expression profiling predicts clinical outcome of breast cancer' NATURE vol. 415, 2002, pages 530 - 536, XP002259781**
- **SINGH D. ET AL.: 'Gene expression of clinical prostate cancer behaviour' CANCER CELL vol. 1, 2002, pages 203 - 209, XP002984257**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 683 862 B1

## Description

### Technical Field

The invention of this application relates to a microarray for predicting the prognosis of neuroblastoma. More particularly, the invention of this application relates to a microarray for performing a molecular biological diagnosis of whether the prognosis of a patient with neuroblastoma after medical treatment is good or poor, and a method for predicting the prognosis of neuroblastoma using this microarray.

### Background Art

Neuroblastoma is one of the most common solid tumors in children and is originated from the sympathoadrenal lineag133e of the neural crest (Bolande, 1974: non-patent document 1). Its clinical behavior is heterogeneous: the tumors found in infants frequently regress spontaneously by inducing differentiation and/or programmed cell death, while those occurred in the patients over one year of age are often aggressive and acquire the resistance to intensive chemotherapy. Though the recent progress in the therapeutic strategies against advanced stages of neuroblastomas has improved the survival rate, the long-term results are still very poor. In addition, some of the tumors categorized to the intermediate group (in stage 3 or 4, and possessing a single copy of the *MYCN* gene) often recur after a complete response to the initial therapy. It is conceivable that such differences in the final outcome among the tumors maybe due to the differences in genetic and biological abnormalities which are reflected to the expression profile of genes and proteins in the tumor.

The prediction of the prognosis is one of the most emergent demands for starting the treatment of neuroblastoma. A patient's age (over or under one year of age), as expected from the natural history of neuroblastoma, is an important factor to segregate the outcome into favorable and unfavorable groups (Evans et al., 1971: non-patent document 2). The disease stage is also a powerful indicator of prognosis (Brodeur et al., 1993: non-patent document 3). Moreover, recent advances in basic research have found more than several molecular markers which are useful in the clinic. They include amplification of *MYCN* oncogene (Schwab et al., 1983: non-patent document 4; Brodeur et al., 1984: non-patent document 5), DNA ploidy (Look et al., 1984, 1991: non-patent document 6, 7), deletion of chromosome 1p (Brodeur et al., 1988: non-patent document 8) and TrκA expression (Nakagawara et al., 1992, 1993: non-patent document 9, 10), some of which are already used as prognostic indicators to choose the therapeutic strategy at the bedside. The other indicators also include telomerase (Hiyama et al., 1995: non-patent document 11), CD44 (Favrot et al., 1993: non-patent document 12), pleiotrophin (Nakagawara et al., 1995: non-patent document 13), N-cadherin (Shimono et al., 2000: non-patent document 14), CDC10 (Nagata et al., 2000: non-patent document15), and Fyn (Berwanger et al., 2002: non-patent document 16). However, even their combination often fails to predict the patients' outcome. Therefore, new diagnostic tools in the postgenomic era have been expected to become available. Recently, DNA microarray method has been applied to comprehensively demonstrate expression profiles of primary neuroblastomas as well as cell lines. It has already identified several genes differentially expressed between favorable and unfavorable subsets (Yamanaka et al., 2002: non-patent document 17; Berwanger et al., 2002: non-patent document 16) or the genes changed during retinoic acid-induced neuronal differentiation (Ueda, 2001: non-patent document 18). However, the study to predict the prognosis by microarray using a large number of neuroblastoma samples has never been reported.

The present inventors have recently isolated 5,500 independent genes from the cDNA libraries generated from the primary neuroblastomas, a part of which has been previously reported (Ohira et al., 2003a, 2003b: non-patent document 19, 20). Further the present inventors have files patent applications relating to full disclosure of the isolated genes, and a relationship between the outcome predictability of neuroblastoma and the genes' expressions (patent documents 1-5)

Patent documents

1 : JP 2001-245671A
2 : JP 2001-321175A
3 : PCT/JPO163 pamphlet
4 : PCT/JPO1629 pamphlet
5 : JP2004-147563A

Non-patent documents

1 : Bolande, R. P. Hum Pathol 5, 409 - 429 (1974).
2 : Evans, A. E. et al. Cancer 27, 374 - 8 (1971).
3 : Brodeur, G. M. et al. J Clin Oncol 11, 1466-77 (1993).
4 : Schwab, M. et al. Nature 305, 245 - 8 (1983).
5 : Brodeur, G. M. et al. Science 224, 1121 - 4 (1984).
6 : Look, A. T. et al. N Engl J Med 311, 231 - 5 (1984).
7 : Look, A. T. et al. J Clin Oncol 9, 581 - 91 (1991).
8 : Brodeur, G. M. et al. Prog Clin Biol Res 271, 3 - 15 (1988).
9 : Nakagawara, A. et al. Cancer Res 52, 1364 - 8 (1992).
10 : Nakagawara, A. et al. N Engl J Med 328, 847 - 54 (1993).
11 : Hiyama, E. et al. Nat Med 1, 249 - 55 (1995).
12 : Favrot, M. C. et al. N Engl J Med 329 (1993).
13 : Nakagawara, A. et al. Cancer Res 55, 1792 - 7 (1995).
14 : Shimono, R. et al. Anticancer Res 20, 917 - 23 (2000).
15 : Nagata, T. et al. J Surg Res 92, 267 - 75 (2000).
16 : Berwanger, B. et al. Cancer Cell 2, 377 - 86 (2002).
17 : Yamanaka, Y. et al. Int Oncol 21, 803 - 7 (2002).
18 : Ueda, K. Kurume Med J 48, 159 - 64 (2001).
19 : Ohira, M. et al. Oncogene 22, 5526 - 36 (2003a).
20 : Ohira, M. et al. Cancer Lett 197, 63 - 8 (2003b).

**Disclosure of Invention**

It is extremely important for selecting a better medical treatment method for a patient to accurately predict whether the prognosis after medical treatment of neuroblastoma is good or poor. So far, several molecular markers which are capable of performing such a prediction have been identified. However, even if such molecular markers were used alone or in combination, the prediction of diagnosis of neuroblastoma was not always accurate.

The invention of this application has been carried out in view of the circumstances as above, and makes it an object to provide a novel method capable of accurate and convenient prediction of the prognosis of neuroblastoma.

This application provides the following inventions in order to solve the foregoing problems.

A first invention is a microarray having 24 probes related to good prognosis which respectively hybridize to the gene transcripts of genes in Group F of Table 2 (page 35), and 30 probes related to poor prognosis which respectively hybridize to gene transcripts of genes in Group UF in said Table 2. The probes are defined in the claims.

A second invention is a method for predicting prognosis of neuroblastoma using the microarray according to claim 1, wherein the method comprises:

(a) a step of labeling a gene transcript obtained from a tumor cell of a patient diagnosed as having neuroblastoma;
(b) a step of bringing the labeled gene transcript into contact with the microarray according to claim 1;
(c) a step of measuring the labeling signal of each of the gene transcripts hybridized to 24 or more of the probes related to good prognosis and 25 or more of the probes related to poor prognosis on the microarray, respectively, and determines that the prognosis of the patient is good if significant labeling signals for 24 or more of the probes related to good prognosis were obtained. and that the prognosis of the patient is poor if significant labeling signals for 25 or more of the probes related to poor prognosis were obtained.

In other words, the inventors of this application used a microarray capable of analyzing the expression of 5,340 genes specific to neuroblastoma (non-patent documents 19, 20, and patent document 1), and analyzed the expression of the 5,340 genes using mRNAs isolated from 136 patients with neuroblastoma as a target. In addition, the inventors constructed a kernel-based probabilistic classification model and found out that the probabilistic output thereof defines the molecular signature of neuroblastoma for prediction of the prognosis and that the analysis of the expression level of specific genes is superior to a conventional method using a known molecular marker as a target in terms of the prediction of the prognosis, thus this invention has been worked out.

Specifically, the microarrays and methods described herein predict good and poor prognosis of neuroblastoma using 200 genes shown in Table 1 as a target. In Table 1, No.1 to 200 in the first row correspond to Seq. ID No. 1 to 200 of the sequence table, and measurement value with a control sequence and with water are shown respectively in No. 201 to 212 (the numerical values in the sixth to ninth rows, which will be explained later). With respect to Seq. ID No. 140, the nucleotide sequence from 1 to 977 is the 5' sequence of the gene named Nbla12151 and the nucleotide sequence from 983 to 1869 is the 3' sequence thereof.

Table 1

| Seq. ID No. | Gene Name on Spot | Acc.No.(known genes UCSC etc) | UCSC Homology | UCSC Mapping | rankin g6/2 | pairwise F-value | pairwise F-value | logrank p-value | |
|---|---|---|---|---|---|---|---|---|---|
| 1 | gene022 | NM_002051 | GATA3 | 10p14 | 253 | 0.580 | F | 0.001971 | |
| 2 | gene052-1 | NM_005378 | MYCN | 2p24.3 | 20 | 0.784 | UF | 0.001253 | |
| 3 | gene053-1 | NM_005378 | MYCN | : 2p24.3 | 46 | 0.750 | UF | 0.00133 | |
| 4 | gene056 | NM_000546 | TP53 | 17p13.1 | 66 | 0.721 | UF | 0.004087 | |
| 5 | gene071 | NM_000360 | TH | 11p15.5 | 60 | 0.723 | F | 0.000787 | |
| 6 | gene073 | NM_002529 | NTRK1 | 1q23.1 | 118 | 0.667 | F | 0.000002 | |
| 7 | Nbla00013 | NM_006098 | GNB2L1 | 5q35.3 | 25 | 0.772 | UF | 0.000006 | |
| 8 | Nbla00083 | BC010577 | GRN | 17q21.3 | 131 | 0.657 | UF | 0.147089 | o |
| 9 | Nbla00127 | U26710 | CBLB | 3q13.11 | 315 | 0.553 | UF | 0.001669 | |
| 10 | Nbla00138 | D83779 | KIAA0195 | 17q25.1 | 339 | 0.535 | UF | 0.052854 | o |
| 11 | Nbla00139 | BC006772 | RPS13 | 11p15.1 | 153 | 0.646 | UF | 0.000912 | o |
| 12 | Nbla00202 | NM_014347 | ZF5128 | 19q13.4 | 254 | 0.579 | UF | 0.020624 | |
| 13 | Nbla00214 | BC007512 | RPL18A | 19p13.1 | 31 | 0.762 | UF | 0.000002 | o |
| 14 | Nbla00217 | S72871 | GATA2 | 3q21.3 | 95 | 0.678 : | F | 0.010245 | |
| 15 | Nbla00259 | NM_001010 | RPS6 | 9p22.1 | 163 | 0.638 | UF | 0.001115 | |
| 16 | Nbla00260 | NM_006082 | K-ALPHA-1 | 12q13.1 | 1 | 0.873 | F | 0.000003 | |
| 17 | Nbla00269 | NM_000787 | DBH | 9q34.2 | 57 | 0.724 | F | 0.00362 | |
| 18 | Nbla00332 | NM_001404 | EEF1G | 11q12.3 | 5 | 0.836 | UF | 0.000055 | |
| 19 | Nbla00347 | X59798 | CCND1 | 11q13.3 | 235 | 0.592 | F | 0.001629 | |
| 20 | Nbla00359 | AF083811 | MAD1L1 | 7p22.3 | 69 | 0.708 | UF | 0.00112 | |
| 21 | Nbla00383 | NM_001023 | RPS20 | i 8q12.1 | 359 | 0.519 | UF | 0.056573 | |
| 22 | Nbla00391 | T09492 | AF036613 | 7q11.23 | 102 | 0.676 | F | 0.000539 | |
| 23 | Nbla00487 | NM_024909 | FLJ13158 | 6p21.33 | 47 | 0.745 | F | 0.002751 | |

(continued)

| Seq. ID No. | Gene Name on Spot | Acc.No.(known genes UCSC etc) | UCSC Homology | UCSC Mapping | rankin g6/2 | pairwise F-value | pairwise F-value | logrank p-value | |
|---|---|---|---|---|---|---|---|---|---|
| 24 | Nbla00488 | AK055378 | AK055378 | 17q21.1 | 165 | 0.636 | F | 0.00289 | |
| 25 | Nbla00501 | NM_000969 | HPL5, corresponding to intron | 1p22.1 | 15 | 0.786 | UF | 0.005786 | |
| 26 | Nbla00503 | NM_004793 | PRSS15, corresponding to intron | 19p13.3 | 91 | 0.679 | UF | 0.000169 | |
| 27 | Nbla00576 | BC016346 | FTL | 19q13.3 | 323 | 0.545 | UF | 0.215576 | o |
| 28 | Nbla00578 . | NM_006818 | AF1Q | 1q21.3 | 79 | 0.690 | F | 0.009397 | |
| 29 | Nbla00610 | U03105 | PROL2 | 6q15 | 203 | 0.609 | F | 0.033502 | |
| 30 | Nbla00696 | X04098 | ACTG1 | 17q25.3 | 199 | 0.611 | UF | 0.10486 | |
| 31 | Nbla00715 | AF131776 | AF131776 | 7p13 | 273 | 0.575 | F | 0.000342 | |
| 32 | Nbla00754 | M17886 | RPLP1 | 15q23 | 123 | 0.657 | UF | 0.000068 | |
| 33 | Nbla00772 | NM_000681 | ADRA2A | 10q25.2 | 353 | 0.525 | UF | 0.022749 | |
| 34 | Nbla00781 | BC009970 | TKT | 3p21.1 | 26 | 0.772 | UF | 0.048075 | o |
| 35 | Nbla00800 | D84294 | TTC3 | 21q22.1 | 311 | 0.554 | UF | 0.020169 | o |
| 36 | Nbla00824 | NM_003958 | RNF8 | 6p21.2 | 239 | 0.590 | UF | 0.004012 | |
| 37 | Nbla00890 | NM_003899 | ARHGEF7 | 13q34 | 62 | 0.721 | F | 0.000001 | |
| 38 | Nbla00901 | NM_005663 | WHSC2 | 4p16.3 | 83 | 0.689 | UF | 0.090789 | |
| 39 | Nbla02965 | X63432 | ACTB | 7p22.1 | 137 | 0.649 | F | 0.700325 | |
| 40 | Nbla02985 | NM_001386 | DPYSL2 | Bp21.2 | 275 | 0.571 | F | 0.005059 | |
| 41 | Nbla02990 | NM_006597 | HSPA8 | 11q24.1 | 221 | 0.600 | UF | 0.386365 | |
| 42 | Nbla03025 | NM_007103 | NDUFV1 | 11q13.2 | 73 | 0.696 | UF | 0.143343 | |
| 43 | Nbla03135 | BC04574 | BC045747 | 22q13.1 | 295 | 0.567 | F | 0.001318 | |
| 44 | Nbla03145 | NM_004826 | ECEL1 | 2q37.1 | 55 | 0.727 | F | 0.000494 | |

(continued)

| Seq. ID No. | Gene Name on Spot | Acc.No.(known genes UCSC etc) | UCSC Homology | UCSC Mapping | rankin g6/2 | pairwise F-value | pairwise F-value | logrank p-value | |
|---|---|---|---|---|---|---|---|---|---|
| 45 | Nbla03251 | AF078866 | SURF4 | 9q34.2 | 296 | 0.563 | UF | 0.015889 | |
| 46 | Nbla03286 | NM_020198 | GK001, AF226054 | 17q23.3 | 28 | 0.772 | UF | 0.000175 | |
| 47 | Nbla03323 | D78014 | RYSL3 | 5q32 | 140 | 0.648 | F | 0.000019 | |
| 48 | Nbla03342 | XB0199 | MLN51 | 17q21.1 | 212 | 0.603 | F | 0.000093 | |
| 49 | Nbla03401 | NM_064772 | C5orf13 | 5q22.1 | 299 | 0.563 | F | 0.00298 | |
| 50 | Nbla03430 | NM_007029 | STMN2 | 8q21.13 | 213 | 0.600 | F | 0.000276 | |
| 51 | Nbla03499 | NM_002074 | GNB1 | 1p36.33 | 33 | 0.762 | F | 0.000195 | |
| 52 | Nbla03518 | U14394 | TIMP3 | 22q12.3 | 119 | 0.667 | F | 0.000661 | |
| 53 | Nbla03521 | NM_032015 | RNF26 | 11q23.3 | 93 | 0.679 | UF | 0.010481 | |
| 54 | Nbla03533 | AK000237 | VAT1 | 17q21.3 | 182 | 0.629 | F | 0.20487 | |
| 55 | Nbla03534 | NM_005381 | NCL | 2q37.1 | 84 | 0.689 | UF | 0.015632 | |
| 56 | Nbla03604 | NM_001626 | AKT2 | 19q13.2 | 154 | 0.638 | UF | 0.05307 | |
| 57 | Nbla03646 | NM_014762 | DHCR24 | 1p32.3 | 289 | 0.571 | F | 0.010653 | |
| 58 | Nbla03651 | NM_003885 | CDK5R1 | 17q11.2 | 256 | 0.579 | F | 0.000002 | |
| 59 | Nbla03682 | NM_001843 | CNTN1 | 12q12 | 360 | 0.517 | F | 0.002928 | |
| 60 | Nbla03740 | NM_000615 | NCAM1 | 11q23.1 | 215' | 0.600 | F | 0.000002 | |
| 61 | Nbla03750 | L22557 | MGC8407 | 3p21.31 | 222 | 0.597 | UF | 0.256038 | |
| 62 | Nbla03755 NM_ | 005910 | MAPT | 17q21.3 | 208 | 0.605 | F | 0.000413 | |
| 63 | Nbla03761 | NM_014213 | HOXD9 | 2q31.1 | 330 | 0.543 | UF | 0.015653 | |
| 64 | Nbla03767 | AKU25927 | MGC8721 | 8p12 | 75 | 0.694 | F | 0.000011 | |
| 65 | Nbla03819 NM_ | 000240 | MAOA | Xp11.3 | 257 | 0.579 | F | 0.001533 | |
| 66 | Nbla03836 | NM_000972 | RPL7A | 9q34.2 | 98 | 0.677 | UF | 0.048031 | |

(continued)

| Seq. ID No. | Gene Name on Spot | Acc.No.(known genes UCSC etc) | UCSC Homology | UCSC Mapping | rankin g6/2 | pairwise F-value | pairwise F-value | logrank p-value | |
|---|---|---|---|---|---|---|---|---|---|
| 67 | Nbla03873 | NM 006054 | RTN3 | 11q13.1 | 58 | 0.724 | F | 0.00001 | |
| 68 | Nbla03896 | BC022509 | SCG2 | 2q36.1 | 306 | 0.557 | F | 0.001898 | o |
| 69 | Nbla03899 | NM 001641 | APEX1 | 14q11.2 | 201 | 0.609 | UF | 0.02278 | |
| 70 | Nbla03925 | BC015654 | LAMR1 | 3p22.2 | 63 | 0.721 | UF | 0.001773 | o |
| 71 | Nbla03938 | NM 002948 | RPL15 | 3p24.2 | 244 | 0.588 | UF | 0.136289 | |
| 72 | Nbla03949 | BC011520 | STMN4 | 8p21.2 | 265 | 0.576 | F | 0.001411 | o |
| 73 | Nbla03954 | NM 000610 | CD44 | 11p13 | 141 | 0.647 | F | 0.000045 | |
| 74 | Nbla03969 | AB058781 | MAP6 | 11q13.5 | 223 | 0.597 | F | 0.000025 | |
| 75 | Nbla04104 | D00099 | ATP1A1 | 1p13.1 | 331 | 0.541 | F | 0.072373 | |
| 76 | Nbla04029 | NM 016091 | EIF3S6IP | 22q13.1 | 248 | 0.583 | UF | 0.05877 | |
| 77 | Nbla04134 | T13156 | MBC2 | 12q13.2 | 107 | 0.667 | F | 0.015693 | |
| 78 | Nbla04181 | AK055112 | AK055112 | 5q13.2 | 183 | 0.627 | F | 0.001425 | |
| 79 | Nbla04200 | BC007748 | RPL4 | 15q22.3 | 81 | 0.690 | UF | 0.04097 | o |
| 80 | Nbla04225 | NM 021814 | HELO1 | 6p12.1 | 258 | 0.579 | F | 0.061412 | |
| 81 | Nbla04269 | NM 006386 | DDX17 | 22q13.1 | 348 | 0.529 | F | 0.006945 | |
| 82 | Nbla04270 | AJ132695 | RAC1 | 7p22.1 | 173 | 0.633 | F | 0.012286 | |
| 83 | Nbla04293 | NM 002654 | PKM2 | 15q23 | 49 | 0.738 | UF | 0.001516 | |
| 84 | Nbla04314 | NM 003347 | UBE2L3 | 22q11.2 | 198 | 0.613 | F | 0.082094 | |
| 85 | Nbla04332 | NM_152344 | FLJ30656 | 17q21.3 | 341 | 0.532 | UF | 0.006093 | |
| 86 | Nbla10054 | NM 002520 | NPM1 | 5q35.1 | 82 | 0.690 | UF | 0.000104 | |
| 87 | Nbla10093 | NM_000183 | HADHB | 2p23.3 | 8 | 0.828 | F | 0.000018 | |
| 88 | Nbla10153 | AB062057 | TM4SF2 | Xp11.4 | 313 | 0.553 | UF | 0.262965 | o |
| 89 | Nbla10203 | NM_015342 | KIAA0073 | 5q12.3 | 147 | 0.647 | F | 0.009215 | |
| 90 | Nbla10275 | NM 002567 | PBP | 12q24.2 | 277. | 0.571 | F | 0.001161 | |

(continued)

| Seq. ID No. | Gene Name on Spot | Acc.No.(known genes UCSC etc) | UCSC Homology | UCSC Mapping | rankin g6/2 | pairwise F-value | pairwise F-value | logrank p-value | |
|---|---|---|---|---|---|---|---|---|---|
| 91 | Nbla10296 | U50733 | DCTN2 | 12q13.3 | 332 | 0.541 | F | 0.002154 | |
| 92 | Nbla10302 | NM 001428 | ENO1 | 1p36.23 | 3 | 0.857 | UF | 0.007702 | |
| 93 | Nbla10313 | NM_002300 | LDHB=3', (I-7q21.11) chimera | 12p12.1 | 109 | 0.667 | UF | 0.12083 | |
| 94 | Nbla10327 | NM 014868 | RNF10 | 12q24.3 | 191 | 0.618 | F | 0.002878 | |
| 95 | Nbla10371 | NM 005370 | MEL | 19p13.1 | 192 | 0.615 | UF | 0.712687 | |
| 96 | Nbla10393 | NM 005412 | SHMT2 | 12q13.3 | 365 | 0.517 | UF | 0.108676 | |
| 97 | Nbla10395 | NM 002593 | PCOLCE | 7q22.1 | 110 | 0.667 | UF | 0.000164 | |
| 98 | Nbla10398 NM | 004713 | SDCCAG1 | 14q21.3 | 142 | 0.647 | UF | 0.012774 | |
| 99 | Nbla10400 | NM 014225 | PPP2R1A | 19q13.4 | 184 | 0.627 | UF | 0.112705 | |
| 100 | Nbla10400 | NM_003611 | OFD1 | Xp22.22 | 337 | 0.537 | F | 0.005758 | |
| 101 | Nbla10472 | NM 006666 | RUVBL2 | 19q13.3 | 158 | 0.638 | UF | 0.018914 | |
| 102 | Nbla10497 | NM_005275 | GNL1 | 6p21.33 | 278 | 0.571 | UF | 6.086044 | |
| 103 | Nbla10516 | BC016867 | TSC22 | 13q14.1 | 351 | 0.526 | F | 0.015244 | o |
| 104 | Nbla10530 | U01038 | PLK | 16p12.2 | 343 | 0.532 | UF | 0.001388 | |
| 105 | Nbla10579 | AB002334 | AF432211 | 2q12.3 | 16 | 0.786 | UF | 0.000962 | |
| 106 | Nbla10671 | NM_003707 | RUVBL1 | 3q21.3 | 100 | 0.676 | UF | 0.052258 | |
| 107 | Nbla1072 | AK055935 | AK05b935 | 17q25.1 | 349 | 0.528 | UF | 0.000198 | |
| 108 | Nbla10765 | NM_001168 | BIRC5 | 17q25.3 | 237 | 0.590 | UF | 0.000426 | |
| 109 | Nbla10788 | X02152 | LDHA | 11p15.1 | 303 | 0.559 | UF | 0.014818 | o |
| 110 | Nbla10836 | AF006043 | PHGDH | 1p12? | 187 | 0.627 | UF | 0.002437 | o |
| 111 | Nbla10849 | NM_002823 | PTMA | 2q37.1 | 290 | 0.567 | UF | 0.022365 | |
| 112 | Nb1a10851 | BC004975 | CCNI | 4q21.1 | 159 | 0.638 | F | 0.009974 | o |

(continued)

| Seq. ID No. | Gene Name on Spot | Acc.No.(known genes UCSC etc) | UCSC Homology | UCSC Mapping | rankin g6/2 | pairwise F-value | pairwise F-value | logrank p-value | |
|---|---|---|---|---|---|---|---|---|---|
| 113 | Nbla10856 | AF026402 | U5-100K | 12q13.1 | 74 | 0.696 | F | 0.074918 | |
| 114 | Nbla10873 | NM_005762 | TRIM28 | 19q13.4 | 48 | 0.745 | UF | 0.004984 | |
| 115 | Nbla10925 | AB082924 | RPL13A | 19q13.3 | 111 | 0.667 | UF | 0.021005 | o |
| 116 | Nbla11013 | NM_000998 | RPL37A | 2q35 | 204 | 0.605 | UF | 0.059121 | |
| 117 | Nbla11084 | AF226604 | SR-BP1 | 9p13.3 | 148 | 0.646 | UF | 0.013851 | o |
| 118 | Nbla11092 | AK021601 | FLJ11539 | 4q34.1 | 307 | 0.554 | F | 0.225491 | |
| 119 | Nbla11148 | BC003655 | RPLPO | 12q24.2 | 14 | 0.800 | UF | 0.000049 | o |
| 120 | Nbla11212 | AK001024 | FLJ10162 | 14q22.1 | 350 | 0.526 | F | 0.000039 | |
| 121 | Nbla11280 | NM_000984 | RPL23A | 17q11.2 | 263 | 0.579 | UF | 0.120135 | |
| 122 | Nbla11337 | NM_004487 | GOLGB1 | 3q21.1- 1-q13.33 | 291 | 0.567 | UF | 0.032809 | |
| 123 | Nbla11400 | NM_001235 | SERPINH1 | 11q13.5 | 314 | 0.553 | UF | 0.125758 | |
| 124 | Nblal11459 | X70649 | DDX1 | 2p24.3 | 6 | 0.836 | UF | 0.000024 | o |
| 125 | Nbla11536 | NM_002394 | SLC3A2 | 11q12.3 | 112 | 0.667 | UF | 0.000897 | |
| 126 | Nbla11561 | NM_005742 | P5 | 2p25.1 | 308 | 0.554 | UF | 0.299715 | |
| 127 | Nbla11584 | J00231 | IGHG3 | 14q32.3 | 346 | 0.532 | UF | 0.151893 | o |
| 128 | Nbla11602 | NM_024034 | GDAP1L1 | 20q13.1 | 169 | 0.635 | UF | 0.357468 | |
| 129 | Nbla11606 | AF141347 | TUBA3 | 12q13.1 | 17 | 0.786 | F | 0 | o |
| 130 | Nbla11662 | NM_006761 | YWHAE | 17p13.3 | 120 | 0.667 | F | 0.00009 | |
| 131 | Nbla11732 | U14966 | RPL5 | 1p22.1 | 76 | 0.694 | UF | 0.001 | o |
| 132 | Nbla11788 | BC032703 | PRPH | 12q13.1 | 18 | 0.786 | F | 0.0000171 | o |
| 133 | Nbla11890 | NM_001402 | EEF1A1 | 6q13 | 89-319 | 0.688 | UF | 0.191622 | |
| 134 | Nbla11919 | BC000502 | APL17 | 18q21.1 | 280 | 0.571 | UF | 0.002429 | o |
| 135 | Nbla11970 | NM_002136 | HNRPA1 | 12q13.1 | 121 | 0.667 | UF | 0.001383 | |

(continued)

| Seq. ID No. | Gene Name on Spot | Acc.No.(known genes UCSC etc) | UCSC Homology | UCSC Mapping | rankin g6/2 | pairwise F-value | pairwise F-value | logrank p-value | |
|---|---|---|---|---|---|---|---|---|---|
| 136 | Nbla11993 | NM_015980 | HMP19 | 5q35.2 | 9 | 0.824 | F | 0.204274 | |
| 137 | Nbla12021 | BC007945 | RPS11 | 19q13.3 | 177 | 0.629 | UF | 0.005294 | o |
| 138 | Nbla12044 | Z48950 | H3F3B | 17q25.1 | 178 | 0.629 | F | 0.019723 | |
| 139 | Nbla12061 | AK055935 | AK055935 | 17q25.1 | 104 | 0.676 | UF | 0.000351 | |
| 140 | Nbla12151 | AU254033 AU254034 | LPIN1 intron, may be not | 2p25.1 | 281 | 0.571 | UF | 0.388543 | o |
| 141 | Nbla12165 | NM_001728 | BSG | 19p13.3 | 210 | 0.603 | UF | 0.015224 | |
| 142 | Nbla20089 | NM_006363 | SEC23B | 20p11.2 | 36 | 0.762 | F | 0.000764 | |
| 143 | Nbla20164 | NM_024827 | HDAC11 | 3p25.1 | 228 | 0.597 | UF | 0.023978 | |
| 144 | Nbla20393 | NM_021136 | RTN1 | 14q23.1 | 282 | 0.571 | F | 0.007075 | |
| 145 | Nbla20490 | AK125587 | AK125587 | 12q13.1 | 114 | 0.667 | F | 0.000013 | |
| 146 | Nbla20509 | NM_003016 | SFRS2 | 17q25.1 | 259 | 0.579 | UF | 0.105982 | |
| 147 | Nbla20562 | NM_001636 | SLC25A6 | Xp22.33 | 149 | 0.646 | UF | 0.001187 | |
| 148 | Nbla20713 | NM 021973 | HAND2? | 4q34.1 | 170 | 0.633 | F | 0.07252 | |
| 149 | Nbla20730 | AK027759 | AK02759 | 6q16.2 | 283 | 0.571 | UF | 0.050407 | |
| 150 | Nbla20771 | NM_002792 | PSMA7 | 20q13.3 | 251 | 0.581 | F | 0.44511 | |
| 151 | Nbla20790 | NM_002933 | RNASE1 | 14q11.2 | 316 | 0.551 | F | 0.04873 | |
| 152 | Nbla21270 | NM_001915 alternative | CYB561 form? | 17q23.3 | 44 | 0.750 | F | 0.00016 | |
| 153 | Nbla21298 | NM_144967 | FLJ30058 | Xq26.1 | 189 | 0.618 | F | 0.100113 | |
| 154 | Nbla21322 | NM_000175 | GPI | 19q13.1 | 333 | 0.541 | UF | 0.009434 | |
| 155 | Nbla21394 | NM_000743 | CHRNA3 | 15q25.1 | 64 | 0.721 | F | 0.072464 | |
| 156 | Nbla21432 | NM_000034 | ALDOA | 16p11.2 | 284 | 0.571 | UF | 0.04041 | |
| 157 | Nbla21595 | NM_004499 | HNRPAB | 5q35.3 | 336 | 0.541 | UF | 0.007699 | |

(continued)

| Seq. ID No. | Gene Name on Spot | Acc.No.(known genes UCSC etc) | UCSC Homology | UCSC Mapping | rankin g6/2 | pairwise F-value | pairwise F-value | logrank p-value | |
|---|---|---|---|---|---|---|---|---|---|
| 158 | Nbla21642 | NM_003487 | TAF15 | 17q12 | 231 | 0.597 | F | 0.001076 | |
| 159 | Nbla21784 | NM_002276 | KRT19 | 17q21.2 | 136 | 0.655 | F | 0.000015 | |
| 160 | Nbla21844 | NM_138394 | LOC9290S | Zp22.1 | 124 | 0.657 | F | 0.000082 | |
| 161 | Nbla21852 | NM_006034 | TP-53I11 intron | 11p11.2 | 267 | 0.575 | UF | 0.010103 | |
| 162 | Nbla21871 | NM_001129 | AEBP1 | 7p13 | 352 | 0.525 | UF | 0.129418 | |
| 163 | Nbla21891 | NM_014396 | VPS41 | 7p14.1 | 19 | 0.784 | F | 0.000006 | |
| 164 | Nbla21984 | NM_005386 | NNAT | 20q11.2 | 234 | 0.595 | F | 0.025244 | |
| 165 | Nbla22156 | NM_014944 | CLSTN1 | 1p36.22 | 50 | 0.738 | F | 0.005233 | |
| 166 | Nbla22328 | NM_005507 | CFL1 | 11q13.1 | 334 | 0.541 | UF | 0.008023 | |
| 167 | Nbla22411 | NM_015665 | AAAS | 12q13.1 | 324 | 0.543 | UF | 0.044806 | |
| 168 | Nbla22424 | NM_004375 | COX11 | 17q22 | 217 | 0.600 | UF | 0.305225 | |
| 169 | Nbla22426 | NM_145900 | HMGA1 | 6p21.31 | 304 | 0.557 | F | 0.163535 | |
| 170 | Nbla22510 | NM_016250 | NDRG2 | 14q11.2 | 262 | 0.579 | F | 0.028274 | |
| 171 | Nbla22531 | NM_002045 | GAP43 | 3q13.31 | 24 | 0.776 | F | 0.004394 | |
| 172 | Nbla22554 | NM_000687 | AHCY | 20q11.2 | 65 | 0.721 | UF | 0.003946 | |
| 173 | Nbla22572 | NM_000790 | DDC | 7p12.2 | 41 | 0.754 | F | 0.000035 | |
| 174 | Nbla22633 | NM_080607 | C20orf102 | 20q11.2 | 317 | 0.551 | F | 0.002731 | |
| 175 | Nbla22643 NM_ | 017705 | FLJ20190 | 15q23 | 115 | 0.667 | UF | 0.046801 | |
| 176 | Nbla22960 | NM_021131 | PPP2R4 | 9q34.11 | 318 | 0.551 | UF | 0.053406 | |
| 177 | Nbla22997 | NM_005389- | PCMT1 | 6q25.1 | 310 | 0.554 | F | 0.00074 | |
| 178 | Nbla23003- | NM_001281 | CKAP1 | 19q13.1 | 321 | 0.551 | F | 0.50794 | |
| 179 | Nbla23007 | NM_021939 | FKBP10 | 17q21.2 | 90 | 0.687 | UF | 0.069405 | |
| 180 | Nbla23017 | MM_007178 | UNRIP | 12p12.3 | 326 | 0.543 | F | 0.028015 | |

(continued)

| Seq. ID No. | Gene Name on Spot | Acc.No.(known genes UCSC etc) | UCSC Homology | UCSC Mapping | rankin g6/2 | pairwise F-value | pairwise F-value | logrank p-value | |
|---|---|---|---|---|---|---|---|---|---|
| 181 | Nbla23089 | NM_014232 | VAMP2 | 17p13.1 | 132 | 0.655 | F | 0.001788 | |
| 182 | Nbla23144 | NM_014841 | SNAP91 | 6q14.2 | 264 | 0.576 | F | 0.000026 | |
| 183 | Nbla23163 | NM_003754 | EIF3S5 | 11p15.4 | 42 | 0.754 | UF | 0.000341 | |
| 184 | Nbla23178 | NM_004627 | WRB | 21q22.2 | 270 | 0.575 | F | 0.000244 | |
| 185 | Nbla23181 | NM_080725 | C20orf139 | 20p13 | 338 | 0.535 | UF | 0.108356 | |
| 186 | Nbla23325 | NM_003275 | TMOD1 | 9q2233 | 205 | 0.605 | F | 0.000088 | |
| 187 | Nbla23420 | NM_173798 | LOC170261 | Xq24 | 206 | 0.605 | F | 0.000033 | |
| 188 | Nbla23424 | NM_001404 | EEF1G | 11q12.3 | 45 | 0.750 | UF | 0.003579 | |
| 189 | Nbla23443 | NM_014718 | CLSTN3 | 12p13.3 | 167 | 0.635 | F | 0.000234 | |
| 190 | Nbla23458 | NM 005053 | RAD23A | 19p13.2 | 358 | 0.521 | UF | 0.143918 | |
| 191 | Nbla23525 | BC035249 | BC035249 | Xq222 | 70 | 0.708 | F | 0.000003 | |
| 192 | Nbla23668 | AB028962 | KIAA1039 | 17p13.3 | 224 | 0.597 | F | 0.000634 | |
| 193 | Nbla23741 | NM_002404 | MFAP4 | 17p11.2 | 354 | 0.521 | UF | 0.005134 | |
| 194 | Nbla23949- | NM_015331 | NCSTN | 1q23.2 | 219 | 0.600 | F | 0.056869 | |
| 195 | Nbla24098 | NM_003127 | SPTAN1 | 9q34.11 | 144 | 0.647 | F | 0 | |
| 196 | Nbla24174 | NM_000521 | HEXB | 5q13.3 | 322 | 0.545 | UF | 0.273185 | |
| 197 | Nbla24848 | NM_017722 | FLJ20244 | 19p13.2 | 168 | 0.635 | UF | 0.015188 | |
| 198 | Nbla24920 | NM_006266 | RALGDS | 9q34.2 | 220 | 0.600 | F | 0.007387 | |
| 199 | Nbla24963 | NM_005517 | HMGN2 | 1p36.11 | 180 | 0.629 | F | 0.022671 | |
| 200 | Nbla24987 | NM_001978 | EPB49 | 8p21.3 | 196 | 0.615 | F | 0.004811 | |
| 201 | gene033-1 | | | | 363 | 0.513 | | 0.016162 | control |
| 202 | gene033-1 | | | | 363 | 0.513 | | 0.016162 | control |
| 203 | gene033-1 | | | | 363 | 0.513 | | 0.016162 | control |
| 204 | gene033-1 | | | | 363 | 0.513 | | 0.016162 | control |

(continued)

| Seq. ID No. | Gene Name on Spot | Acc.No.(known genes UCSC etc) | UCSC Homology | UCSC Mapping | rankin g6/2 | pairwise F-value | pairwise F-value | logrank p-value | |
|---|---|---|---|---|---|---|---|---|---|
| 205 | qene019-1 | | | | 125 | 0.657 | | 0.47227 | control |
| 206 | gene019-1 | | | | 125 | 0.657 | | 0.47227 | control |
| 207 | gene019-1 | | | | 125 | 0.657 | | 0.47227 | control |
| 208 | gene019-1 | | | | 125 | 0.657 | | 0.47227 | control |
| 209 | H2O | | | | | 0.000 | | - | control |
| 210 | H2O | | | | | 0.000 | | - | control |
| 211 | H2O | | | | | 0.000 | | - | control |
| 212 | H2O | | | | | 0.000 | | - | control |

In this invention, "polynucleotide" is referred to as a molecule in which a plural of, preferably not less than 30 phosphate esters of nucleosides in which a purine or a pyrimidine is attached to a sugar via a β-N-glycosidic bond (ATP, GTP, CTP, UTP, dATP, dGTP, dCTP or dTTP) are bound to one another. "Gene transcript" is referred to as a mRNA transcribed from genomic gene or a cDNA synthesized from this mRNA.

"Predicting prognosis" means to predict whether the postoperative status of a patient with neuroblastoma is good or poor. More specifically, the "good prognosis" indicates the status in which a neuroblastoma is localized or regressed, or it becomes a benign sympathetic ganglion cell tumor. Examples include the case where the patient is alive 5 years or more after the operation without recurrence. The "poor prognosis" indicates the status in which the progression of neuroblastoma is confirmed, and examples include the status where there is a risk that the patient will die within 3 years after the operation.

Other terms and concepts in this invention will be defined in detail in the description of the embodiments or Examples of the invention. The terms are basically in accordance with IUPAC-IUB Commission on Biochemical Nomenclature or the meanings of terms used commonly in the art. In addition, various techniques used for implementing the invention can be easily and surely carried out by those skilled in the art based on a known literature or the like except for the techniques whose sources are particularly specified. For example, techniques of genetic engineering and molecular biology can be carried out according to the methods described in J. Sambrook, E. F. Fritsch & T. Maniatis, "Molecular Cloning: A Laboratory Manual (2nd edition)", Cold Spring Harbor Laboratory Press, Cold Spring Harbor, New York (1989); D. M. Glover et al. ed., "DNA Cloning", 2nd ed., Vol. 1 to 4, (The Practical Approach Series), IRL Press, Oxford University Press (1995); Ausubel, F. M. et al., Current Protocols in Molecular Biology, John Wiley & Sons, New York, N.Y, 1995; Japanese Biochemical Society ed., " Zoku Seikagaku Jikken Koza 1, Idenshi Kenkyuho II" Tokyo Kagaku Dozin (1986); Japanese Biochemical Society ed., " Shin Seikagaku Jikken Koza 2, Kakusan III (Kumikae DNA Gijutsu)" Tokyo Kagaku Dozin (1992); R. Wu ed., "Methods in Enzymology", Vol. 68 (Recombinant DNA), Academic Press, New York (1980); R. Wu et al. ed., "Methods in Enzymology", Vol. 100 (Recombinant DNA, Part B) & 101 (Recombinant DNA, Part C), Academic Press, New York (1983); R. Wu et al. ed., "Methods in Enzymology", Vol. 153 (Recombinant DNA, Part D), 154 (Recombinant DNA, Part E) & 155 (Recombinant DNA, Part F), Academic Press, New York (1987), etc. or the methods described in the references cited therein or substantially the same methods or the modifications thereof.

**Brief Description of Drawings**

Fig. 1 is a schematic representation of machine learning and cross validation. Originally 136 patient samples were prepared. All of them were used in the Kaplan-Meier analysis. In the subsequent supervised classification analysis, 116 samples whose prognosis was known at 24 month after diagnosis were used. 116 samples were divided into 87 samples for cross-validation and 29 samples for the final test. In the cross-validation analysis, the outcome of randomly selected 9 samples are predicted by a classifier constructed from the rest 78 samples, and repeated this process 100 times by varying the set of 9 samples. The scale parameter of the Gaussian kernel and the number of genes were determined so as to minimize the mean prediction error (validation error). The classifier using those parameter values was assessed by the 29 samples as the final test. 116 samples were also assessed again by leave-one-out (LOO) analysis.

Fig. 2 shows discrimination accuracy (F-value) by the Gaussian-kernel GP classifier for various numbers of genes, *N*. Different line type indicates a different parameter value (scale parameter used in the Gaussian kernel). Blue circle denotes the best accuracy at scale=0.02. (*N*=70)

Fig. 3 is posterior probability of unfavorable prognosis after 24 months for 87 learning data samples, output by the Gaussian-kernel GP classifier. Left panel: Neuroblastoma samples. Right panel: Prediction by a GP classifier with a Gaussian kernel of scale 0.02 and *N*=70. A green circle denotes an answer; if it is located to the rightmost (leftmost) position, the answer for that sample is 'dead' ('alive'). A '+' mark denotes the posterior value predicted by the GP classifier, in a case that the sample belonged to a validation data set among 100 cross-validation trials, and a red circle or cross denotes the mean over such validation trials. The red line is the difference between the answer and the mean (red circle or cross); the longer, the worse the prediction of the classifier is.

Fig. 4 is posterior probability of unfavorable prognosis after 24 months, output by the Gaussian-kernel GP classifier. 29 new samples were used for test and additive 20 samples are also shown whose prognosis at 24 months is unknown. Other information is same as those of Fig. 3.

Fig. 5 is disease-free survival of patients stratified based on the posterior value. Kaplan-Meier's survival curves for neuroblastoma samples with posterior>0.5 (red) and those with posterior<0.5 (blue). The posterior was obtained by a leave-one-out analysis with the Gaussian-kernel GP classifier. P-value of log-rank test between red and blue lines was much smaller than $10^{-5}$.

Fig. 6 is disease-free survival of patients stratified based on the posterior value, as same as Fig. 5. Kaplan-Meier's survival curves for neuroblastoma samples in the intermediate subset (Type III) with posterior>0.5 (red), posterior<0.5 (blue) and together (green). P-value of log-rank test between red and blue was much smaller than $10^{-5}$.

Fig. 7 shows receiver operating characteristic (ROC) curves. Performance of prognosis markers and the Gaussian-kernel GP classifier in the two-dimensional plane of sensitivity and specificity is shown. Sensitivity (horizontal axis) is the rate of correct prediction among favorable samples, and specificity (vertical axis) is the rate of correct prediction among unfavorable samples. Since the upper-right corner represents 100% sensitivity and 100% specificity, a classifier located at that position is ideal. A blue cross 'x' denotes a sensitivity-specificity point achieved by prognosis marker. A blue circle 'o' denotes the prediction by the combination of three existing markers, 'Age', 'Stage' and 'MYCN'. A GP classifier outputs its prediction as posterior, a real value. Since its binary prediction, favorable or unfavorable, depends on the threshold, a curve on the sensitivity-specificity plane can be plotted by changing the threshold. Such a curve is called a receiver operating characteristic (ROC) curve. A magenta broken line denotes prediction using only microarray data, a green broken line denotes prediction using microarray data and the 'Stage' marker, and a red real line denotes prediction using microarray data, and 'Age', 'MYCN' and 'Stage' markers.

Fig. 8 shows expression profiles of the 70 genes selected for predicting the prognosis. Unsupervised clustering of 136 neuroblastoma samples and the 70 genes selected in this study, based on the Gaussian kernel. Blue; type I tumor, Green; type II tumor. Red; type III tumor (see text). The expression of each gene in each sample is represented by the number of standard deviations above (red) or below (blue) the mean for that gene across all 136 samples.

Fig. 9 is clustering of the samples within the three tumor groups according to the 70 genes' expression shown in Fig. 9.

Fig. 10 shows chip quality and reproducibility. Deviation of the normalized log expression ratio from its average. For each gene spot, blue dots, a red circle, and a pair of green dots denote log expression ratio for the 136 samples, the average over the samples, and the standard deviation (upper and lower) over the samples, respectively. The horizontal axis denotes a gene identifier, and duplicated spots have the same identifier. If red circles do not much vary within the spots labeled by a single identifier, the log expression ratio of that gene has high reproducibility.

Fig. 11 also shows chip quality and reproducibility. Scatter plots for eight pairs of duplicated spots in a slide, where each dot denotes the expression of two spots of the same gene in a single slide. Horizontal and vertical axis denote $\log_2$ expression ratios. Root mean squared variance of each pair is about 0.2.

Fig. 12 further shows chip quality and reproducibility. Reproducibility of the same spot between two different slides. Horizontal and vertical axis denote $\log_2$ expression ratios. Root mean squared difference between each pair is about 0.4.

Fig. 13 shows posterior variation and robustness against artificially added Gaussian noise. In each panel, the vertical axis denotes the posterior value and the horizontal axis denotes the samples sorted in order of the original (without noise) posterior value (green). For each sample, posterior was calculated 20 times by adding Gaussian noise with 4 types of std.: 0.5, 1.0, 1.5 and 2.0, where std.=1 means that the noise scale is as large as the standard deviation of the original log expression ratio. Red points denote answers and blue points denote posterior in the 20 trials. Posterior value $y$ denotes the probabilistic prognosis prediction, where its binarized $y$<0.5 or $y$>0.5 means that the sample is predicted as favorable or unfavorable, respectively, and when $y$ is around 0.5, the prediction is supposed as unconfident. The original posterior values (green) are y<0.5 for patients whose prognosis is actually favorable, and $y$>0.5 for actually unfavorable. When noise with std.=0.5 is added (upper right panel), each posterior value (a small blue dot) changes from its original posterior (green). However, it rarely goes over the $y$=0.5 line, especially when the classifier is originally confident of the prediction, which indicates the robustness of the guess against the additional noise. When noise gets further large, the posterior values approach $y$=0.5 but their binarization seldom leads to wrong guess. In addition, when noise is extremely large and the gene expression shows a different pattern with those of the given samples, our supervised classifier outputs an unconfident posterior (lower right panel). Such a feature makes the prediction reliable like when applied in the clinical field.

## Best Mode for Carrying Out the Invention

Each of the polynucleotides consisting of the nucleotide sequences of SEQ ID NOs. 1, 5, 6, 14, 16, 17, 19, 22-24, 28, 29, 31, 37, 39,40, 43, 44, 47-52, 54, 57-60, 62, 64, 65, 67, 68, 72-75, 77, 78, 80-82, 84, 87, 89-91, 94, 100, 103, 112, 113, 118, 120, 129, 130, 132, 136, 138, 142, 144, 145, 148, 150- 153, 155, 158-160, 163-165, 169-171, 173, 174, 177, 178, 180-182, 184, 186, 187, 189, 191. 192. 194, 195, 198-200 is a cDNA of each of the specific 96 genes (see Table 1) whose expression is increased in a good prognosis patient with neuroblastoma. Each of the polynucleotides of SEQ ID NOs. 2-4, 7-13, 15, 18, 20, 21, 25-27, 30, 32-36, 38, 41, 42, 45, 46, 53, 55, 56, 61, 63, 66, 69-71, 76, 79, 83, 85, 86, 88, 92, 93, 95-99, 101, 102, 104-111, 114-117, 119, 121-128, 131. 133-135, 137, 139-141, 143, 146-147, 149, 154, 156, 157, 161, 162, 166-168, 172, 175, 176, 179, 183, 185, 188, 190, 193, 196, 197 is a cDNA of each of the specific 104 genes (see Table 1) whose expression is increased in a poor prognosis patient with neuroblastoma. The microarray described herein is a microarray having probes related to good prognosis, which are hybridized to each of the 25 to 45 types among 96 genes related to good prognosis, and probes related to poor prognosis, which are hybridized to each of the 25 to 45 types among 104 gene transcripts related to poor prognosis. In other words, this microarray has 50 to 90 types, preferably 60 to 80 types, more preferably 65 to 75 types of probes which are hybridized to each of the total of 200 types of gene transcripts related to good prognosis and poor prognosis. Incidentally, from the results of the Examples described later, 70 genes (33 genes related to good prognosis and 37 genes related to poor prognosis) shown in Table 2 are illustrated as a preferred test target, however, the microarray described herein is not intended to be limited to using these genes as a target. It will be easily conceived by those skilled in the art that the number and the types of probes can be determined by, for example, selecting more preferred target genes as needed from the results obtained by the diagnostic method of the second invention (see the Examples described later), the results of the subsequent follow-up study on the patient and the like. 50 to 90 types, preferably 60 to 80 types, more preferably 65 to 75 types of probes which are hybridized to each of the total of 200 types of gene transcripts related to good prognosis and poor prognosis. Incidentally, from the results of the Examples described later, 70 genes (33 genes related to good prognosis and 37 genes related to poor prognosis) shown in Table 2 are illustrated as a preferred test target, however, the microarray described herein is not intended to be limited to using these genes as a target. It will be easily conceived by those skilled in the art that the number and the types of probes can be determined by, for example, selecting more preferred target genes as needed from the results obtained by the diagnostic method of the second invention (see the Examples described later), the results of the subsequent follow-up study on the patient and the like.

With respect to the probes for the microarray described herein, for example, in the case where RNAs (mRNAs) of respective genes related to good prognosis and poor prognosis are used as a target, respective cDNAs of SEQ ID NOs. 1, 5, 6, 14, 16, 17, 19, 22-24, 28, 29, 31, 37, 39,40, 43, 44, 47-52, 54, 57-60, 62, 64, 65, 67, 68, 72-75, 77, 78, 80-82. 84, 87, 89-91, 94, 100, 103, 112, 113, 118, 120, 129, 130, 132, 136, 138, 142, 144, 145, 148, 150- 153, 155, 158-160, 163-165, 169-171, 173, 174, 177, 178, 180-182, 184, 186, 187, 189, 191, 192, 194, 195, 198-200 and SEQ ID NOs. 2-4, 7-13, 15, 18, 20, 21, 25-27, 30, 32-36, 38, 41, 42, 45, 46, 53, 55, 56, 61, 63, 66, 69-71, 76, 79, 83, 85, 86, 88, 92, 93, 95-99, 101, 102, 104-111, 114-117, 119, 121-128, 131, 133-135, 137, 139-141, 143, 146-147, 149, 154, 156, 157, 161, 162, 166-168, 172, 175, 176, 179, 183, 185. 188, 190, 193, 196, 197 or their partial continuous sequences (for example, about 15 to 50 bp) may be used as the probes. In addition, in the case where cDNAs of genes related to good prognosis and poor prognosis are used as a target for detection, complementary polynucleotide strands for the respective cDNAs may be used as the probes.

As the cDNA probe for targeting a gene mRNA, for example, a full length cDNA prepared by a known method (Mol. Cell. Biol. 2, 167-170,1982; J. Gene 25, 263-269, 1983; Gene, 150, 243-250, 1994) using poly(A)+RNA extracted from a human cell as a template can be used. Also, it can be synthesized by the RT-PCR method using a mRNA isolated from a human cell as a template and using a primer set designed based on the information of the nucleotide sequences of Seq. ID No. 1 to 200. Further, a target full length cDNA can be synthesized by synthesizing partial sequences with a DNA oligo synthesizer and ligating them by an enzymatic method and a subcloning method. In addition, in the case where a polynucleotide consisting of a partial continuous sequence of a cDNA is used as a probe, an objective short-chain cDNA can be prepared by a method of digesting the obtained full length cDNA with an appropriate restriction enzyme or by a DNA oligo synthesizer or a known chemical synthesis technique (for example, Carruthers (1982) Cold Spring Harbor Symp. Quant. Biol. 47: 411-418; Adams (1983) J. Am. Chem. Soc. 105: 661; Belousov (1997) Nucleic Acid Res. 25: 3440-3444; Frenkel (1995) Free Radic. Biol. Med. 19: 373-380; Blommers (1994) Biochemistry 33: 7886-7896; Narang (1979) Meth. Enzymol. 68: 90; Brown (1979) Meth. Enzymol. 68:109; Beaucage (1981) Tetra. Lett. 22: 1859; US Patent No. 4,458,066).

On the other hand, a probe in the case of targeting a cDNA synthesized from a gene mRNA is a complementary polynucleotide for a full length or a partial continuous sequence of respective cDNAs, and can be prepared by the same DNA oligo synthesizer or known chemical synthesis technique as described above.

The microarray of the first invention uses probes as described above and can be prepared in the same manner as a common DNA microarray. As a method of preparing the microarray, a method of synthesizing the probes directly on the surface of a solid phase support (on-chip method) and a method of immobilizing the probes prepared in advance on the surface of a solid phase substrate are known, however, it is preferred that the microarray of this invention be prepared by the latter method. In the case where the probes prepared in advance are immobilized on the surface of a solid phase substrate, a probe in which a functional group was introduced is synthesized, the probe is spotted on the surface of the solid phase substrate subjected to a surface treatment, and have it covalently bound thereto (for example, Lamture, J.B. et al. Nucl. Acids Res. 22: 2121-2125, 1994; Guo, Z. et al. Nucl. Acids Res. 22:5456-5465, 1994). In general, the probe is covalently bound to the solid phase substrate subjected to a surface treatment via a spacer or a crosslinker. A method of aligning small pieces of polyacrylamide gel on the surface of glass and having the probe covalently bound thereto (Yershov, G. et al. Proc. Natl. Acad. Sci. USA 94: 4913, 1996), or a method of binding the probe to the solid phase substrate coated with poly L-lysine (JP 2001-186880A) are also known. In addition, a method of preparing an array of microelectrode on a silica microarray, in which a permeation layer of agarose containing streptavidin is provided on the electrode to make it a reactive region, immobilizing a biotinylated probe by positively charging this region and controlling the electric charge of the region, thereby enabling high-speed and stringent hybridization is also known (Sosnowski, R.G. et al. Proc. Natl. Acad. Sci. USA 94: 1119-1123, 1997). The microarray of this invention can be prepared by any one of the foregoing methods. In the case where the probe is dropped on the surface of the solid phase substrate to perform spotting, it can be performed by a pin system (for example, US Patent No. 5,807,5223), however, it is preferred that an inkjet system disclosed in JP 2001-116750A or JP 2001-186881A be adopted because uniform spots in a specific shape are formed. In addition, this inkjet system can make the number of probes contained in the respective probe spots equal, therefore, the difference in hybridization due to the difference in the probe length can be accurately measured. Further, it is recommended for forming preferred spots that spotting be repeated as disclosed in JP 2001-186880A, or a probe solution (a solution containing a moisturizing substance) comprising the composition disclosed in WO 03/038089 A1 be used.

After the spotting, each spot is immobilized on the solid phase substrate by cooling, adding moisture to the spots (maintaining a humidity of up to about 80% for a given period of time) and performing such as an immobilization treatment or the like by calcination and drying, whereby the microarray can be completed.

As the solid phase substrate for the microarray, other than glass (slide glass) used for a common microarray, plastic, silicone, ceramic or the like can be also used.

The prediction of the prognosis of neuroblastoma of the second invention is carried out by using the foregoing microarray. In other words, this diagnostic method is a method comprising the following steps (a) to (c):

(a) a step of labeling a gene transcript obtained from a tumor cell of a patient diagnosed as having neuroblastoma;
(b) a step of bringing the labeled gene transcript into contact with the microarray according to claim 1;
(c) a step of measuring the labeling signal of each of the gene transcripts hybridized to 24 or more of the probes related to good prognosis and 25 or more of the probes related to poor prognosis on the microarray, respectively.

For example, in the case where the gene transcript to become a target for detection is a cDNA, a cDNA is prepared as a PCR product from a genomic gene isolated from an examinee or total RNAs in the step (a). During the PCR amplification, the cDNA is labeled by incorporating a labeling primer (for example, a primer to which a cyanine organic dye such as Cy3 or Cy5 was attached) thereinto. In the step (b), the targeting cDNA is brought into contact with the microarray to be hybridized to the probe on the microarray. In the case where the gene transcript to become a target for detection is a mRNA, total RNAs extracted from the cells of an examinee are labeled by using a commercially available labeling kit (for example, CyScribe™ RNA labeling kit: manufactured by Amersham Pharmacia Biotech Co.) or the like.

Hybridization in the step (b) can be carried out by spotting an aqueous solution of the labeled cDNA dispensed on a 96-well or 384-well plastic plate on the microarray. The amount to be spotted can be about 1 to 100 nl. It is preferred that hybridization be carried out at a temperature from room temperature up to 70°C for 1 to 20 hours. After finishing the hybridization, washing is carried out by using a mixed solution of a surfactant and a buffer solution to remove unreacted labeled polynucleotides. As the surfactant, it is preferred that sodium dodecyl sulfate (SDS) be used. As the buffer solution, citrate buffer solution, phosphate buffer solution, borate buffer solution, Tris buffer solution, Good's buffer solution or the like can be used, however, it is preferred that citrate buffer solution be used. In the step (c), the signal obtained by the labeled gene product hybridized to the probe is measured.

The diagnostic method of the second method determines from the signal obtained as above that the prognosis of the patient is good if significant labeling signals for 24 or more (25 to 45, preferably 30 to 40, more preferably 32 to 38) of the probes related to good prognosis were obtained, and that the prognosis of the patient is poor if significant labeling signals for 25 or more (25 to 45, preferably 30 to 40, more preferably 32 to 38) of the probes related to poor prognosis were obtained.

Hereunder, this invention will be explained in detail by showing as the Examples the experimental results of identifying the target genes for the microarray or the diagnostic method of this invention, however, this invention is not intended to be limited to the following examples.

## Examples

1. Materials and methods

1-1. Patients and tumor specimens

Fresh, frozen tumor tissues were sent to the Division of Biochemistry, Chiba Cancer Center Research Institute, from a number of hospitals in Japan. The informed consents were obtained in each institution or hospital. Most of the samples were resected by pre-operational biopsy or surgery, without treatment by chemotherapy or radiotherapy. After the operation, patients were treated according to previously described common protocols (Kaneko, M. et al. Med. Pediatr Oncol 31, 1-7 (1998)). Biological information on each tumor including *MYCN* gene copy number, *TrkA* gene expression, and DNA ploidy, was analyzed in our laboratory. All tumors were classified according to the International Neuroblastoma Staging System (INSS): stages 1 and 2, localized neuroblastomas; stages 3 and 4, locally and regionally growing and distantly metastatic neuroblastomas; and stage 4s, neuroblastomas in children under one year of age, with metastases restricted to skin, liver, and bone marrow, usually regressing spontaneously (Brodeur et al., 1993: non-patent document 3).

In Japan, a mass screening program for infants at the age of 6 months has been performed since 1985. Patients found by this screening have been mostly classified to the early stage of the disease, although a small proportion had unfavorable prognoses (Sawada et al., Lancet 2, 271-3 (1984)). Among the 136 tumors of being analyzed, 68 of those were found by this screening. All diagnoses of neuroblastoma were confirmed by histological assessment of a surgery resected tumor specimen.

Frozen tissues were homogenized in guanidinium isothiocyanate, and total RNA was extracted from each sample using the AGPC method (Chomczynski and Sacchi, Anal Biochem 162, 156-9 (1987)). RNA integrity, quality, and quantity were then assessed by electrophoresis on Agilent RNA 6000 nano chip using Agilent 2100 BioAnalyzer (Agilent Technologies, Inc.).

1-2. cDNA microarray experiments

To make a neuroblastoma-specific cDNA microarray (named as CCC-NB5000-Chip ver.1), 5,340 cDNA clones were selected from -10,000 of those isolated from three types of neuroblastoma oligo-capping cDNA libraries (favorable, unfavorable and stage 4s neuroblastomas) after a removal of highly duplicated genes. Insert DNAs were amplified by polymerase chain reaction (PCR) from these cDNA clones, purified by ethanol precipitation, and spotted onto a glass slide in a high density manner by an ink-jet printing tool (NGK insulators, Ltd.). Additional 80 cDNAs that had been described as candidates for prognostic indicators for neuroblastoma were also spotted on the array.

Ten micrograms of each total RNA were labeled by using CyScribe™ RNA labeling kit according to a manufacturer's manual (Amersham Pharmacia Biotech), followed by probe purification with Qiagen MinElute™ PCR purification kit (Qiagen). A mixture of an equal amount of RNA from each of four neuroblastoma cell lines (NB69, NBLS, SK-N-AS, and SH-SY5Y) was used as a reference. RNAs extracted from primary neuroblastoma tissues and those of reference mixture were labeled with Cy3 and Cy5 dye, respectively, and used as probe together with yeast tRNA and polyA for suppression. Subsequent hybridization and washing were performed as described previously (Takahashi, M. et al. Cancer Res 62, 2203-9 (2002) ; Yoshikawa, T. et al. Biochem Biophys Res Commun 275, 532-7 (2000)). The hybridized microarrays were scanned using an Agilent G2505A confocal laser scanner (Agilent Technologies, Inc.) and the fluorescent intensities were quantified by GenePix™ Pro microarray analysis software (Axon Instruments, Inc.).

1-3. Data preprocessing

To remove the biases of microarray system, the LOWESS normalization (Quackenbush, J. Nat Genet 32, 496-501 (2002)) was used. When the Cy3 or Cy5 strength for a clone was smaller than 3, it is regarded as abnormally small, and the log expression ratio of the corresponding clone is treated as a missing value. The rate of such missing entries was less than 1%. After the normalization of a 5,340 (genes)-by-136 (samples) log expression matrix and missing value removal, each missing entry was imputed to an estimated value (Oba, S. et al. Bioinformatics (2003)).

Normalization is necessary for removing various uninteresting artifacts like unequal cDNA quantities on a slide, efficiency difference between two fluorescence dyes, and others. Several reports have suggested that the log Cy3-Cy5 ratio is significantly dependent on fluorescence intensity of each gene. In order to remove such systematic biases, a locally weighted linear regression (LOWESS) normalization (Cleveland, 1979; Quackenbush, 2002) was used, which removes the intensity-dependent biases. The normalized log expression ratio $y_i$ of gene $i$ is given by

$$y_i = \log Cy3_i - \log Cy5_i - f(\log Cy3_i + \log Cy5_i),$$

where $Cy3_i$ and $Cy5_i$ are Cy3 and Cy5 fluorescence strength of gene *i*, respectively. $f(x)$ is a normalization function, which represents the intensity-ratio (I-R) bias, and is estimated using all spots on a single slide. Normalization across slides was not considered.

For a 5,340-by-136 log expression ratio matrix after the LOWESS normalization and the removal of suspicious log-ratio values, each missing entry was imputed to an estimated value, by the Bayesian PCA imputation method (BPCAfill) proposed by us previously (Oba et al., 2003). By evaluating the BPCAfill prediction for 1% missing values added artificially to the expression matrix, the root mean squared prediction error by BPCAfill was estimated as 0.2, which is consistent with the reproduction standard deviation of duplicated genes, 0.3.

1-4. Supervised machine learning and cross validation

The 116 samples whose prognosis after 24 months had been checked were used to train a supervised classifier that predicts the prognosis of a new patient. Selecting genes that are related to the classification is an important preprocess for reliable prediction. Therefore, after omitting genes whose standard deviation over the 116 slides was smaller than 0.5, *N* genes where *N* is determined by a cross-validation technique were selected, based on the pair-wise correlation method.

If a supervised classifier using all of the 5,340 genes was constructed, the prediction for a new sample is not reliable. This is a typical problem of microarray analyses, in which the number of genes is usually much larger than that of samples. Therefore, selecting genes that are related to the classification (discrimination) is important for reliable prediction.

The inventors first omitted genes whose standard deviation over the 116 slides was smaller than 0.5. After that, the inventors selected *N* genes based on the following criterion, where the number *N* is determined by a cross-validation technique. In the fields of statistical pattern recognition, univariate feature extraction based on *t* statistics, permutation *p*-value, or so on, has been used for feature extraction. In our case, a univariate feature extraction corresponds to a gene-wise selection ignoring correlation among genes. According to the pair-wise method (Bo, T, & Jonassen, I. Genome Biol 3, (2002)), on the other hand, a pair-wise correlation is considered in the gene selection so that higher discrimination accuracy is obtained using a smaller number of genes. Although *t* statistics was used in the original work (Bo and Jonassen, 2002), the following pair-wise *F* score was used in the gene selection.

In a binary discrimination problem between class 1 ($n_1$ samples) and class 2 ($n_2$ samples), using the expression ratio of a single gene, it is required to determine a discrimination threshold. Let $p_1$ and $p_2$ denote the discrimination accuracy for samples in classes 1 and 2, respectively. The *F* value for this single gene is then given by the harmonic mean of $p_1$ and $p_2$: $F = 2 p_1 p_2 / (p_1 + p_2)$. When the *F* value is maximized with respect to the discrimination threshold, it is called the *F* score of that gene. The *F* value is more robust than the *t* statistics especially when outliners exist and/or there is unbalance between $n_1$ and $n_2$. Similarly to an *F* value of a single gene, the inventors define an *F* value of a gene pair. Using two genes, *i* and *j*, construct a linear discriminator in the two dimensional space composed by expression ratios of genes *i* and *j*. By optimizing the linear discriminator in the two dimensional space, an *F* score for a gene pair *(i, j)* is obtained. Pair-wise *F*-value (PF) scores are then calculated by the following procedure.

Calculate *F* scores for all genes and select into a pool of 500 genes whose individual *F* scores are the largest. Let PF scores of the not-selected genes be zero.

For every pair of 500 genes in the pool, calculate an *F* score.

Take out the pair whose *F* score is the largest from the pool, so that the *F* scores for the two genes are the same as the *F* score of that pair.

Until there are no more genes in the pool, repeat step 3.

The inventors used PF scores for selecting *N* genes in the gene selection.

GP classifiers were used for the supervised classification. Among the 116 samples, 29 test samples were selected so that their prognosis factors have similar distributions to those of the 116 samples. The remaining 87 training samples were further separated into 78 learning samples and 9 validation samples. A supervised GP classifier was trained by the learning samples and assessed by the validation samples. This process was repeated 100 times (see Figure 1) by varying the learning and training samples and obtained mean discrimination accuracy. Here, the gene selection based on the pair-wise correlation method was executed for each learning data. Thus, the gene selection procedure was also assessed, though this assessment has often been ignored in various microarray studies.

From the analysis to compare two types of kernel functions, a polynomial kernel and a Gaussian kernel, a Gaussian kernel was better, because the number of genes was smaller, the accuracy of the outcome prediction was higher, and more stable against the noise with a Gaussian kernel. The inventors therefore concluded that the Gaussian kernel is better than the polynomial kernel in the outcome prediction of neuroblastoma, and chose the former in this study.

1-5. Clustering analysis and survival analysis

For unsupervised clustering, Gaussian kernel functions were also used. The inventors defined distance measure based on Gaussian kernels obtained through the supervised classification process (see above). Each sample is represented by a feature vector defined by the kernel function, and the distance of two feature vectors was measured as a Pearson's correlation of the vectors. This clustering in the kernel space could exhibit more robust cluster structures than those by the conventional hierarchical clustering.

The Kaplan-Meier survival analysis was also programmed by us and used to compare patient survival. To assess the association of selected gene expression with patient's clinical outcome, the statistical *p*-value was generated by the log-rank test.

2. Results

2-1. Neuroblastoma-proper cDNA microarray and gene expression in 136 primary tumors

The inventors have so far obtained 5,500 genes from the mixture of oligo-capping cDNA libraries generated from 3 primary neuroblastomas with favorable outcome (stage 1, high *TrkA* expression and a single copy of *MYCN*), 3 tumors with poor prognosis (stage 3 or 4, low expression of *TrkA* and amplification of *MYCN*), and a stage 4s tumor (just before starting rapid regression) (Ohira et al., 2003a, 2003b: non-patent documents 19 and 20). The inventors then made a neuroblastoma-proper cDNA microarray harboring the spots of 5,340 genes onto a slide glass using a ceramics-based ink-jet printing system. This in-house cDNA microarray appeared to have overcome the previous problems caused by pin-spotting such as an uneven quantity or shape of the individual spots on an array. Ten μg each of total RNA extracted from the 136 frozen tissues of primary neuroblastomas was labeled with Cy3 dye. As a common reference, the mixture of total RNA obtained from 4 neuroblastoma cell lines with a single copy of *MYCN* (NB69, NBLS, SK-N-AS, and SH-SY5Y) was labeled with Cy5 dye. The inventors have randomly selected the tumor samples from the neuroblastoma tissue bank and hybridization was successfully performed in 136 tumors consisting of 41 in stage 1, 21 in stage 2, 34 in stage 3, 28 in stage 4, and 12 in stage 4s. The stage 4s neuroblastoma shows special pattern of clinical behavior and its widespread metastases to skin, liver and bone marrow regress spontaneously. Sixty-eight tumors were found by mass screening of the urinary cathecolamine metabolites at 6 months after birth. The follow-up duration was ranged from 3 to 239 months (median: 32 months, mean: 50.6 months) after diagnosis (see Figure 3).

The inventors first evaluated the quality of our cDNA microarray. The log Cy3/Cy5 fluorescence ratio of each gene spot was normalized to eliminate the intensity-dependent biases. Since our cDNA microarray contains 260 duplicated or multiplicated genes, the expression ratio of such a duplicated gene was represented by the average of the multiple spots. Based on the estimation performance for missing values (see Supplemental data, below) and the reproduction variance of duplicated genes, the standard deviation of log-ratio of a single gene was about 0.2-0.3, which was sufficiently small (Figure 10). The scattered plots of log Cy3/Cy5 fluorescence ratio between the duplicated gene spots in 136 experiments and those between repeated experiments also indicated the reproducibility of spotting and experiment (Suppl. Figure S1B and SIC). These suggest that our cDNA microarray was highly quantitative and reproducible.

2-2. Supervised classification

To develop a statistical tool that predicts the prognosis of a new patient with the tumor, the inventors introduced a supervised classification. Since the variation of follow-up duration created the noise in the supervised classification, the inventors used the patient's outcome (dead or alive) at 24 months after diagnosis as the target label to be predicted. Because the outcome of 20 of 136 samples are unknown at 24 months after diagnosis, the rest 116 sample data were used subsequently (Figure 1). The inventors first omitted the genes whose standard deviation over the 116 slides was smaller than 0.5, because the background noise level was about 0.3 (see above). The inventors then selected *N* genes based on the following criterion, where the number *N* is determined by a cross-validation technique. Gene selection was performed according to a variation of the pair-wise correlation method (Bo and Jonassen, 2002) to obtain a higher discrimination accuracy using a smaller number of genes (see Figure 13).

The inventors decided to use Gaussian-kernel Gaussian Process (GP) classifiers for the supervised classification. A GP classifier is one of kernel-based classifiers (MacKay. D. J. C. Neural Network and Machine Learning, 133-165 (1998)). It resembles support vector machine (SVM) classifiers, but is based on a probabilistic model and has an advantage when interpreting the output.

2-3. Test and cross validation

The 116 samples were in advance separated into 87 training samples used for calculating the supervised classifier and 29 test samples to evaluate the obtained classifier (Figure 1). In the training phase, the inventors never used the 29 test samples. The training samples were further separated into learning samples (~90%) and validation samples (~10%), and both of the gene selection and the parameter determination were assessed by a cross-validation technique.

A GP classifier outputs a *posteriori* probability (posterior) of each sample, which represents the predictive probability that the patient's prognosis is poor. An accuracy represents the rate of correct prediction, when binary prognosis prediction is done based on whether the posterior is larger than a threshold 0.5. *F*-value is the harmonic mean of accuracy over favorable and unfavorable neuroblastoma samples (see Figure 13). Figure 2 shows the *F*-value by the Gaussian-kernel GP classifier, for various numbers of genes, *N*. The best number of genes was thus determined as *N*=70 by the cross-validation technique.

Figure 3 shows the posterior of the 87 training samples by the GP classifier whose parameter was optimally tuned by the cross-validation. Accuracy for the training samples, which was evaluated by the cross-validation, was 87% (76/87). Figure 4 shows the results when the prognosis of the 29 test samples was predicted by the GP classifier. *F*-value and accuracy were 0.80 and 93%, respectively. Except for S113 (posterior: 0.32; stage 4, 22-month-old, single copy of *MYCN,* low *TrkA*, dead 12 months after diagnosis) and S081 (posterior: 0.86; stage 3, 6-month-old, single copy of *MYCN,* low *TrkA*, alive 62 months after diagnosis), the prognosis for all the test samples was correctly predicted (27/29, 93%).

Figure 5 shows survival curves for the patients with posterior<0.5 (favorable) and posterior>0.5 (unfavorable) according to the GP classifier. The 5-year survival rate of the former is 90%, whereas that of the latter 23% ($p<10^{-5}$). To further evaluate the efficiency of our system, the posterior value was calculated for the intermediate subset of neuroblastoma (stage 3 or 4, without amplification of *MYCN*) whose prognosis is usually difficult to be predicted. As shown in Figure 4B, the survival curves were significantly segregated into two groups. The 5-year survival rate of the patients with posterior<0.5 was 86%, while that of the patients with posterior>0.5 was 40% ($p<10^{-5}$). These results suggest that the posterior value obtained by our supervised classifier is able to classify the outcome of neuroblastomas with high efficiency, even of the intermediate type of the tumors.

1-4. Leave-one-out analysis

To evaluate how useful the posterior value is for predicting the prognosis as compared with the other conventional markers, the inventors introduced the leave-one-out cross-validation method to the predicted prognosis of all 116 patients. Figure 7 shows the receiver operating characteristics (ROC) curve which indicates performance of each or combination of the GP classifier and the other clinical as well as molecular prognostic factors (age, stage, TrkA expression, *MYCN* amplification, DNA ploidy, and the tumors found by mass screening) in the two-dimensional plane of sensitivity (the rate of correct prediction among alive samples) and specificity (the rate of correct prediction among dead samples). The markers are good to predict the outcome at either high sensitivity or high specificity. In good accordance with the previous reports, age (less than one-year-old), stages (1, 2 and 4s), high *TrkA* expression, hyperdiploidy (aneuploidy), and the tumors found by mass screening showed high sensitivities of 80%, 97%, 97%, 92%, and 93%, respectively, whereas their specificities were 76%, 69%, 66%, 37%, and 58%, respectively. On the other hand, *MYCN* amplification showed 72% sensitivity and 97% specificity. In comparison to these conventional markers, prediction by the GP classifier exhibited good balance between sensitivity (96%) and specificity (90%), and totally it is superior to the other markers. Moreover, the combination of supervised classification and three typical prognostic markers (age, stage and *MYCN* amplification) has achieved as much as 92% sensitivity and 96% specificity.

1-5. Clustering analysis

To assess the relationship between the clinically defined subsets of neuroblastoma and expression of the 70 genes selected as top-scored based on the pair-wise correlation method, the inventors performed an unsupervised clustering analysis in the kernel space (Figures 8 and 9). For better understanding of the results, the inventors introduced Brodeur's classification of neurblastoma subsets: type I (stages 1, 2 or 4s, a single copy of *MYCN*; blue marks in Figures 3, 4, 8 and 9), type II (stage 3 or 4, a single copy of *MYCN*; green marks in Figures 3, 4, 8 and 9), and type III (all stages, amplification of *MYCN*; red marks in Figures 3, 4, 8 and 9) (Brodeur et al., 199?). Figure 8 shows that many of the type III tumors were clustered in a group with highly expressed genes in about a half of 70 (gene group UF, as the gene group strongly correlated with unfavorable prognosis, see below) and lowly expressed genes in the rest half (gene group F, as the gene group, strongly correlated with favorable prognosis, see below). On the other hand, type I tumors formed a broad expression pattern with heterogeneous gene clusters. Interestingly, type II tumors were not uniformly clustered but distributed among the types I and III tumors. To further understand from the clinical point of view, the unsupervised clustering was reorganized according to each type (Figure 9). Intriguingly, a part of the type II tumors of the patients with poor prognosis showed a similar expression pattern to that of the type III and many of them were dead. On the other hand, expression profiles of the rest of the type II tumors seemed to be heterogeneous similarly to those of the type I tumors with favorable outcome. Most of the tumors with high expression of *TrkA* and hyperdiploidy as well as the mass screening tumors were included in the latter group. Thus, the tumors in the type II intermediate group were roughly segregated into two subgroups with favorable and unfavorable prognosis. The fact that the clustering pattern in Figures 8 and 9 is rather complex may also support the fact that our prognostic prediction is based on the decision by majority of the selected genes.

Table 2 shows the list of the 70 top-scored genes and their *p*-values of the log-rank test. The gene with the highest score was *tubulin alpha* (*TUBA1*). Based on the above clustering, the 70 genes were segregated into two groups (group F and group UF) (Figures 8 and 9, and Table 2). The genes in group F had a tendency to show high levels of expression in the type I tumors, whereas those in group UF were expressed at high levels in the type III tumors. The differential expression of those genes between the subsets of neuroblastoma was further confirmed by semi-quantitative RT-PCR (a part of the results were reported in Ohira et al., 2003a: non-patent document 19). The genes in group F contained those related to neuronal differentiation [*tubulin alpha*, *peripherin*, *HMP19,* and *neuromodulin* (*GAP43*), etc.] and those related to catecholamine metabolism [*tyrosine hydroxylase* (*TH*) and dopa *decarboxylase* (*DDC*)]. On the other hand, the genes in group UF involved many members of the genes related to protein synthesis (ribosomal protein genes, elongation factor genes *EEF1A, G*, and *EIF3S5,* etc.) and those related to metabolism [*nucleophosmin*, *enolase 1* (*ENO1*), and *transketolase* (*TKT*), etc.]. *MYCN* gene was also a member of group UF as expected. The very high levels of expression of *MYCN* and *DDX-1*, both of which are frequently co-amplified, were found in the type III tumors with poor prognosis. The *p*-values of the log-rank test in 24 out of 33 genes in group F and those in 30 of 37 genes in group UF were less than 0.05, indicating that all of the 54 genes with a significant *p*-value can be the independent prognostic factors of primary neuroblastomas.

## Table 2

The 70 genes selected by the Gaussian-kernel GP classifier

| | Gene Code | Gene Name | Accession No. | Definition | log rank P-value |
|---|---|---|---|---|---|
| Group F | gene071 | TH | NM_000360 | tyrosine hydroxylase | <0.001 |
| | Nbla21270 | AK095244 | AK095244 | EST | <0.001 |
| | Nbla11788 | PRPH | NM_006262 | peripherin | 0.022 |
| | Nbla20490 | PRPH? | NM_006262 | 5'upstream region of peripherin | 0.004 |
| | Nbla03145 | ECEL1 | NM_004826 | endothelin converting enzyme like 1 | 0.227 |
| | Nbla20713 | HAND2 | NM_021973 | basic helix-loop-helix transcription factor HAND2 | 0.473 |
| | Nbla00269 | DBH | NM_000787 | dopamine beta-hydroxylase type a (EC 1.14.17.1). | 0.935 |
| | Nbla00487 | FLJ13158 | NM_024909 | EST | 0.603 |
| | Nbla00579 | VAT1 | NM_006373 | synaptic vesicle membrane protein VAT1 | 0.924 |
| | Nbla22186 | CLSTN1 | NM_014944 | calsyntenin 1 | 0.814 |
| | Nbla10856 | U5-100K | NM_004818 | U5 snRNP 100 kD protein | 0.865 |
| | Nbla04134 | MBC2 | NM_015292 | membrane bound C2 domain containing protein | 0.439 |
| | Nbla21394 | CHRNA3 | NM_000743 | neuronal acetylcholine receptor protein alpha-3 | 0.066 |
| | Nbla20089 | SEC23B | NM_032986 | protein transport protein SEC23B | 0.003 |
| | Nbla21844 | LOC82906 | NM_138394 | EST | <0.001 |
| | Nbla10093 | HADHB | NM_000183 | mitochondrial 3-ketoacyl-CoA thiolase beta-subunit of trifunctional protein | <0.001 |
| | Nbla00280 | TUBA1 | NM_006000 | tubulin alpha-1 | <0.001 |
| | Nbla23526 | MORF4L2 | NM_012286 | mortality factor 4 like 2 | <0.001 |
| | Nbla03499 | GNB1 | NM_002074 | guanine nucleotide binding protein beta 1 | 0.027 |
| | Nbla22572 | DDC | NM_000790 | dopa decarboxylase | 0.002 |
| | Nbla21891 | VPS41 | NM_014396 | vacuolar protein sorting 41 | <0.001 |
| | Nbla11606 | TUBA3 | NM_006009 | tubulin alpha 3 | <0.001 |
| | Nbla00890 | ARHGEF7 | NM_145735 | Rho guanine nucleotide exchange factor 7 | <0.001 |
| | Nbla11993 | NMP19 | NM_016880 | neuron specific protein family member 2 | <0.001 |
| | Nbla22531 | GAP43 | NM_002045 | neuronal growth associated protein 43 | <0.001 |
| | Nbla03873 | RTN3 | NM_006054 | reticulon 3, neuroendocrine-specific protein | <0.001 |
| | Nbla04181 | AK055112 | NM_032010 | EST | <0.001 |
| | Nbla03767 | MGC8721 | NM_016127 | EST | <0.001 |
| | Nbla11652 | YWHAE | NM_006761 | 14-3-3 epsilon | <0.001 |
| | Nbla10296 | DCTN2 | NM_006400 | dynactin complex 50 kD subunit | <0.001 |
| | Nbla00578 | AF1Q | NM_006818 | ALL1-fused gene from chromosome 1q | 0.002 |
| | Nbla00391 | AF036613 | AF036613 | general transcription factor 2-I | <0.001 |
| | Nbla04023 | RGS5 | NM_003617 | regulator of G-protein signaling 5 | 0.020 |
| Group UF | Nbla11890 | EEF1A1 | NM_001402 | eukaryotic translation elongation factor 1 alpha | 0.634 |
| | Nbla23163 | EIF3S5 | NM_003754 | eukaryotic translation initiation factor 3, subunit 5 | 0.015 |
| | Nbla10054 | NPM1 | NM_002520 | nucleophosmin | 0.079 |
| | Nbla04200 | RPL4 | NM_000968 | ribosomal protein L4 | 0.196 |
| | Nbla11970 | HNRPA1 | NM_031157 | heterogeneous nuclear ribonucleoprotein A1 | 0.036 |
| | Nbla00259 | RPS6 | NM_001010 | ribosomal protein S6 | 0.168 |
| | Nbla03925 | LAMR1 | NM_002295 | laminin receptor 1 | 0.266 |
| | Nbla00139 | RPS13 | NM_001017 | ribosomal protein S13 | 0.002 |
| | Nbla11732 | RPL5 | NM_000969 | ribosomal protein L5 | 0.016 |
| | Nbla03836 | RPL7A | NM_000972 | ribosomal protein L7a | 0.444 |
| | Nbla10579 | AF432211 | NM_014635 | KIAA0336 | <0.001 |
| | Nbla23424 | no hit | no hit | no hit | <0.001 |
| | Nbla00332 | EEF1G | NM_001404 | eukaryotic translation elongation factor 1 | <0.001 |
| | Nbla03266 | GK001 | NM_020198 | GK001 protein | <0.001 |
| | Nbla00013 | GNB2L1 | NM_006098 | guanine nucleotide binding protein, receptor of activated protein kinase C 1 | <0.001 |
| | Nbla00764 | RPLP1 | NM_001003 | ribosomal protein P1 | <0.001 |
| | Nbla00214 | RPL18A | NM_000980 | ribosomal protein L18a | <0.001 |
| | gene052 | MYCN | NM_005378 | N-myc proto-oncogene protein. | <0.001 |
| | gene053 | MYCN | NM_005378 | N-myc proto-oncogene protein. | <0.001 |
| | Nbla00601 | no hit | NM_000983 | ribosomal protein L57 | <0.001 |
| | Nbla11459 | DDX1 | NM_004939 | DEAD/H (Asp-Glu-Ala-Asp/His) box polypeptide 1 | <0.001 |
| | Nbla00500 | PRSS15 | NM_004793 | protease, serine, 15 | <0.001 |
| | Nbla22643 | FLJ20190 | NM_017706 | hypothetical protein FLJ20190 | <0.001 |
| | Nbla10395 | PCOLCE | NM_002593 | procollagen C-endopeptidase enhancer | <0.001 |
| | Nbla10393 | SHMT2 | NM_005412 | serine hydroxymethyltransferase 2 | <0.001 |
| | Nbla00359 | MAD1L1 | NM_003550 | MAD1 mitotic arrest deficient-like 1 | <0.001 |
| | Nbla22564 | AHCY | NM_006621 | S-adenosylhomocysteine hydrolase-like 1 | <0.001 |
| | Nbla10302 | ENO1 | NM_001428 | enolase 1, (alpha) | <0.001 |
| | Nbla04293 | PKM2 | NM_002654 | pyruvate kinase, M1 isozyme | <0.001 |
| | Nbla00781 | TKT | NM_001064 | transketolase | <0.001 |
| | Nbla23007 | FKBP10 | NM_021939 | FK506 binding protein 10 | <0.001 |
| | Nbla21322 | GPI | NM_000175 | glucose phosphate isomerase | <0.001 |
| | Nbla11536 | SLC3A2 | NM_002394 | solute carrier family 3 member 2 | <0.001 |
| | Nbla12163 | BSG | NM_001728 | basigin long isoform | <0.001 |
| | Nbla10873 | TRIM28 | NM_005762 | tripartite motif-containing 28 protein | <0.001 |
| | Nbla00004 | RNU2 | BC003629 | small nuclear RNA U2 | 0.003 |
| | Nbla03362 | no hit | no hit | no hit | 0.277 |

3. Discussion

The experimental study demonstrates that the microarray classifier has the best balance between sensitivity (96%) and specificity (90%) among the prognostic factors for predicting the outcome of neuroblastoma. In addition, when it is combined with age at diagnosis, disease stage and MYCN amplification, all of which are currently used as diagnostic tools at the bedside, the specificity can be increased up to 96%. Furthermore, the intermediate subset of neuroblastomas (type II), which are usually difficult to predict the long term outcome, have also been segregated by the microarray into the groups with favorable and unfavorable prognosis.

As far as the inventors know, there have been only several reports of microarray analysis to predict the cancer prognosis in a similar way to this report. van't Veer et al. (Nature 415, 530-6 (2002)) have recently applied supervised classification to a breast cancer signature predictive of a short interval to distant metastases in the 78 patients initially without local lymph node metastasis. Their cross-validation analysis chose 70 genes as a classifier which predicted correctly the actual outcome of disease for 65 out of the 78 patients (83%). Singh et al. (Cancer Cell 1, 203-9 (2002)) used microarray expression analysis for determining genes predictive of the prognosis of prostate cancers using 52 patients. While no single gene was statistically correlated with recurrence, a 5-gene model with 2 nearest neighbors reached 90% accuracy in predicting recurrence during leave-one-out cross-validation. Ye et al. (Nat Med 9, 416 - 23 (2003)) also predicted metastasis and survival of hepatocellular carcinoma using metastasis predictor model with 20 samples for training and the other 20 for testing. Their supervised machine learning algorithm identified 153 significant genes. These reports have suggested the feasibility of microarray as a diagnostic tool in the clinic in some focused issues such as metastasis or recurrence. In contrast to these analyses, in the present study, the inventors have not selected the tumor subsets but included all 136 tumor samples randomly picked up from the tissue bank which have been collected from the hospitals all over Japan and treated under the control of therapeutic protocols proposed by the group study. The accuracy by the GP classifier determined 70 genes as the best number by the cross-validation technique. When the 87 training samples are evaluated by the cross-validation, the accuracy is 87%. More strikingly, the prognosis for the 29 new test samples is correctly predicted by 93% (27/29) that is extremely high as compared with those reported previously (van 't Veer et al., 2002; Singh et al., 2002; Ye et al., 2003). One of the two tumors apparently misdiagnosed (S081 in Figure 3) shows the posterior value of 0.86 but the patient is alive for 62 months after diagnosis. However, since the primary tumor of this patient is in stage 3 and shows low levels of *TrkA* expression, it may still have a possibility to recur after a further long time follow-up. In addition to the high accuracy, the method of this invention has a practical advantage to choose a suitable therapeutic protocol. In fact, the outcome prediction is almost perfect when the posterior value is large enough (unfavorable) or small enough (favorable) (Figures 3 and 4). Moreover, it is found that the probabilistic output by the GP classifier, as posterior, is very stable under the existence of noise. Even when artificial noise whose variance is as large as the estimated noise variance of microarray is added to the expression profile data, the prognosis prediction does not degrade very much (Figure 13). This robustness is confirmed when the noise variance goes up to 1.0 which is larger enough than the actual reproduction noise level 0.6 (Figures 10-12). Although the prediction confidence, represented by the posterior, decays as the noise level increases, this feature is suitable for clinical applications, because the uncertain prediction reflects the large noise possibly involved on the microarray. Thus, the present results suggest that the microarray system in this invention is extremely powerful to predict the prognosis of neuroblastoma.

The high outcome predictability of the system in this invention may be due to multiple reasons. The quality of the tumor samples is high since the system of neuroblastoma tissue bank has been established and handling of tumor tissues is rather uniform in every hospital with obtaining informed consent. The array with application of a new apparatus installed a piezo micro ceramic pump, gives highly quantitative as well as reproducible signals. The non-contact spotting method makes the spot shape almost a perfect circle. As a result, the spot excels in signal uniformity. In addition, the inventors introduced kernel-based supervised classification and selected top-scored 70 genes to predict the prognosis by decision of majority, or vote. The two-fold feature extraction, the gene selection based on the pair-wise correlation method and extracting the low-dimensional gene expression similarity by the Gaussian kernel, makes the classifier robust against noise involved in the test samples. Though the inventors did not perform microdissection of the parts of the tumor, it is already known that, in neuroblastoma, the stromal components such as Schwannian cells are very important to characterize the tumor's biology (for review, see Ambros, I. M. & Ambros, P. F. Eur J Cancer 4, 429 - 34 (1995) ; Ambros, I. M. & Ambros, P. F. Neuroblastoma, 229 - 243 (2000)). Thus, a good combination or choice of those procedures may have given a high level of the outcome predictability.

The gene with the highest score is *tubulin alpha (TUBA1),* which has never been reported as a prognostic factor in neuroblastoma. Its prognostic significance has also been confirmed by RT-PCR in primary tumors. High expression of *TUBA1* in neuronal cells is associated with axonal outgrowth during development as well as axonal degeneration after axotomy in adult animal (Knoops, B. & Octave, J. N. Neuroreport 8, 795-8 (1997)). Its family gene, *TUBA3*, is also ranked in the top 70. Expression of *TUBA3* is reported to be restricted to the adherent, morphologically differentiated neuronal and glial cells (Hall, J. L. & Cowan, N. J. Nucleic Acid Res 13, 207-23 (1985)). *DDX1* gene, which is frequently co-amplified with *MYCN* in advanced neuroblastomas (Godbout, R. & Squire, J. Proc Natl Acad Sci USA 90, 7578-82 (1993) ; Noguchi, T. et al. Genes Chromosomes Cancer 15, 129-33 (1996)), is also ranked at higher score than *MYCN.* This may be concordant with the previous reports that *MYCN* mRNA expression is a weaker prognostic marker than its genomic amplification (Slavc, I. et al. Cancer Res 50, 1459-63 (1990)). The another important prognostic factor, *TrkA*, is not included in the top 70 genes but in the 120, probably because of its relatively low levels of mRNA expression as compared with those of the other genes. The prognostic influence of *TrkA* expression may be compensated by the other genes affected or regulated by a *TrkA* intracellular signaling. Notably, the log-rank test of each gene shows that 54 out of 70 genes have the *p*-value with less than 0.05 on the microarray when used the 136 primary neuroblastomas (Table 1) indicating that the inventors have identified a large number of genes which can be significant predictors of the outcome. Indeed, the significance of most of those genes as prognostic factors has been confirmed by using semi-quantitative RT-PCR. As for the expression profile of the 70 genes, it is relatively heterogeneous, since the inventors have chosen them by supervised classification but not by the pattern of expression profiling. Nevertheless, the poor-prognostic tumors show a typical pattern of differential expression in the selected genes. Of interest, a part of the intermediate type of neuroblastomas with poor outcome also shows a similar pattern, suggesting that the tumors with aggressive potential can be predictive. On the other hand, the clustering pattern of neuroblastomas in favorable stages is rather heterogeneous, which may be due to the mixed populations with different stages of differentiation and programmed cell death of the tumor cells.

The ROC curves (Figure 7) clearly show that microarray alone can be the most powerful prognostic indicator among the prognostic factors. Furthermore, they have shown that the combination of microarray with age, stage and *MYCN* amplification should give a confident prediction of prognosis in neuroblastoma at the bedside. The posterior value will help the decision of therapeutic way, and the outcome prediction based on the posterior value is extremely robust to possible noise. Thus, application of the highly qualified cDNA microarray into the clinic may give a reality leading to a tailored medicine to enable better treatment of the cancer patients.

**Industrial Applicability**

As explained in detail above, according to the invention of this application, it becomes possible to predict the postoperative prognosis of a patient with neuroblastoma with extreme convenience and high accuracy. An accurate prediction will be able to eliminate excess medical treatment for a good prognosis patient, and to give sufficient medical treatment to a patient who is suspected of poor prognosis. Therefore, the invention of this application is extremely useful in industrial fields related to medical practices.

SEQUENCE LISTING

SEQ ID NO: 2
<211> 2458
<212> DNA
<213> Homo sapiens

SEQ ID NO: 2

```
ctccggtgtg tctgtcggtt gcagtgttgg aggtcggcgc cggcccccgc cttccgcgcc      60
ccccacggga aggaagcacc cccggtatta aaacgaacgg ggcggaaaga agccctcagt     120
cgccggccgg gaggcgagcc gatgccgagc tgctccacgt ccaccatgcc gggcatgatc     180
tgcaagaacc cagacctcga gtttgactcg ctacagccct gcttctaccc ggacgaagat     240
gacttctact tcggcggccc cgactcgacc cccccggggg aggacatctg gaagaagttt     300
gagctgctgc ccacgccccc gctgtcgccc agccgtggct tcgcggagca cagctccgag     360
cccccgagct gggtcacgga gatgctgctt gagaacgagc tgtggggcag cccggccgag     420
gaggacgcgt tcggcctggg gggactgggt ggcctcaccc ccaacccggt catcctccag     480
gactgcatgt ggagcggctt ctccgcccgc gagaagctgg agcgcgccgt gagcgagaag     540
ctgcagcacg gccgcgggcc gccaaccgcc ggttccaccg cccagtcccc gggagccggc     600
gccgccagcc ctgcgggtcg cgggcacggc ggggctgcgg gagccggccg cgccggggcc     660
gccctgcccg ccgagctcgc ccacccggcc gccgagtgcg tggatcccgc cgtggtcttc     720
ccctttcccg tgaacaagcg cgagccagcg cccgtgcccg cagccccggc cagtgccccg     780
gcggcgggcc ctgcggtcgc ctcgggggcg ggtattgccg ccccagccgg ggccccgggg     840
gtcgcccctc cgcgcccagg cggccgccag accagcggcg gcgaccacaa ggccctcagt     900
acctccggag aggacaccct gagcgattca gatgatgaag atgatgaaga ggaagatgaa     960
gaggaagaaa tcgacgtggt cactgtggag aagcggcgtt cctcctccaa caccaaggct    1020
gtcaccacat tcaccatcac tgtgcgtccc aagaacgcag ccctgggtcc cgggagggct    1080
cagtccagcg agctgatcct caaacgatgc cttcccatcc accagcagca caactatgcc    1140
gccccctctc cctacgtgga gagtgaggat gcacccccac agaagaagat aaagagcgag    1200
gcgtccccac gtccgctcaa gagtgtcatc cccccaaagg ctaagagctt gagcccccga    1260
```

aactctgact cggaggacag tgagcgtcgc agaaaccaca acatcctgga gcgccagcgc 1320

cgcaacgacc ttcggtccag ctttctcacg ctcagggacc acgtgccgga gttggtaaag 1380

aatgagaagg ccgccaaggt ggtcattttg aaaaaggcca ctgagtatgt ccactccctc 1440

caggccgagg agcaccagct tttgctggaa aaggaaaaat tgcaggcaag acagcagcag 1500

ttgctaaaga aaattgaaca cgctcggact tgctagacgc ttctcaaaac tggacagtca 1560

ctgccacttt gcacattttg attttttttt taaacaaaca ttgtgttgac attaagaatg 1620

ttggtttact ttcaaatcgg tcccctgtcg agttcggctc tgggtgggca gtaggaccac 1680

cagtgtgggg ttctgctggg accttggaga gcctgcatcc caggatgctg ggtggccctg 1740

cagcctcctc cacctcacct ccatgacagc gctaaacgtt ggtgacggtt gggagcctct 1800

ggggctgttg aagtcacctt gtgtgttcca agtttccaaa caacagaaag tcattccttc 1860

tttttaaaat ggtgcttaag ttccagcaga tgccacataa ggggtttgcc atttgatacc 1920

cctggggaac atttctgtaa ataccattga cacatccgcc ttttgtatac atcctgggta 1980

atgagaggtg gcttttgcgg ccagtattag actggaagtt catacctaag tactgtaata 2040

atacctcaat gtttgaggag catgttttgt atacaaatat attgttaatc tctgttatgt 2100

actgtactaa ttcttacact gcctgtatac tttagtatga cgctgataca taactaaatt 2160

tgatacttat attttcgtat gaaaatgagt tgtgaaagtt ttgagtagat attactttat 2220

cactttttga actaagaaac ttttgtaaag aaatttacta tatatatatg ccttttttcct 2280

agcctgtttc ttcctgttaa tgtatttgtt catgtttggt gcatagaact gggtaaatgc 2340

aaagttctgt gtttaatttc ttcaaaatgt atatatttag tgctgcatct tatagcactt 2400

tgaaatacct catgtttatg aaaataaata gcttaaaatt aaaaaaaaaa aaaaaaaa 2458

SEQ ID NO: 3
<211> 2458
<212> DNA
<213> Homo sapiens

SEQ ID NO: 3

ctccggtgtg tctgtcggtt gcagtgttgg aggtcggcgc cggcccccgc cttccgcgcc 60

```
ccccacggga aggaagcacc cccggtatta aaacgaacgg ggcggaaaga agccctcagt    120
cgccggccgg gaggcgagcc gatgccgagc tgctccacgt ccaccatgcc gggcatgatc    180
tgcaagaacc cagacctcga gtttgactcg ctacagccct gcttctaccc ggacgaagat    240
gacttctact tcggcggccc cgactcgacc cccccggggg aggacatctg gaagaagttt    300
gagctgctgc ccacgccccc gctgtcgccc agccgtggct tcgcggagca cagctccgag    360
cccccgagct gggtcacgga gatgctgctt gagaacgagc tgtggggcag cccggccgag    420
gaggacgcgt tcggcctggg gggactgggt ggcctcaccc ccaacccggt catcctccag    480
gactgcatgt ggagcggctt ctccgcccgc gagaagctgg agcgcgccgt gagcgagaag    540
ctgcagcacg gccgcgggcc gccaaccgcc ggttccaccg cccagtcccc gggagccggc    600
gccgccagcc ctgcgggtcg cgggcacggc ggggctgcgg gagccggccg cgccggggcc    660
gccctgcccg ccgagctcgc ccacccggcc gccgagtgcg tggatcccgc cgtggtcttc    720
ccctttcccg tgaacaagcg cgagccagcg cccgtgcccg cagccccggc cagtgccccg    780
gcggcgggcc ctgcggtcgc ctcgggggcg ggtattgccg ccccagccgg ggccccgggg    840
gtcgcccctc cgcgcccagg cggccgccag accagcggcg gcgaccacaa ggccctcagt    900
acctccggag aggacaccct gagcgattca gatgatgaag atgatgaaga ggaagatgaa    960
gaggaagaaa tcgacgtggt cactgtggag aagcggcgtt cctcctccaa caccaaggct   1020
gtcaccacat tcaccatcac tgtgcgtccc aagaacgcag ccctgggtcc cgggagggct   1080
cagtccagcg agctgatcct caaacgatgc cttcccatcc accagcagca caactatgcc   1140
gccccctctc cctacgtgga gagtgaggat gcacccccac agaagaagat aaagagcgag   1200
gcgtccccac gtccgctcaa gagtgtcatc cccccaaagg ctaagagctt gagcccccga   1260
aactctgact cggaggacag tgagcgtcgc agaaaccaca acatcctgga gcgccagcgc   1320
cgcaacgacc ttcggtccag ctttctcacg ctcagggacc acgtgccgga gttggtaaag   1380
aatgagaagg ccgccaaggt ggtcattttg aaaaaggcca ctgagtatgt ccactccctc   1440
caggccgagg agcaccagct tttgctggaa aaggaaaaat tgcaggcaag acagcagcag   1500
ttgctaaaga aaattgaaca cgctcggact tgctagacgc ttctcaaaac tggacagtca   1560
```

ctgccacttt gcacattttg attttttttt taaacaaaca ttgtgttgac attaagaatg 1620

ttggtttact ttcaaatcgg tcccctgtcg agttcggctc tgggtgggca gtaggaccac 1680

cagtgtgggg ttctgctggg accttggaga gcctgcatcc caggatgctg ggtggccctg 1740

cagcctcctc cacctcacct ccatgacagc gctaaacgtt ggtgacggtt gggagcctct 1800

ggggctgttg aagtcacctt gtgtgttcca agtttccaaa caacagaaag tcattccttc 1860

tttttaaaat ggtgcttaag ttccagcaga tgccacataa ggggtttgcc atttgatacc 1920

cctggggaac atttctgtaa ataccattga cacatccgcc ttttgtatac atcctgggta 1980

atgagaggtg gcttttgcgg ccagtattag actggaagtt catacctaag tactgtaata 2040

atacctcaat gtttgaggag catgttttgt atacaaatat attgttaatc tctgttatgt 2100

actgtactaa ttcttacact gcctgtatac tttagtatga cgctgataca taactaaatt 2160

tgatacttat attttcgtat gaaaatgagt tgtgaaagtt ttgagtagat attactttat 2220

cactttttga actaagaaac ttttgtaaag aaatttacta tatatatatg ccttttttcct 2280

agcctgtttc ttcctgttaa tgtatttgtt catgtttggt gcatagaact gggtaaatgc 2340

aaagttctgt gtttaatttc ttcaaaatgt atatatttag tgctgcatct tatagcactt 2400

tgaaatacct catgtttatg aaaataaata gcttaaaatt aaaaaaaaaa aaaaaaaa 2458

SEQ ID NO: 5
<211> 1838
<212> DNA
<213> Homo sapiens

SEQ ID NO: 5

cggacctcca cactgagcca tgcccacccc cgacgccacc acgccacagg ccaagggctt 60

ccgcagggcc gtgtctgagc tggacgccaa gcaggcagag gccatcatgt ccccgcggtt 120

cattgggcgc aggcagagcc tcatcgagga cgcccgcaag gagcgggagg cggcggtggc 180

agcagcggcc gctgcagtcc cctcggagcc cggggacccc ctggaggctg tggcctttga 240

ggagaaggag gggaaggccg tgctaaacct gctcttctcc ccgagggcca ccaagccctc 300

ggcgctgtcc cgagctgtga aggtgtttga gacgtttgaa gccaaaatcc accatctaga 360

gacccggccc gcccagaggc cgcgagctgg gggcccccac ctggagtact tcgtgcgcct 420

```
cgaggtgcgc cgaggggacc tggccgccct gctcagtggt gtgcgccagg tgtcagagga   480
cgtgcgcagc cccgcggggc ccaaggtccc ctggttccca agaaaagtgt cagagctgga   540
caagtgtcat cacctggtca ccaagttcga ccctgacctg gacttggacc acccgggctt   600
ctcggaccag gtgtaccgcc agcgcaggaa gctgattgct gagatcgcct tccagtacag   660
gcacggcgac ccgattcccc gtgtggagta caccgccgag gagattgcca cctggaagga   720
ggtctacacc acgctgaagg gcctctacgc cacgcacgcc tgcggggagc acctggaggc   780
ctttgctttg ctggagcgct tcagcggcta ccgggaagac aatatccccc agctggagga   840
cgtctcccgc ttcctgaagg agcgcacggg cttccagctg cggcctgtgg ccggcctgct   900
gtccgcccgg gacttcctgg ccagcctggc cttccgcgtg ttccagtgca cccagtatat   960
ccgccacgcg tcctcgccca tgcactcccc tgagccggac tgctgccacg agctgctggg  1020
gcacgtgccc atgctggccg accgcacctt cgcgcagttc tcgcaggaca ttggcctggc  1080
gtccctgggg gcctcggatg aggaaattga gaagctgtcc acgctgtcat ggttcacggt  1140
ggagttcggg ctgtgtaagc agaacgggga ggtgaaggcc tatggtgccg ggctgctgtc  1200
ctcctacggg gagctcctgc actgcctgtc tgaggagcct gagattcggg ccttcgaccc  1260
tgaggctgcg gccgtgcagc cctaccaaga ccagacgtac cagtcagtct acttcgtgtc  1320
tgagagcttc agtgacgcca aggacaagct caggagctat gcctcacgca tccagcgccc  1380
cttctccgtg aagttcgacc cgtacacgct ggccatcgac gtgctggaca gcccccaggc  1440
cgtgcggcgc tccctggagg gtgtccagga tgagctggac acccttgccc atgcgctgag  1500
tgccattggc taggtgcacg gcgtccctga gggcccttcc caacctcccc tggtcctgca  1560
ctgtcccgga gctcaggccc tggtgagggg ctgggtcccg ggtgcccccc atgccctccc  1620
tgctgccagg ctcccactgc ccctgcacct gcttctcagc gcaacagctg tgtgtgcccg  1680
tggtgaggtt gtgctgcctg tggtgaggtc ctgtcctggc tcccagggtc ctgggggctg  1740
ctgcactgcc ctccgccctt ccctgacact gtctgctgcc ccaatcaccg tcacaataaa  1800
agaaactgtg gtctctaaaa aaaaaaaaaa aaaaaaa                           1838
```

SEQ ID NO: 7
<211> 1093

<212> DNA
<213> Homo sapiens

SEQ ID NO: 7

```
ctgcaaggcg gcggcaggag aggttgtggt gctagtttct ctaagccatc cagtgccatc      60
ctcgtcgctg cagcgacacc gctctcgccg ccgccatgac tgagcagatg acccttcgtg     120
gcaccctcaa gggccacaac ggctgggtaa cccagatcgc tactaccccg cagttcccgg     180
acatgatcct ctccgcctct cgagataaga ccatcatcat gtggaaactg accagggatg     240
agaccaacta tggaattcca cagcgtgctc tgcggggtca ctcccacttt gttagtgatg     300
tggttatctc ctcagatggc cagtttgccc tctcaggctc ctgggatgga accctgcgcc     360
tctgggatct cacaacgggc accaccacga ggcgatttgt gggccatacc aaggatgtgc     420
tgagtgtggc cttctcctct gacaaccggc agattgtctc tggatctcga gataaaacca     480
tcaagctatg gaataccctg ggtgtgtgca aatacactgt ccaggatgag agccactcag     540
agtgggtgtc ttgtgtccgc ttctcgccca acagcagcaa ccctatcatc gtctcctgtg     600
gctgggacaa gctggtcaag gtatggaacc tggctaactg caagctgaag accaaccaca     660
ttggccacac aggctatctg aacacggtga ctgtctctcc agatggatcc ctctgtgctt     720
ctggaggcaa ggatggccag gccatgttat gggatctcaa cgaaggcaaa cacctttaca     780
cgctagatgg tggggacatc atcaacgccc tgtgcttcag ccctaaccgc tactggctgt     840
gtgctgccac aggccccagc atcaagatct gggatttaga gggaaagatc attgtagatg     900
aactgaagca agaagttatc agtaccagca gcaaggcaga accaccccag tgcacttccc     960
tggcctggtc tgctgatggc cagactctgt ttgctggcta cacggacaac ctggtgcgag    1020
tgtggcaggt gaccattggc acacgctaga agtttatggc agagctttac aaataaaaaa    1080
aaaatggctt ttc                                                       1093
```

SEQ ID NO: 11
<211> 540
<212> DNA
<213> Homo sapiens

SEQ ID NO: 11

gatcgccgcc atcatgggtc gcatgcatgc tcccgggaag ggcctgtccc agtcggcttt 60

accctatcga cgcagcgtcc ccacttggtt gaagttgaca tctgacgacg tgaaggagca 120

gatttacaaa ctggccaaga agggccttac tccttcacag atcggtgtaa tcctgagaga 180

ttcacatggt gttgcacaag tacgttttgt gacaggcaat aaaattttaa gaattcttaa 240

gtctaaggga cttgctcctg atcttcctga agatctctac catttaatta agaaagcagt 300

tgctgttcga aagcatcttg agaggaacag aaaggataag gatgctaaat tccgtctgat 360

tctaatagag agccggattc accgtttggc tcgatattat aagaccaagc gagtcctccc 420

tcccaattgg aaatatgaat catctacagc ctctgccctg gtcgcataaa tttgtctgtg 480

tactcaagca ataaaatgat tgtttaacta aaaaaaaaaa aaaaaaaaaa aaaaaaaaaa 540

SEQ ID NO: 13
<211> 616
<212> DNA
<213> Homo sapiens

SEQ ID NO: 13

cgaacgcgga gagcacgcca tgaaggcctc gggcacgcta cgagagtaca aggtagtggg 60

tcgctgcctg cccacccccca aatgccacac gccgcccctc taccgcatgc gaatctttgc 120

gcctaatcat gtcgtcgcca agtcccgctt ctggtacttt gtatctcagt taaagaagat 180

gaagaagtct tcaggggaga ttgtctactg tgggcaggtg tttgagaagt ccccccctgcg 240

ggtgaagaac ttcgggatct ggctgcgcta tgactcccgg agcggcaccc acaacatgta 300

ccgggaatac cgggacctga ccaccgcagg cgctgtcacc cagtgctacc gagacatggg 360

tgcccggcac cgcgcccgag cccactccat tcagatcatg aaggtggagg agatcgcggc 420

cagcaagtgc cgccggccgg ctgtcaagca gttccacgac tccaagatca agttcccgct 480

gccccaccgg gtcctgcgcc gtcagcacaa gccacgcttc accaccaaga ggcccaacac 540

cttcttctag gtgcagggcc ctcgtccggg tgtgccccaa ataaactcag gaacgccaaa 600

aaaaaaaaaa aaaaaa 616

SEQ ID NO: 16
<211> 1596
<212> DNA
<213> Homo sapiens

SEQ ID NO: 16

```
tgtcggggac ggtaaccggg acccgtgctc tgctcctgtc gccttcgcct cctgaatccc   60
tagccatatg cgtgagtgca tctccatcca cgttggccag gctggtgtcc agattggcaa  120
tgcctgctgg gagctctact gcctggaaca cggcatccag cccgatggcc agatgccaag  180
tgacaagacc attgggggag gagatgactc cttcaacacc ttcttcagtg agacgggcgc  240
tggcaagcac gtgccccggg ctgtgtttgt agacttggaa cccacagtca ttgatgaagt  300
tcgcactggc acctaccgcc agctcttcca ccctgagcag ctcatcacag gcaaggaaga  360
tgctgccaat aactatgccc gagggcacta caccattggc aaggagatca ttgaccttgt  420
gttggaccga attcgcaagc tggctgacca gtgcacccgt cttcagggct tcttggtttt  480
ccacagcttt ggtgggggaa ctggttctgg gttcacctcc ctgctcatgg aacgcctgtc  540
agttgattat ggcaagaaat ccaagctgga gttctccatt tacccggcac cccaggtttc  600
cacagctgta gttgagccct acaactccat cctcaccacc cacaccaccc tggagcactc  660
tgattgtgcc ttcatggtag acaatgaggc catctatgac atctgtcgta gaaacctcga  720
tatcgagcgc ccaacctaca ctaaccttaa ccgccttatt agccagattg tgtcctccat  780
cactgcttcc ctgagatttg atggagccct gaatgttgac ctgacagaat tccagaccaa  840
cctggtcccc tacccccgca tccacttccc tctggccaca tatgcccctg tcatctctgc  900
tgagaaagcc taccatgaac agctttctgt agcagacatc accaatgctt gctttgagcc  960
agccaaccag atggtgaaat gtgaccctgg ccatggtaaa tacatggctt gctgcctgtt 1020
gtaccgtggt gacgtggttc ccaaagatgt caatgctgcc attgccacca tcaaaaccaa 1080
gcgcacgatc cagtttgtgg attggtgccc cactggcttc aaggttggca tcaactacca 1140
gcctcccact gtggtgcctg gtggagacct ggccaaggta cagagagctg tgtgcatgct 1200
gagcaacacc acagccattg ctgaggcctg ggctcgcctg gaccacaagt ttgacctgat 1260
gtatgccaag cgtgcctttg ttcactggta cgtgggtgag ggaatggagg aaggcgagtt 1320
ttcagaggcc cgtgaagata tggctgccct tgagaaggat tatgaggagg ttggtgtgga 1380
```

ttctgttgaa ggagagggtg aggaagaagg agaggaatac taattatcca ttccttttgg 1440

ccctgcagca tgtcatgctc ccagaatttc agcttcagct taactgacag atgttaaagc 1500

tttctggtta gattgttttc acttggtgat catgtctttt ccatgtgtac ctgtaatatt 1560

tttccatcat atctcaaagt aaagtcatta acatca 1596

SEQ ID NO: 18
<211> 1429
<212> DNA
<213> Homo sapiens

SEQ ID NO: 18

cgccgagaac cccacccccct ttctttgcgg aatcaccatg gcggctggga ccctgtacac 60

gtatcctgaa aactggaggg ccttcaaggc tctcatcgct gctcagtaca gcggggctca 120

ggtccgcgtg ctctccgcac caccccactt ccattttggc caaaccaacc gcacccctga 180

atttctccgc aaatttcctg ccggcaaggt cccagcattt gagggtgatg atggattctg 240

tgtgtttgag agcaacgcca ttgcctacta tgtgagcaat gaggagctgc ggggaagtac 300

tccagaggca gcagcccagg tggtgcagtg ggtgagcttt gctgattccg atatagtgcc 360

cccagccagt acctgggtgt tccccacctt gggcatcatg caccacaaca aacaggccac 420

tgagaatgca aaggaggaag tgaggcgaat tctggggctg ctggatgctt acttgaagac 480

gaggactttt ctggtgggcg aacgagtgac attggctgac atcacagttg tctgcaccct 540

gttgtggctc tataagcagg ttctagagcc ttctttccgc caggcctttc ccaataccaa 600

ccgctggttc ctcacctgca ttaaccagcc ccagttccgg gctgtcttgg gcgaagtgaa 660

actgtgtgag aagatggccc agtttgatgc taaaaagttt gcagagaccc aacctaaaaa 720

ggacacacca cggaaagaga agggttcacg ggaagagaag cagaagcccc aggctgagcg 780

gaaggaggag aaaaaggcgg ctgcccctgc tcctgaggag gagatggatg aatgtgagca 840

ggcgctggct gctgagccca aggccaagga ccccttcgct cacctgccca agagtacctt 900

tgtgttggat gaatttaagc gcaagtactc caatgaggac acactctctg tggcactgcc 960

atatttctgg gagcactttg ataaggacgg ctggtccctg tggtactcag agtatcgctt 1020

ccctgaagaa ctcactcaga ccttcatgag ctgcaatctc atcactggaa tgttccagcg 1080

actggacaag ctgaggaaga atgccttcgc cagtgtcatc ctttttggaa ccaacaatag 1140

cagctccatt tctggagtct gggtcttccg aggccaggag cttgcctttc cgctgagtcc 1200

agattggcag gtggactacg agtcatacac atggcggaaa ctggatcctg gcagcgagga 1260

gacccagacg ctggttcgag agtacttttc ctgggagggg gccttccagc atgtgggcaa 1320

agccttcaat cagggcaaga tcttcaagtg aacatctctt gccatcacct agctgcctgc 1380

acctgccctt cagggagatg ggggtcatta aaggaaactg aacattgaa 1429

SEQ ID NO: 20
<211> 2683
<212> DNA
<213> Homo sapiens

SEQ ID NO: 20

tgggcgccgg cggagcggag ggagatccga gcggcggcgg caagctggct gcgagcggct 60

gagcactcca agctcgccgg cctttggtct ccaggacttg tcccagcagc ccctcgaact 120

gagaattaca ccatcggacc cctggctctg aggccttcag acttggactg tgtcacactg 180

ccaggcttcc agggctccaa cttgcagacg gcctgttgtg ggacagtctc tgtaatcgcg 240

aaagcaacca tggaagacct gggggaaaac accatggttt tatccaccct gagatctttg 300

aacaacttca tctctcagcg tgtggaggga ggctctggac tggatatttc tacctcggcc 360

ccaggttctc tgcagatgca gtaccagcag agcatgcagc tggaggaaag agcagagcag 420

atccgttcga agtcccacct catccaggtg gagcgggaga aaatgcagat ggagctgagt 480

cacaagaggg ctcgagtgga gctggagaga gcagccagca ccagtgccag gaactacgag 540

cgtgaggtcg accgcaacca ggagctcctg acgcgcatcc ggcagcttca ggagcgggag 600

gccggggcgg aggagaagat gcaggagcag ctggagcgca acaggcagtg tcagcagaac 660

ttggatgctg ccagcaagag gctgcgtgag aaagaggaca gtctggccca ggctggcgag 720

accatcaacg cactgaaggg gaggatctcg gaactgcagt ggagcgtgat ggaccaggag 780

atgcgggtga agcgcctgga gtcggagaag caggacgtgc aggagcagct ggacctgcaa 840

cacaaaaaat gccaggaagc caatcagaaa atccaggaac tccaggccag ccaagaagca 900

agagcagacc acgagcagca gattaaggat ctggagcaga agctgtccct gcaagagcag 960

gatgcagcga ttgtgaagaa catgaagtct gagctggtac ggctccctag gctggaacgg 1020

gagctggagc agctgcggga ggagagcgcg cacctgcggg agatgagaga gaccaacggg 1080

ctgctccagg aagagctgga agggctgcag aggaagctgg ggcgccagga gaagatgcag 1140

gagacgctgg ttggcttgga gctggagaac gagaggctgc tggccaagct gcaaagctgg 1200

gagagactgg accagaccat gggcctgagc atcaggactc cagaagacct ttccagattc 1260

gtggttgagc tgcagcagag ggagcttgcc ttgaaggaca agaacagcgc cgtcaccagc 1320

agcgcccggg ggctggagaa ggccaggcag cagctgcagg aggagctccg gcaggtcagc 1380

ggccagctgt tggaggagag gaagaagcgc gagacccacg aggcgctggc ccggaggctc 1440

cagaaacggg tcctgctgct caccaaggag cgggacggta tgcgggccat cctggggtcc 1500

tacgacagcg agctgacccc ggccgagtac tcaccccagc tgacgcggcg catgcgggag 1560

gctgaggata tggtgcagaa ggtgcacagc cacagcgccg agatggaggc tcagctgtcg 1620

caggccctgg aggagctggg aggccagaaa caaagagcag acatgctgga gatggagctg 1680

aagatgctga agtctcagtc cagctctgcc gaacagagct tcctgttctc cagggaggag 1740

gcggacacgc tcaggttgaa ggtcgaggag ctggaaggcg agcggagtcg gctggaggag 1800

gaaaagagga tgctggaggc acagctggag cggcgagctc tgcagggtga ctatgaccag 1860

agcaggacca aagtgctgca catgagcctg aaccccacca gtgtggccag gcagcgcctg 1920

cgcgaggacc acagccagct gcaggcggag tgcgagcgac tgcgcgggct cctgcgcgcc 1980

atggagagag gaggcaccgt cccagccgac cttgaggctg ccgccgcgag tctgccatcg 2040

tccaaggagg tggcagagct gaagaagcag gtggagagtg ccgagctgaa gaaccagcgg 2100

ctcaaggagg ttttccagac caagatccag gagttccgca aggcctgcta cacgctcacc 2160

ggctaccaga tcgacatcac cacggagaac cagtaccggc tgacctcgct gtacgccgag 2220

cacccaggcg actgcctcat cttcaaggcc accagcccct cgggttccaa gatgcagcta 2280

ctggagacag agttctcaca caccgtgggc gagctcatcg aggtgcacct gcggcgccag 2340

gacagcatcc ctgccttcct cagctcgctc accctcgagc tcttcagccg ccagaccgtg 2400

gcgtagcctg caggctcggg ggcatagccg gagccactct gcttggcctg acctgcaggt 2460

cccctgcccc gccagccaca ggctgggtgc acgtcctgcc tctccagccc cacagggcag 2520

cagcatgact gacagacacg ctgggaccta cgtcgggctt cctgctgggg cggccagcac 2580

cctctccacg tgcagacccc atgcgtcccg gagcctggtg tgtgggcgtc ggccaccagc 2640

ctgggttcct caccttgtga aataaaatct tctcccctaa aaa 2683

SEQ ID NO: 22
<211> 344
<212> DNA
<213> Homo sapiens

<220>
<221> misc_feature
<222> (187)..(187)
<223> n is a, t, g or c

<220>
<221> misc_feature
<222> (90) .. (90)
<223> n is a, t, g or c

<220>
<221> misc_feature
<222> (252)..(252)
<223> n is a, t, g or c

<220>
<221> misc_feature
<222> (271).. (271)
<223> n is a, t, g or c

<220>
<221> misc_feature
<222> (302)..(302)
<223> n is a, t, g or c

<220>
<221> misc_feature
<222> (322)..(322)
<223> n is a, t, g or c

<220>
<221> misc_feature
<222> (331)..(331)
<223> n is a, t, g or c

<220>
<221> misc_feature
<222> (33)..(33)
<223> n is a, t, g or c

SEQ ID NO: 22

```
agcagttctg aaagtttatt tacacagtaa gtnccgagct agaagtggac agtctccagt      60
cttcctgtca gactctgtag ttatggggtn ttcccagtca gccaaagcag caccaagtgc     120
agtgggaatg aagagaaccc agataaaaaa ctgactcagt attcttgttt tattggttta     180
tggcttntgg gcagctggct gaggccagga acaccagagg ccctccttgt atttccctct     240
ccctgatcag gnaattccac acttagcagg ncccatacct ggaagcagcg ggttgaccag     300
gnaggagtag gatgcagtgg gnatactagg naaacccagc tctt                      344
```

SEQ ID NO: 25
<211> 1033
<212> DNA
<213> Homo sapiens

SEQ ID NO: 25

```
cctagcgccg ctgggcctgc aggtctctgt cgagcagcgg acgccggtct ctgttccgca      60
ggatggggtt tgttaaagtt gttaagaata aggcctactt taagagatac caagtgaaat     120
ttagaagacg acgagagggt aaaactgatt attatgctcg gaaacgcttg gtgatacaag     180
ataaaaataa atacaacaca cccaaataca ggatgatagt tcgtgtgaca aacagagata     240
tcatttgtca gattgcttat gcccgtatag agggggatat gatagtctgc gcagcgtatg     300
cacacgaact gccaaaatat ggtgtgaagg ttggcctgac aaattatgct gcagcatatt     360
gtactggcct gctgctggcc cgcaggcttc tcaataggtt tggcatggac aagatctatg     420
aaggccaagt ggaggtgact ggtgatgaat acaatgtgga aagcattgat ggtcagccag     480
```

gtgccttcac ctgctatttg gatgcaggcc ttgccagaac taccactggc aataaagttt 540

ttggtgccct gaagggagct gtggatggag gcttgtctat ccctcacagt accaaacgat 600

tccctggtta tgattctgaa agcaaggaat ttaatgcaga agtacatcgg aagcacatca 660

tgggccagaa tgttgcagat tacatgcgct acttaatgga agaagatgaa gatgcttaca 720

agaaacagtt ctctcaatac ataaagaaca gcgtaactcc agacatgatg gaggagatgt 780

ataagaaagc tcatgctgct atacgagaga atccagtcta tgaaaagaag cccaagaaag 840

aagttaaaaa gaagaggtgg aaccgtccca aaatgtccct tgctcagaag aaggatcggg 900

tagctcaaaa gaaggcaagc ttcctcagag ctcaggagcg ggctgctgag agctaaaccc 960

agcaattttc tatgattttt tcagatatag ataataaact tatgaacagc aactaaaaaa 1020

aaaaaaaaaa aaa 1033

SEQ ID NO: 26
<211> 3111
<212> DNA
<213> Homo sapiens

SEQ ID NO: 26

gcgagttgtg tgagccgcca gtatggccgg gctatggcgg cgagcactgg ctacgtgcga 60

ctgtggggag cggcgcggtg ctgggtgctg cggcggccga tgctggccgc cgccgggggg 120

cgggttccca ctgcagcagg agcgtggttg ctccgaggcc agcggacctg cgacgcctct 180

cctccttggg cactgtgggg ccgaggcccg gcaattgggg gccaatggcg ggggttttgg 240

gaagcgagca gccgcggcgg aggcgcattc tcgggggcg aggacgcctc cgagggcggc 300

gcggaggaag gagccggcgg cgcgggggc agcgcgggcg ccggggaagg cccggtcata 360

acggcgctca cgcccatgac gatccccgat gtgtttccgc acctgccgct catcgccatc 420

acccgcaacc cggtgttccc gcgctttatc aagattatcg aggttaaaaa taagaagttg 480

gttgagctgc tgagaaggaa agttcgtctc gcccagcctt atgtcggcgt ctttctaaag 540

agagatgaca gcaatgagtc ggatgtggtc gagagcctgg atgaaatcta ccacacgggg 600

acgtttgccc agatccatga gatgcaggac cttggggaca agctgcgcat gatcgtcatg 660

ggacacagaa gagtccatat cagcagacag ctggaggtgg agcccgagga gccggaggcg 720

gagaacaagc acaagccccg caggaagtca aagcggggca agaaggaggc ggaggacgag 780

ctgagcgcca ggcacccggc ggagctggcg atggagccca cccctgagct cccggctgag 840

gtgctcatgg tggaggtaga gaacgttgtc cacgaggact tccaggtcac ggaggaggtg 900

aaagccctga ctgcagagat cgtgaagacc atccgggaca tcattgcctt gaaccctctc 960

tacagggagt cagtgctgca gatgatgcag gctggccagc gggtggtgga caaccccatc 1020

tacctgagcg acatgggcgc cgcgctcacc ggggccgagt cccatgagct gcaggacgtc 1080

ctggaagaga ccaatattcc taagcggctg tacaaggccc tctccctgct gaagaaggaa 1140

tttgaactga gcaagctgca gcagcgcctg ggcgggaggt tggaggagaa gatcaagcag 1200

acccaccgta agtacctgct gcaggagcag ctaaagatca tcaagaagga gctgggcctg 1260

gagaaggacg acaaggatgc catcgaggag aagttccggg agcgcctgaa ggagctcgtg 1320

gtccccaagc acgtcatgga tgttgtggac gaggagctga gcaagctggg cctgctggac 1380

aaccactcct cggagttcaa tgtcacccgc aactacctag actggctcac gtccatccct 1440

tggggcaagt acagcaacga gaacctggac ctggcgcggg cacaggcagt gctggaggaa 1500

gaccactacg gcatggagga cgtcaagaaa cgcatcctgg agttcattgc cgttagccag 1560

ctccgcggct ccacccaggg caagatcctc tgcttctatg gccccccctgg cgtgggtaag 1620

accagcattg ctcgctccat cgcccgcgcc ctgaaccgag agtacttccg cttcagcgtc 1680

gggggcatga ctgacgtggc tgagatcaag ggccacaggc ggacctacgt gggcgccatg 1740

cccgggaaga tcatccagtg tttgaagaag accaagacgg agaaccccct gatcctcatc 1800

gacgaggtgg acaagatcgg ccgaggctac cagggggacc cgtcgtcggc actgctggag 1860

ctgctggacc cagagcagaa tgccaacttc ctggaccact acctggacgt gcccgtggac 1920

ttgtccaagg tgctgttcat ctgcacggcc aacgtcacgg acaccatccc cgagccgctg 1980

cgagaccgta tggagatgat caacgtgtcg ggctacgtgg cccaggagaa gctggccatt 2040

gcggagcgct acctggtgcc ccaggctcgc gccctgtgtg gcttggatga gagcaaggcc 2100

aagctgtcat cggacgtgct gacgctgctc atcaagcagt actgccgcga gagcggtgtc 2160

cgcaacctgc agaagcaagt ggagaaggtg ttacggaaat cggcctacaa gattgtcagc 2220

ggcgaggccg agtccgtgga ggtgacgccc gagaacctgc aggacttcgt ggggaagccc 2280

gtgttcaccg tggagcgcat gtatgacgtg acaccgcccg gcgtggtcat ggggctggcc 2340

tggaccgcaa tgggaggctc cacgctgttt gtggagacat ccctgagacg gccacaggac 2400

aaggatgcca agggtgacaa ggatggcagc ctggaggtga caggccagct gggggaggtg 2460

atgaaggaga gcgcccgcat agcctacacc ttcgccagag ccttcctcat gcagcacgcc 2520

cccgccaatg actacctggt gacctcacac atccacctgc atgtgcccga gggcgccacc 2580

cccaaggacg gcccaagcgc aggctgcacc atcgtcacgg ccctgctgtc cctggccatg 2640

ggcaggcctg tccggcagaa tctggccatg actggcgaag tctccctcac gggcaagatc 2700

ctgcctgttg gtggcatcaa ggagaagacc attgcggcca agcgcgcagg ggtgacgtgc 2760

atcgtcctgc cagccgagaa caagaaggac ttctacgacc tggcagcctt catcaccgag 2820

ggcctggagg tgcacttcgt ggaacactac cgggagatct tcgacatcgc cttcccggac 2880

gagcaggcag aggcgctggc cgtggaacgg tgacggccac cccgggactg caggcggcgg 2940

atgtcaggcc ctgtctgggc cagaactgag cgctgtgggg agcgcgcccg gacctggcag 3000

tggagccacc gagcgagcag ctcggtccag tgacccagat cccagggacc tcagtcggct 3060

taatcagagt gtggcataga agctatttaa tgattaaagt catttgcagt a 3111

SEQ ID NO: 28
<211> 1653
<212> DNA
<213> Homo sapiens

SEQ ID NO: 28

agtcagcacg ggggtgctgg aagagatcgg gaataatagc gcagaccaat gagcctaggg 60

agatgctttc atcgtctctc cttccctcaa gtgttctgga acctatcatt tgaattagcc 120

gagtcaggca ggagggggcg gggaatcctt ccgcccttct taggaggggc tgcattgcag 180

ggggagagtg aactgacaga ctcagtcact gaagagggaa aaggagtgag aagacaaagc 240

cgtcaaagcc ccaacagctt tgtatttctc cagcccggcg cagaccccgg agctcccgag 300

gcactccctc catctttgga acacgccagt aattgattga taacaggaag ctatgaggga 360

ccctgtgagt agccagtaca gttcctttct tttctggagg atgcccatcc cagaactgga 420

tctgtcggag ctggaaggcc tgggtctgtc agatacagcc acctacaagg tcaaagacag 480

cagcgttggc aaaatgatcg ggcaagcaac tgcagcagac caggagaaaa accctgaagg 540

tgatggcctc cttgagtaca gcaccttcaa cttctggaga gctcccattg ccagcatcca 600

ctccttcgaa ctggacttgc tctaaggcca agacttctct ctcccatcac cttgccctca 660

ttgtcttccc tctcaagccc cttcctttcc actcctttcc cattttaatc ttgttctctc 720

cctactgtgt tggtggtgct gatgaatctg ccagagttga gttctatgta tttatttatc 780

tatctgtcta ctccatttct ctcaaaagcc ctcaagtcac aaagtaaatg gttcaagcaa 840

tggagtactg ggtcacaggg attcctcctt tcccccccaa atattaactc cagaaactag 900

gcctgactgg ggacacctga gagtagtata gtagtgcaaa atggaagact gatttttgac 960

tctattataa tcagcttcag agattcctta aaccttccta atttcctgct ccagggcagt 1020

aaacacaaat atttcttcaa ggggtgatga aaacctcgga agttttaatt tgaggttatc 1080

tgctacgaaa cagtatttct aaaaggctaa agtgataagt ctcttgcttt tttttgatcc 1140

tgctcttata ttcttttttt tcctcagaga aatcaggagg gtagttagag gtataaaaca 1200

ggaggaaata ttatggaaaa tgaaaatagg gaaaataatt gaatcatttt agaagtagct 1260

aatttctttt ctcaaaagag tgtcccttct tcacacctac tcactttaca actttgctcc 1320

taactgtggg ttgaaaactc tagctaaaga aagttatcaa atcttaacat gcattcctac 1380

tattatgata gtttttaagg tttcaattca atcttctgaa cggcataagt cctattttag 1440

ccttacctcc tgcatttgca atacgtaata ctgatcagtg ggcacagttc ttcagctaca 1500

ttgagaccct gaaatgaaca attatattct gactcgacat cttgtcccca atccttccaa 1560

aaatattgat ggtgatttgt gctaccattt actcgtttat ttaataaaga cattcaatcc 1620

cagaaaaaaa aaaaaaaaaa aaaaaaaaaa aaa 1653

SEQ ID NO: 32
<211> 512
<212> DNA
<213> Homo sapiens

SEQ ID NO: 32

```
cttttcctca gctgccgcca aggtgctcgg tccttccgag gaagctaagg ctgcgttggg     60
gtgaggccct cacttcatcc ggcgactagc accgcgtccg gcagcgccag ccctacactc    120
gcccgcgcca tggcctctgt ctccgagctc gcctgcatct actcggccct cattctgcac    180
gacgatgagg tgacagtcac ggaggataag atcaatgccc tcattaaagc agccggtgta    240
aatgttgagc ctttttggcc tggcttgttt gcaaaggccc tggccaacgt caacattggg    300
agcctcatct gcaatgtagg ggccggtgga cctgctccag cagctggtgc tgcaccagca    360
ggaggtcctg ccccctccac tgctgctgct ccagctgagg agaagaaagt ggaagcaaag    420
aaagaagaat ccgaggagtc tgatgatgac atgggctttg gtctttttga ctaaacctct    480
tttataacat gttcaataaa aagctgaact tt                                  512
```

SEQ ID NO: 34
<211> 2069
<212> DNA
<213> Homo sapiens

SEQ ID NO: 34

```
gcctgtcgcc gcgggagcag ccgctatctc tgtgtgtccg cgtgtgcgcc cggtccccgc     60
ctgccgcacc atggagagct accacaagcc tgaccagcag aagctgcagg ccttgaagga    120
cacggccaac cgcctacgta tcagctccat ccaggccacc actgcggcgg gctctggcca    180
ccccacgtca tgctgcagcg ccgcagagat catggctgtc ctctttttcc acaccatgcg    240
ctacaagtcc caggaccccc ggaatccgca caatgaccgc tttgtgctct ccaagggcca    300
tgcagctccc atcctctacg cggtctgggc tgaagctggt ttcctggccg aggcggagct    360
gctgaacctg aggaagatca gctccgactt ggacgggcac ccggtcccga aacaagcttt    420
caccgacgtg gccactggct ccctgggcca gggcctcggg gccgcttgtg ggatggccta    480
caccggcaaa tacttcgaca aggccagcta ccgagtctat tgcttgctgg gagatgggga    540
gctgtcagag ggctctgtat gggaggccat ggccttcgcc agcatctata agctggacaa    600
ccttgtggcc attctagaca tcaatcgcct gggccagagt gacccggccc cgctgcagca    660
ccagatggac atctaccaga agcggtgcga ggccttcggt tggcatgcca tcatcgtgga    720
```

tggacacagc gtggaggagc tgtgcaaggc ctttggccag gccaagcacc agccaacagc 780

catcattgcc aagaccttca agggccgagg gatcacgggg gtagaagata aggagtcttg 840

gcatgggaag cccctcccca aaaacatggc tgagcagatc atccaggaga tctacagcca 900

gatccagagc aaaaagaaga tcctggcaac ccctccacag gaggacgcac cctcagtgga 960

cattgccaac atccgcatgc ccagcctgcc cagctacaaa gttggggaca agatagccac 1020

ccgcaaggcc tacgggcagg cactggccaa gctgggccat gccagtgacc gcatcatcgc 1080

cctggatggg gacaccaaaa attccacctt ctcggagatc ttcaaaaagg agcacccgga 1140

ccgcttcatc gagtgctaca ttgccgagca gaacatggtg agcatcgcgg tgggctgtgc 1200

cacccgcaac aggacggtgc ccttctgcag cacttttgca gccttcttca cgcgggcctt 1260

tgaccagatt cgcatggccg ccatctccga gagcaacatc aacctctgcg gctcccactg 1320

cggcgtttcc atcggggaag acgggccctc ccagatggcc ctagaagatc tggctatgtt 1380

tcggtcagtc cccacatcaa ctgtctttta cccaagtgat ggcgttgcta cagagaaggc 1440

agtggaacta gccgccaata caaagggtat ctgcttcatc cggaccagcc gcccagaaaa 1500

tgccatcatc tataacaaca atgaggactt ccaggtcgga caagccaagg tggtcctgaa 1560

gagcaaggat gaccaggtga ccgttatcgg ggctggggtg accctgcacg aggccttggc 1620

cgctgccgaa ctgctgaaga aagaaaagat caacatccgc gtgctggacc ccttcaccat 1680

caagcccctg gacagaaaac tcattctcga cagcgctcgt gccaccaagg gcaggatcct 1740

caccgtggag gaccattatt atgaaggtgg cattggtgag gctgtgtcca gtgcagtagt 1800

gggcgagcct ggcatcactg tcacccacct ggcagttaac cgggtaccaa gaagtgggaa 1860

gccagctgag ctgctgaaga tgtttggtat cgacagggat gccattgcac aagctgtgag 1920

gggcctcatc accaaggcct agggcgggta tgaagtgtgg ggcgggggtc tatacattcc 1980

tgagattctg ggaaaggtgc tcaaagatgt actgagagga ggggtaaata tatgttttga 2040

gaaaaatgaa aaaaaaaaaa aaaaaaaaa 2069

SEQ ID NO: 37
<211> 5032
<212> DNA
<213> Homo sapiens

SEQ ID NO: 37

```
gctgagcggg ttggcatctg gggcagcggg ctcgctccag gccgtcgggg gccgctcgcc     60
agcgtcgccc gctgtgttgg gagcgcgggc cgtgggcgtc gctcggcctt gtccgcggcg    120
tccccgctgc cggccacggc gctcagcgct tgtgctctgt attgcaggtc taccccgagc    180
cccggagcga gagcgagtgc ctgagcaaca tccgcgagtt cctgcgcggc tgcggggctt    240
ccctgcggct ggagacgttt gatgcaaatg atttgtatca ggggcagaat tttaacaagg    300
tcctcagttc cttagtgact ctaaataaag taacagcaga catcgggctg ggagtgact     360
ccgtgtgtgc ccggccctcg tctcaccgca taaagtcttt tgactccctt ggatcacagt    420
ctttgcacac tcggacttca aaactgttcc agggccagta tcggagtttg gacatgaccg    480
ataatagcaa caatcaactg gtagtaagag caaagtttaa cttccagcag accaatgagg    540
acgagctttc cttctcaaaa ggagacgtca tccatgtcac ccgtgtggaa gagggaggct    600
ggtgggaggg cacactcaac ggccggaccg gctggttccc cagcaactac gtgcgcgagg    660
tcaaggccag cgagaagcct gtgtctccca aatcaggaac actgaagagc cctcccaaag    720
gatttgatac gactgccata aacaaaagct attacaatgt ggtgctacag aatattttag    780
aaacagaaaa tgaatattct aaagaacttc agactgtgct ttcaacgtac ctacggccat    840
tgcagaccag tgagaagtta agttcagcaa acatttcata tttaatggga aatctagaag    900
aaatatgttc tttccagcaa atgctcgtac agtctttaga agaatgcacc aagttgcccg    960
aagctcagca gagagtcgga ggctgctttt taaacctgat gccacagatg aaaaccctgt   1020
acctcacgta ttgtgccaat caccccttctg cagtgaatgt cctcacggaa cacagtgagg   1080
agttggggga gttcatggag accaaaggtg ccagcagccc tgggattctc gtgctgacca   1140
cgggcctgag caaacccttc atgcgcctgg ataaataccc tacgctgctc aaagagctcg   1200
agagacacat ggaggattat catacagata gacaagatat tcaaaaatcc atggctgcct   1260
tcaaaaacct ttcagcccaa tgtcaagaag tccggaagag gaaagagctt gagctgcaga   1320
tcctgacgga agccatccgg aactgggagg gcgatgacat taaaactctg ggcaacgtca   1380
```

cttacatgtc ccaggtcctg attcagtgtg ccggaagtga ggaaaagaat gaaagatatc 1440

ttctactctt cccaaatgtt ttgctaatgt tgtctgccag tcctaggatg agtggcttta 1500

tctatcaggg aaagcttcca acgacaggaa tgacaatcac aaagcttgag gacagtgaaa 1560

atcatagaaa tgcatttgaa atatcaggga gcatgattga gcggatatta gtgtcgtgca 1620

acaaccagca ggatctgcag gaatgggtgg agcacctaca gaagcaaacg aaggtcacgt 1680

ctgtgggaaa ccccaccata aagcctcatt cagtgccatc tcataccctc ccctcccacc 1740

cggtcactcc gtccagcaag cacgcagaca gcaagcccgc gccgctgacg cccgcctacc 1800

acacgctgcc ccacccctcc caccacggca ccccgcacac caccatcaac tggggacccc 1860

tggagcctcc gaaaacaccc aagccctgga gcctgagctg cctgcggccc gcgcctcccc 1920

tccggccctc agctgctctc tgctacaagg aggatcttag taagagccct aagaccatga 1980

aaaagctgct gcccaagcgc aaacctgaac ggaagccttc agatgaggag ttcgcgtccc 2040

ggaaaagcac agctgctttg gaagaagatg ctcagattct gaaagtcatt gaagcttact 2100

gcaccagcgc caaaacaagg caaacactca attcaagttc acgcaaagaa tctgctccac 2160

aagttttgct tccagaagaa gagaaaatta tagtggaaga aactaaaagt aatggtcaga 2220

cagtgataga agaaaagagt cttgtggata ccgtatatgc attaaaggat gaagttcaag 2280

aattaagaca ggacaacaaa aagatgaaga aatctctaga ggaagaacag agagcccgca 2340

aagacctgga gaagctggtg aggaaagtcc tgaagaacat gaatgatcct gcctgggatg 2400

agaccaatct ataagggacg tcctcagttc tttctgttga agaccagttc tgaggtgaag 2460

ctgggcaccc ctgacccaag tcggggtgca ctcaggacca cagggcaggg ctgggtgggg 2520

cgccaccttg ctctctgtat atagaaaagc tggagcttat tctgcgaatg gagacgatca 2580

aaccatgact gatgaatcca gacaggaggg attgactctg aggacctgag ctacatcaat 2640

ccactctgtg aacatctcag ttacctcatt ctgcaataag ttcagtgact gactaaaagt 2700

cttgtttttc cagactttga attgaatata taaatattat atatacatgt ttcttgtaaa 2760

tatcccattt tgaatgcata cctgtggtgg ttctgtccgg gctaatcccc atgctagaat 2820

gtcctttcca gctacgtgaa taagaagtcc catgcccgca cccaccggaa gcagaagcct 2880

ggtggatgcc tggttcgttc cgcagcacca gggcctccac cgtgctgtgg cagcaccccc 2940

catgtcggta tttctaaata accttattta tacctgcaga gatacacttc agtcccattc 3000

agaagtcttc tcttaaagca gcattacagt cccagacctg cgggtttctg agggcaactt 3060

gctggctgac agactcagtc ttgacctcaa ggaaggccca tacggcactg ccgcatccac 3120

ctagaggtgt ttgctcttgt ccgctgtctg agtactgtga ttctcagatg agtttgctgc 3180

gttttgggag gacacagacg gttctgtata ggctagttca gtaacaacaa aatacactgt 3240

tttgtcttcc ctcaaagaga gatcttacta gaacctgtaa atagaatgta ttatttatta 3300

taagtcactg cagctgatga aaacagatgg aggccatgct gcaggctgat actgatgggt 3360

ggagttttgt catcaggcca gcctcatccc gaggtctcct ccaccattgg ccgtagccag 3420

caggcttcag tgctcaccga aagtaaaatc ccctccttca gcaagaataa agcaatatac 3480

accttaggtt ccactaagta acataggcat aagcagggaa cgtttccccc actgtgttcc 3540

agtgcagagg agacgaagcc tgtcctcacc gcggctcgct gggcccaggc tggctctgga 3600

aagcctgtgc ggtcctgggc aggaagcccg gcccgtggag caggttttcg ttctgcttca 3660

gcaataaata agggtgacca cagggacttt gcttttggtt tcctttcctg tgaaaaggtt 3720

ggttttaaag tgagatacac ttttccgtag aacaagtgtt ctatctttaa aaacccaaat 3780

tgcagcaccg tggattactg gtctcagaac aactcattgc gcatcagatt tgactctctg 3840

attttctgtc tattggccaa attgcccttt aactgcacct gaatcctttg tgtactgatg 3900

cctttgagct gggcaccttg ggagagtgtt gtgttgctgt ttacggttct tccttgccct 3960

tgctaattac agtctctggt gcccagcaag cccctttggc ttccttccgt gactggtcac 4020

gttgtctgcc tgggctcagc gtggacctgc cccatgctgc agaacctggc ctcacctgga 4080

cttttcacta gaattgccag cttcctcaac ttagcagatc attcactcat gcgggcacaa 4140

gcaaagatca acactttctt ttttggtaag cttgagtttt acaagttatt ttttggtgat 4200

gcgtaagaca ttgcagtggg aaaccattca acttgagttt attggagttt gctgttgtag 4260

caggttttaa ctcaggaaca actcttgtct gatctctccg cccctctgcc gggaggcgac 4320

attaactgtc ctctcggagc cggtagcgtt gctgtccgag tccccaggac ggatctcctg 4380

cagacctgcc ttaatgctca gatcgaagta tttcacaaga atacttgtgt ttttaacagc 4440

ccttcccctg gacggtgcgg ccatgagggc ctcatgttac ggcattgcct tttctttctg 4500

tggatccagt atcttcctcg gctttttagg gagcaggaaa aatgcgtctg agagcaactc 4560

tttttaaaaa cctgccctgt tgtatataac tgtgtctgtt tcaccgtgtg acctcccaag 4620

ggggtgggaa cttgatataa acgtttaaag gggccacgat ttgcccgagg gttactcctt 4680

tgctctcacc ttgtatggat gaggagatga agccatttct tatcctgtag atgtgaagca 4740

ctttcagttt tcagcgatgt tggaatgtag catcagaagc tcgttccttc acactcagtg 4800

gcgtctgtgc ttgtccacat gcgctgggcg tctgggacct tgaatgcctg ccctggttgt 4860

gtggactcct taatgccaat catttcttca cttctctggg acacccaggg cgcctgttga 4920

caagtgtgga gaaactccta atttaaatgt cacagacaat gtcctagtgt tgactactac 4980

aatgttgatg ctacactgtt gtaattatta aactgattat ttttcttatg tc 5032

SEQ ID NO: 46
<211> 3294
<212> DNA
<213> Homo sapiens

SEQ ID NO: 46

gtgaagagag gcgcggcgtg actgagctac ggttctggct gcgtcctaga ggcatccggg 60

gcagtaaaac cgctgcgatc gcggaggcgg cggccaggcc gagaggcagg ccgggcaggg 120

gtgtcggacg cagggcgctg ggccgggttt cggcttcggc cacagctttt tttctcaagg 180

tgcaatgaaa gccttccaca ctttctgtgt tgtccttctg gtgtttggga gtgtctctga 240

agccaagttt gatgattttg aggatgagga ggacatagta gagtatgatg ataatgactt 300

cgctgaattt gaggatgtca tggaagactc tgttactgaa tctcctcaac gggtcataat 360

cactgaagat gatgaagatg agaccactgt ggagttggaa gggcaggatg aaaaccaaga 420

aggagatttt gaagatgcag atacccagga gggagatact gagagtgaac catatgatga 480

tgaagaattt gaaggttatg aagacaaacc agatacttct tctagcaaaa ataaagaccc 540

aataacgatt gttgatgttc ctgcacacct ccagaacagc tgggagagtt attatctaga 600

aattttgatg gtgactggtc tgcttgctta tatcatgaat tacatcattg ggaagaataa 660

```
aaacagtcgc cttgcacagg cctggtttaa cactcatagg gagcttttgg agagcaactt    720
tactttagtg ggggatgatg gaactaacaa agaagccaca agcacaggaa agttgaacca    780
ggagaatgag cacatctata acctgtggtg ttctggtcga gtgtgctgtg agggcatgct    840
tatccagctg aggttcctca agagacaaga cttactgaat gtcctggccc ggatgatgag    900
gccagtgagt gatcaagtgc aaataaaagt aaccatgaat gatgaagaca tggataccta    960
cgtatttgct gttggcacac ggaaagcctt ggtgcgacta cagaaagaga tgcaggattt   1020
gagtgagttt tgtagtgata aacctaagtc tggagcaaag tatggactgc cggactcttt   1080
ggccatcctg tcagagatgg gagaagtcac agacggaatg atggatacaa agatggttca   1140
ctttcttaca cactatgctg acaagattga atctgttcat tttcagacc agttctctgg    1200
tccaaaaatt atgcaagagg aaggtcagcc tttaaagcta cctgacacta agaggacact   1260
gttgtttaca tttaatgtgc ctggctcagg taacacttac ccaaaggata tggaggcact   1320
gctacccctg atgaacatgg tgatttattc tattgataaa gccaaaaagt tccgactcaa   1380
cagagaaggc aaacaaaaag cagataagaa ccgtgcccga gtagaagaga acttcttgaa   1440
actgacacat gtgcaaagac aggaagcagc acagtctcgg cgggaggaga aaaaaagagc   1500
agagaaggag cgaatcatga atgaggaaga tcctgagaaa cagcgcaggc tggaggaggc   1560
tgcattgagg cgtgcaaaaa agaagttgga aaagaagcaa atgaaaatga aacaaatcaa   1620
agtgaaagcc atgtaaagcc atcccagaga tttgagttct gatgccacct gtaagctctg   1680
aattcacagg aaacatgaaa aacgccagtc catttctcaa ccttaaattt cagacagtct   1740
tgggcaactg agaaatcctt atttcatcat ctactctgtt tggggtttgg ggttttacag   1800
agattgaaga tacctggaaa gggctctgtt tcaagaattt tttttccag ataatcaaat    1860
tattttgatt attttataaa aggaatgatc tatgaaatct gtgtaggttt taaatatttt   1920
aaaaattata atacaaatca tcagtgcttt tagtacttca gtgtttaaag aaataccatg   1980
aaatttatag gtagataacc agattgttgc tttttgttta aaccaagcag ttgaaatggc   2040
tataaagact gactctaaac caagattctg caaataatga ttggaattgc acaataaaca   2100
ttgcttgatg ttttcttgta tgtctacatt aaacttgaga aaaagtaaaa attagaacac   2160
```

tgtatgtagt aatgaaattt cagggaccca gaacataatg tagtatatgt ttttaggtgg 2220
gagatgctga taacaaaatt aataggaagt ctgtaggcat taggatactg acatgtacat 2280
ggaaaattct agggacagga gcatcatttt ttccttacct gataccacga accagtgaca 2340
acgtgaatgc tgtattttaa gtggttgtat gtttattttc ttgagtaaca aatgcatgaa 2400
aaattaatgc ttcacctagg taagatcatt ggtctgtgtg aaatcacaaa tgtttttttcc 2460
ttcttggttg ctgcagcctg ggtggatgtt catggagaag ctctgttctc tatattatgg 2520
~~ctgtgtgccg ttgcttctcc ctctgctttt atctttttcca cagttgaggc tgggtatgtt 2580~~
ctttcaaaga aatggccatg aatatgtgta agtatacttt tgaaaatgag ctttcctaaa 2640
ctattgagag ttctttccac ctcttgcgga accaactctt ggaggagagg cccatgtatc 2700
tgcacgagca cttagcttgt tcagatctct gcattttata aatgcttctt accaagaaag 2760
catttttagg tcattgcttg taccaggtaa tttttgccgg ggatgggtaa gggttgggtt 2820
ttctggtggg agtggggtgg tgggtatttt ttgttgatgc tttagtgcag gcctgttctg 2880
aggcaataac aagttgctgt gaaaacagca tgtgctgctg cctttgtaac tgcatggaaa 2940
cttttcacat gggtttttct ccaagttaat acagaaatat gtaaactgag agatgcaaat 3000
gtaatatttt taacagttca tgaagttgtt attaaaataa ctaacataaa acttaattac 3060
tttaatatta tataattata gtagtggcct tgttttacaa acctttaaat tacattttag 3120
aaatcaaagt tgatagtctt agttatcttt tgagtaagaa aagctttcct aaagtcccat 3180
acatttggac catggcagct aattttgtaa cttaagcatt catatgaact acctatggac 3240
atctattaaa gtgattgaca aaatctcaaa aaaaaaaaaa aaaaaaaaaa aaaa 3294

SEQ ID NO: 51
<211> 3147
<212> DNA
<213> Homo sapiens

SEQ ID NO: 51

aggcgtctga ggggcggacg gaggcggcgg cggcggcggc gggagcggga gcgggcggcg 60
agtggggagc ggggccggga gtggagcagc cgccgcggcg ggactggacc gagcctcgcc 120

ggcgcgcacc tgcccgcagc gcccgcggag cgcgcagcgc ggcccgagcg cgacgacctg 180

ccgagcggcg gccgaggcgg cggtgtgggc gcgtcaggcc gcgacgaggg cgctgagaca 240

aatttacatg tattggagac cagaccagaa gcccttctga attaagatct cacattcttg 300

aaggtggcat tgaagagcac taagatcgga agatgagtga gcttgaccag ttacggcagg 360

aggccgagca acttaagaac cagattcgag acgccaggaa agcatgtgca gatgcaactc 420

tctctcagat cacaaacaac atcgacccag tgggaagaat ccaaatgcgc acgaggagga 480

cactgcgggg gcacctggcc aagatctacg ccatgcactg gggcacagac tccaggcttc 540

tcgtcagtgc ctcgcaggat ggtaaactta tcatctggga cagctacacc accaacaagg 600

tccacgccat ccctctgcgc tcctcctggg tcatgacctg tgcatatgcc ccttctggga 660

actatgtggc ctgcggtggc ctggataaca tttgctccat ttacaatctg aaaactcgtg 720

aggggaacgt gcgcgtgagt cgtgagctgg caggacacac aggttacctg tcctgctgcc 780

gattcctgga tgacaatcag atcgtcacca gctctggaga caccacgtgt gccctgtggg 840

acatcgagac cggccagcag acgaccacgt ttaccggaca cactggagat gtcatgagcc 900

tttctcttgc tcctgacacc agactgttcg tctctggtgc ttgtgatgct tcagccaaac 960

tctgggatgt gcgagaaggc atgtgccggc agaccttcac tggccacgag tctgacatca 1020

atgccatttg cttctttcca aatggcaatg catttgccac tggctcagac gacgccacct 1080

gcaggctgtt tgaccttcgt gctgaccagg agctcatgac ttactcccat gacaacatca 1140

tctgcgggat cacctctgtc tccttctcca agagcgggcg cctcctcctt gctgggtacg 1200

acgacttcaa ctgcaacgtc tgggatgcac tcaaagccga ccgggcaggt gtcttggctg 1260

ggcatgacaa ccgcgtcagc tgcctgggcg tgactgacga tggcatggct gtggcgacag 1320

ggtcctggga tagcttcctc aagatctgga actaacgcca gtagcatgtg gatgccatgg 1380

agactggaag accattccaa cttggacgcg ttaccatgag agcatatcct atccaaccgt 1440

actaacgtgg acaccctaca cctcccctca gaacttcaaa agggcaagat ctttttcct 1500

tcacttattg ctgaaaccaa gagcacaatt cccattgaga gaaagatctc tgtgctgtaa 1560

actaaaacaa attgtgcatt ccttccgggg ccatcgtctt tgttttcttt tttgtcttga 1620

atgaatttta aaaggaaata tataataaaa atgttaacca gaaggtaaac ttgagtgtaa 1680

ttgtcagaca gacacacttt tccaccagtg tatttgaatt ttagaccagt gaccctgttt 1740

tgtggcattc atgcaaaaca tgctgagggc tttgttcatc tggtcatcgt gtccaaattt 1800

cagtcatgtt tgtagcaaga ttttggaagc attcatattt ccttttttaaa atgtattcct 1860

ttgtgttcaa cagttaatca aaaccagaga gtctagggca gcctctctga tgttgtcaat 1920

gatgtaaatt cagtccctgg tttttaattt tctgtctgat gtcacagatc attgttgcac 1980

acaaacgtgg catagaaaag aacatgttca gaagccatgg ggccaagcac atgcggggac 2040

ggtctcaaat gcgtgatcag agaatccttc acctttgctg aaaagtgagc tcagatccag 2100

caccatgttc ctcctgaccc atcctgtcta tcttctcagt tgagttttta atctcacttt 2160

gggtttcctt gtgaagttgg agggaagttt ataatagcct aacactaccc cacccccaac 2220

taggaggaac ctctgttttc aagagagatg cctgtcctgt gcttggatag tcagtcaatt 2280

atttgtgtat gaaacaatgt acaaatcaat gttttgaaaa taatgatctc agactttcta 2340

agttaaattt taaaaatttt gattgtttgc catattgggt gggtttactc ttagaatcgc 2400

atgctgtaga aatgctcaaa agtgcatatg ggactcagtc cttaggtgtt ctttttcttt 2460

taagaaataa cctcttacag ttgtaaccat tgcggctctg tccacttctc gttgctgctc 2520

tgtggcacat atcggaagca gtacagcgcg cggctctaca cgcttgggta gcgggataag 2580

tcactgtttt ctttatttct ttaaaaaaaa aaaagttctg ttgcaaacga ctgctgttgg 2640

attctgaggg tggggaggga gagagaggga gggagaggga gtgaagagcc tgccctccta 2700

tatggattct tcagggccct ccacatctga ggtggctcat tcccatcaca cacagattgt 2760

cctggtgttc atttcaaggc cagtgttcag cagcagcgtt tggaaagcag gttctgtggg 2820

accccccgcc ccgcccccccg cactccttca tagcagcagt agtggcttct ccatcctgtt 2880

ttctgcaaca ttctatacaa aactgtgctg tgaccttgcg gtaggcctgg atctggcaaa 2940

gagaatacaa atgaaacccc ttctttctct ttccgtccaa caactctgta gagctctctg 3000

caccccttacc cctttccacc ttttgtattt aattttaaag tcagtgtact gcaaggaagc 3060

tggatgcaag atagatacta tattaaactg tactgttatt taagatgtaa taaagcagtt 3120

tgacatgaaa aaaaaaaaaa aaaaaaa 3147

SEQ ID NO: 64
<211> 1996
<212> DNA
<213> Homo sapiens

SEQ ID NO: 64

```
agtgacgtag ggttggcgca cggatccgtt gcggctgcag ctctgcagtc gggccgttcc    60
ttcgccgccg ccaggggtag cggtgtagct gcgcagcgtc gcgcgcgcta ccgcacccag   120
gttcggcccg taggcgtctg gcagcccggc gccatcttca tcgagcgcca tggccgcagc   180
ctgcgggccg ggagcggccg ggtactgctt gctcctcggc ttgcatttgt ttctgctgac   240
cgcgggccct gccctgggct ggaacgaccc tgacagaatg ttgctgcggg atgtaaaagc   300
tcttaccctc cactatgacc gctataccac ctcccgcagg ctggatccca tcccacagtt   360
gaaatgtgtt ggaggcacag ctggttgtga ttcttatacc ccaaaagtca tacagtgtca   420
gaacaaaggc tgggatgggt atgatgtaca gtgggaatgt aagacggact tagatattgc   480
atacaaattt ggaaaaactg tggtgagctg tgaaggctat gagtcctctg aagaccagta   540
tgtactaaga ggttcttgtg gcttggagta taatttagat tatacagaac ttggcctgca   600
gaaactgaag gagtctggaa agcagcacgg ctttgcctct ttctctgatt attattataa   660
gtggtcctcg gcggattcct gtaacatgag tggattgatt accatcgtgg tactccttgg   720
gatcgccttt gtagtctata agctgttcct gagtgacggg cagtattctc ctccaccgta   780
ctctgagtat cctccatttt cccaccgtta ccagagattc accaactcag caggacctcc   840
tcccccaggc tttaagtctg agttcacagg accacagaat actggccatg gtgcaacttc   900
tggttttggc agtgctttta caggacaaca aggatatgaa aattcaggac cagggttctg   960
gacaggcttg ggaactggtg gaatactagg atatttgttt ggcagcaata gagcggcaac  1020
acccttctca gactcgtggt actacccgtc ctatcctccc tcctaccctg gcacgtggaa  1080
tagggcttac tcacccttc atggaggctc gggcagctat tcggtatgtt caaactcaga  1140
cacgaaaacc agaactgcat caggatatgg tggtaccagg agacgataaa gtagaaagtt  1200
ggagtcaaac actggatgca gaaattttgg atttttcatc actttctctt tagaaaaaaa  1260
```

gtactacctg ttaacaattg ggaaaagggg atattcaaaa gttctgtggt gttatgtcca 1320

gtgtagcttt ttgtattcta ttatttgagg ctaaaagttg atgtgtgaca aaatacttat 1380

gtgttgtatg tcagtgtaac atgcagatgt atattgcagt ttttgaaagt gatcattact 1440

gtggaatgct aaaaatacat taatttctaa aacctgtgat gccctaagaa gcattaagaa 1500

tgaaggtgtt gtactaatag aaactaagta cagaaaattt cagttttagg tggttgtagc 1560

tgatgagtta ttacctcata gagactataa tattctattt ggtattacat tatttgatgt 1620

~~ttgctgttct tcaaacattt aaatcaagct ttggactaat tatgctaatt tgtgagttct 1680~~

gatcactttt gagctctgaa gctttgaatc attcagtggt ggagatggcc ttctggtaac 1740

tgaatattac cttctgtagg aaaaggtgga aaataagcat ctagaaggtt gttgtgaatg 1800

actctgtgct ggcaaaaatg cttgaaacct ctatatttct ttcgttcata agaggtaaag 1860

gtcaaatttt tcaacaaaag tcttttaata acaaaagcat gcagttctct gtgaaatctc 1920

aaatattgtt gtaatagtct gtttcaatct taaaaagaat caataaaaac aaacaaggga 1980

aaaaaaaaaa aaaaaa 1996

SEQ ID NO: 67
<211> 2583
<212> DNA
<213> Homo sapiens

SEQ ID NO: 67

gcatgcgcgc tcgcgctccc gccctctagc tgcgctcggc tgagtcagtc agtctgtcgg 60

agtctgtcct cggagcaggc ggagtaaagg gacttgagcg agccagttgc cggattattc 120

tatttcccct ccctctctcc cgccccgtat ctcttttcac ccttctccca ccctcgctcg 180

cgtagccatg gcggagccgt cggcggccac tcagtcccat tccatctcct cgtcgtcctt 240

cggagccgag ccgtccgcgc ccggcggcgg cgggagccca ggagcctgcc ccgccctggg 300

gacgaagagc tgcagctcct cctgtgcggt gcacgatctg attttctgga gagatgtgaa 360

gaagactggg tttgtctttg gcaccacgct gatcatgctg ctttccctgg cagctttcag 420

tgtcatcagt gtggtttctt acctcatcct ggctcttctc tctgtcacca tcagcttcag 480

gatctacaag tccgtcatcc aagctgtaca gaagtcagaa gaaggccatc cattcaaagc 540

ctacctggac gtagacatta ctctgtcctc agaagctttc cataattaca tgaatgctgc 600

catggtgcac atcaacaggg ccctgaaact cattattcgt ctctttctgg tagaagatct 660

ggttgactcc ttgaagctgg ctgtcttcat gtggctgatg acctatgttg gtgctgtttt 720

taacggaatc acccttctaa ttcttgctga actgctcatt ttcagtgtcc cgattgtcta 780

tgagaagtac aagacccaga ttgatcacta tgttggcatc gcccgagatc agaccaagtc 840

aattgttgaa aagatccaag caaaactccc tggaatcgcc aaaaaaaagg cagaataagt 900

acatggaaac cagaaatgca acagttacta aaacaccatt taatagttat aacgtcgtta 960

cttgtactat gaaggaaaat actcagtgtc agcttgagcc tgcattccaa gctttttttt 1020

taatttggtg ttttctccca tcctttccct ttaaccctca gtatcaagca caaaaattga 1080

tggactgata aaagaactat cttagaactc agaagaagaa agaatcaaat tcataggata 1140

agtcaatacc ttaatggtgg tagagccttt acctgtagct tgaaagggga aagattggag 1200

gtaagagaga aaatgaaaga acacctctgg gtccttctgt ccagttttca gcactagtct 1260

tactcagcta tccattatag ttttgccctt aagaagtcat gattaactta tgaaaaaatt 1320

atttggggac aggagtgtga taccttcctt ggtttttttt tgcagccctc aaatcctatc 1380

ttcctgcccc acaatgtgag cagctacccc tgatactcct tttctttaat gatttaacta 1440

tcaacttgat aaataactta taggtgatag tgataattcc tgattccaag aatgccatct 1500

gataaaaaag aatagaaatg gaaagtggga ctgagaggga gtcagcaggc atgctgcggt 1560

ggcggtcact ccctctgcca ctatccccag ggaaggaaag gctccgccat ttgggaaagt 1620

ggtttctacg tcactggaca ccggttctga gcattagttt gagaactcgt tcccgaatgt 1680

gctttcctcc ctctcccctg cccacctcaa gtttaataaa taaggttgta cttttcttac 1740

tataaaataa atgtctgtaa ctgctgtgca ctgctgtaaa cttgttagag aaaaaaataa 1800

cctgcatgtg ggctcctcag ttattgagtt tttgtgatcc tatctcagtc tggggggaa 1860

cattctcaag aggtgaaata cagaaagcct ttttttcttg atcttttccc gagattcaaa 1920

tctccgattc ccatttgggg gcaagttttt ttcttcacct tcaatatgag aattcagcga 1980

```
acttgaaaga aaaatcatct gtgagttcct tcaggttctc actcatagtc atgatccttc    2040
agagggaata tgcactggcg agtttaaagt aagggctatg atatttgatg gtcccaaagt    2100
acggcagctg caaaaagtag tggaaggaaa ttgtctacgt gtcttggaaa aattagttag    2160
gaatttggat gggtaaaagg tacccttgcc ttactccatc ttattttctt agcccccttt    2220
gagtgtttta actggtttca tgtcctagta ggaagtgcat tctccatcct catcctctgc    2280
cctcccagga agtcagtgat tgtctttttg ggcttcccct ccaaaggacc ttctgcagtg    2340
gaagtgccac atccagttct tttcttttgt tgctgctgtg tttagataat tgaagagatc    2400
tttgtgccac acaggatttt tttttttttt taagaaaaac ctatagatga aaaattacta    2460
atgaaactgt gtgtacgtgt ctgtgcgtgc aacataaaaa tacagtagca cctaaggagc    2520
ttgaatcttg gttcctgtaa aatttcaaat tgatgtggta ttaataaaaa aaaaaaaaaa    2580
aca                                                                  2583
```

SEQ ID NO: 78
<211> 2015
<212> DNA
<213> Homo sapiens

SEQ ID NO: 78

```
attaccccat ttctttcaag tccttggaaa ataacatatt aagggtacaa gaaattaaca      60
catgatggaa aagtcattgt gacgccaatg aatttcattg agtataaact catctacttc     120
aaatttattt tataagacaa cctaagatac tcaagataat tatttaatgg ttagctctta     180
agttgaattg gtctacataa tgcgtgggaa gaaaaccaga tttttagcct tcttgccaaa     240
tccagacctc tggttgattt ttctttgaca gaagatgcaa gttattttcc aatttcacaa     300
ttaaatgtat ttaacatgaa cattattttg ctttaaaaac tataaacatt gtaggagaat     360
tatagccagt cttcagttat aaccactcca ccctcctcac tttctctctc tctctctctt     420
tttttttttt tttttttgct atgggattta atgggaaaaa tatgtaaaaa ctgtcactag     480
tcagctggct ctttttccta tgaaatctat cagtaccttt ctccatccgt tgttctcaat     540
aatgaccaca gagcctgagt ataccaagaa aaccaatatt cgcattacag gttgctcctg     600
tccttccaga cacctttcct gcctgtgtga ctaacctaat tttgctagtt ccataaatac     660
```

acgattagtt tagtaacagc catcacaatg taccatgtac attcatggtg agagctaaag 720

atcgacacag acttctagga gctttgttca tactgattac ttaatccaat tgtatagatt 780

gaatatttga gtggaaggaa tttacactct gtttaaatga tgggattcta tcgagatagc 840

actcatgatc atgacctttt tggtagtatt cttaaacaaa attctacaga gactaaatgt 900

tagcgatgat cctccatttt caattttaac caattctgtc ccctttctca aaaccctgag 960

ccctgtgcat gctttctcag tcttgtggtg ggactggata caatgactaa cttcccctcc 1020

~~tcccctcttt aaacaccatt ttccatggag ttcaaaaaaa ttttttttct taacgttaca 1080~~

tatcatagtg aatggtttcc ccagtgtata tgaatgtttt aagtgtctcc aatagcttat 1140

gcagtctagg agctttccaa tactcattta attaagattt aatcatttgc taatggaaat 1200

cttaccacct ttcattttcc ctctgttacc aaatttcagc tcttaggagc tgctctacaa 1260

ttctgaattt gcttttcttg cctctcttta gtcacctgtc acaggaggtt cctgctcagt 1320

aatgatattg tgagttagga taataacttt tttttttgt gcttcagatt tagaagaaaa 1380

gatcctgttt ccatttgaaa ggaactgtaa gcttttatct tttaaccaac tgaacaatac 1440

accaaaagca gcctagggat gagcatttct ttgaaagcaa ttaggttatt cacctggtat 1500

taaaactatt tactgttaaa aaatctgtga cttcatgaag ttgattttta aaggcagcat 1560

caaaaactga aaaggaaggg aaaaaatagg cagcttctct gcacttgttt ggagctcccc 1620

aaaacaggag ccatggagaa gtggcatcaa gaccgggctg ccctttcgag aacaccctgt 1680

ggcagttcag agacacgctt ttcctacact gcatgcagcc cctctttcca gcactggaaa 1740

gaagtggtct tgagcccagc tgagaagcac ttcacactcc tctctcttgt tctgaatggt 1800

gtttgtgtca gtctgcagct gtgtatggta ttatgtctta taatcctgca tcacttctat 1860

cctatccagt catatctaat gtagaaaatt agtttccagt gaaagtaata tgtagtgctt 1920

ttatgatatt tgtgtgcaat atcccctctt ccattgagga tatttgatgt aaaggaaaaa 1980

aaaaactcag ttccacaata aaatacaaaa gtggc 2015

SEQ ID NO: 83
<211> 2479
<212> DNA
<213> Homo sapiens

SEQ ID NO: 83

```
cgccgcgctt cctcctgaag gtgactgcgc ccgcggggac gcagggggcg gggcccgggt 60
cgcccggagc cgggattggg cagagggcgg ggcggcggag ggattgcggc ggcccgcagc 120
gggataacct tgaggctgag gcagtggctc cttgcacagc agctgcacgc gccgtggctc 180
cggatctctt cgtctttgca gcgtagcccg agtcggtcag cgccggagga cctcagcagc 240
catgtcgaag ccccatagtg aagccgggac tgccttcatt cagacccagc agctgcacgc 300
agccatggct gacacattcc tggagcacat gtgccgcctg gacattgatt caccacccat 360
cacagcccgg aacactggca tcatctgtac cattggccca gcttcccgat cagtggagac 420
gttgaaggag atgattaagt ctggaatgaa tgtggctcgt ctgaacttct ctcatggaac 480
tcatgagtac catgcggaga ccatcaagaa tgtgcgcaca gccacggaaa gctttgcttc 540
tgaccccatc ctctaccggc ccgttgctgt ggctctagac actaaaggac ctgagatccg 600
aactgggctc atcaagggca gcggcactgc agaggtggag ctgaagaagg gagccactct 660
caaaatcacg ctggataacg cctacatgga aaagtgtgac gagaacatcc tgtggctgga 720
ctacaagaac atctgcaagg tggtggaagt gggcagcaag atctacgtgg atgatgggct 780
tatttctctc caggtgaagc agaaaggtgc cgacttcctg gtgacggagg tggaaaatgg 840
tggctccttg ggcagcaaga agggtgtgaa ccttcctggg gctgctgtgg acttgcctgc 900
tgtgtcggag aaggacatcc aggatctgaa gtttggggtc gagcaggatg ttgatatggt 960
gtttgcgtca ttcatccgca aggcatctga tgtccatgaa gttaggaagg tcctgggaga 1020
gaagggaaag aacatcaaga ttatcagcaa aatcgagaat catgaggggg ttcggaggtt 1080
tgatgaaatc ctggaggcca gtgatgggat catggtggct cgtggtgatc taggcattga 1140
gattcctgca gagaaggtct tccttgctca gaagatgatg attggacggt gcaaccgagc 1200
tgggaagcct gtcatctgtg ctactcagat gctggagagc atgatcaaga agccccgccc 1260
cactcgggct gaaggcagtg atgtggccaa tgcagtcctg gatggagccg actgcatcat 1320
gctgtctgga gaaacagcca aaggggacta tcctctggag gctgtgcgca tgcagcacct 1380
```

gattgcccgt gaggcagagg ctgccatcta ccacttgcaa ttatttgagg aactccgccg 1440

cctggcgccc attaccagcg accccacaga agccaccgcc gtgggtgccg tggaggcctc 1500

cttcaagtgc tgcagtgggg ccataatcgt cctcaccaag tctggcaggt ctgctcacca 1560

ggtggccaga taccgcccac gtgcccccat cattgctgtg acccggaatc cccagacagc 1620

tcgtcaggcc cacctgtacc gtggcatctt ccctgtgctg tgcaaggacc cagtccagga 1680

ggcctgggct gaggacgtgg acctccgggt gaactttgcc atgaatgttg gcaaggcccg 1740

aggcttcttc aagaagggag atgtggtcat tgtgctgacc ggatggcgcc ctggctccgg 1800

cttcaccaac accatgcgtg ttgttcctgt gccgtgatgg accccagagc ccctcctcca 1860

gcccctgtcc cacccccttc ccccagccca tccattaggc cagcaacgct tgtagaactc 1920

actctgggct gtaacgtggc actggtaggt tgggacacca gggaagaaga tcaacgcctc 1980

actgaaacat ggctgtgttt gcagcctgct ctagtgggac agcccagagc ctggctgccc 2040

atcatgtggc cccacccaat caagggaaga aggaggaatg ctggactgga ggcccctgga 2100

gccagatggc aagagggtga cagcttcctt tcctgtgtgt actctgtcca gttcctttag 2160

aaaaaatgga tgcccagagg actcccaacc ctggcttggg gtcaagaaac agccagcaag 2220

agttaggggc cttagggcac tgggctgttg ttccattgaa gccgactctg gccctggccc 2280

ttacttgctt ctctagctct ctaggcctct ccagtttgca cctgtcccca ccctccactc 2340

agctgtcctg cagcaaacac tccaccctcc accttccatt ttcccccact actgcagcac 2400

ctccaggcct gttgctatag agcctacctg tatgtcaata aacaacagct gaagcaccaa 2460

aaaaaaaaaa aaaaaaaaa 2479

SEQ ID NO: 87
<211> 1991
<212> DNA
<213> Homo sapiens

SEQ ID NO: 87

cttgctccga gagggagtcc tcgcggacgt cagccaagat tccagaatga ctatcttgac 60

ttacccctttt aaaaatcttc ccactgcatc aaaatgggcc ctcagatttt ccataagacc 120

tctgagctgt tcctcccagc tacgagctgc cccagctgtc cagaccaaaa cgaagaagac 180

gttagccaaa cccaatataa ggaatgttgt ggtggtggat ggtgttcgca ctccattttt 240

gctgtctggc acttcatata aagacctgat gccacatgat ttggctagag cagcgcttac 300

gggtttgttg catcggacca gtgtccctaa ggaagtagtt gattatatca tctttggtac 360

agttattcag gaagtgaaaa caagcaatgt ggctagagag gctgcccttg gagctggctt 420

ctctgacaag actcctgctc acactgtcac catggcttgt atctctgcca accaagccat 480

gaccacaggt gttggcttga ttgcttctgg ccagtgtgat gtgatcgtgg caggtggtgt 540

tgagttgatg tccgatgtcc ctattcgtca ctcaaggaaa atgagaaaac tgatgcttga 600

tctcaataag gccaaatcta tgggccagcg actgtcttta atctctaaat tccgatttaa 660

tttcctagca cctgagctcc ctgcggtttc tgagttctcc accagtgaga ccatgggcca 720

ctctgcagac cgactggccg ctgcctttgc tgtttctcgg ctggaacagg atgaatatgc 780

actgcgctct cacagtctag ccaagaaggc acaggatgaa ggactccttt ctgatgtggt 840

acccttcaaa gtaccaggaa aagatacagt taccaaagat aatggcatcc gtccttcctc 900

actggagcag atggccaaac taaaacctgc attcatcaag ccctacggca cagtgacagc 960

tgcaaattct tctttcttga ctgatggtgc atctgcaatg ttaatcatgg cggaggaaaa 1020

ggctctggcc atgggttata agccgaaggc atatttgagg gattttatgt atgtgtctca 1080

ggatccaaaa gatcaactat tacttggacc aacatatgct actccaaaag ttctagaaaa 1140

ggcaggattg accatgaatg atattgatgc ttttgaattt catgaagctt tctcgggtca 1200

gattttggca aattttaaag ccatggattc tgattggttt gcagaaaact acatgggtag 1260

aaaaaccaag gttggattgc ctcctttgga gaagtttaat aactggggtg gatctctgtc 1320

cctgggacac ccatttggag ccactggctg caggttggtc atggctgctg ccaacagatt 1380

acggaaagaa ggaggccagt atggcttagt ggctgcgtgt gcagctggag ggcagggcca 1440

tgctatgata gtggaagctt atccaaaata atagatccag aagaagtgac ctgaagtttc 1500

tgtgcaacac tcacactagg caatgccatt tcaatgcatt actaaatgac atttgtagtt 1560

cctagctcct cttaggaaaa cagttcttgt ggccttctat taaatagttt gcacttaagc 1620

cttgccagtg ttctgagctt ttcaataatc agtttactgc tctttcaggg atttctaagc 1680

caccagaatc tcacatgaga tgtgtgggtg gttgtttttg gtctctgttg tcactaaaga 1740

ctaaatgagg gtttgcagtt gggaaagagg tcaactgaga tttggaaatc atctttgtaa 1800

tatttgcaaa ttatacttgt tcttatctgt gtcctaaaga tgtgttctct ataaaataca 1860

aaccaacgtg cctaattaat tatggaaaaa taattcagaa tctaaacacc actgaaaact 1920

tataaaaaat gtttagatac ataaatatgg tggtcagcgt taataaagtg gagaaatatt 1980

ggaaaaaaaa a 1991

SEQ ID NO: 91
<211> 1721
<212> DNA
<213> Homo sapiens

SEQ ID NO: 91

aacccagcct ctcccctacc cgaacaccgg ccccggctcc accgaggccc gggtccccca 60

gcccgtctcg ccgccgccat ggcggaccct aaatacgccg accttcccgg cattgccagg 120

aatgagccag atgtttatga aactagcgac ctacctgagg atgatcaagc ggagttcgat 180

gcgtttgcac aagagctgga ggagctgaca agcacaagtg tggaacacat cattgtcaat 240

cctaatgctg cctatgacaa gttcaaggac aagagagtgg ggacaaaggg acttgatttc 300

tcagatcgta ttggaaaaac caagaggaca ggatatgaat ctggagaata tgagatgctt 360

ggagagggtc tgggagtgaa ggagacaccc cagcaaaagt accagcgcct actgcatgag 420

gtccaagagc tgacaactga agttgaaaaa atcaagacga cagtgaagga gtcagccaca 480

gaggagaagc tgacccctgt gttgctggct aaacagctgg cagccctgaa gcagcagctg 540

gttgcttccc acctggagaa gctgctggga ccagatgctg caatcaacct taccgacccc 600

gatggcgccc tggctaagcg cctactactg cagctggaag caacaaagaa cagcaaaggg 660

ggatcagggg gaaaaaccac tgggaccccc ccagatagca gccttgtcac ttatgaacta 720

cattctcggc ctgagcagga caagttctct caagctgcca aagtcgcaga acttgaaaag 780

cgcctgacag agctggagac agctgtacgt tgtgatcagg atgctcagaa tcccctttct 840

gcaggtctac agggagcctg tctcatggag actgtagagc tgttgcaagc aaaggtgagc 900

gccctagacc ttgcagtttt ggatcaagtg gaggctcggc tacagagtgt cctgggaaag 960

gtgaacgaga ttgccaagca taaagcctct gtagaagatg cagatacaca aagcaaggtg 1020

caccagctat atgaaactat acagcgctgg agccccattg cctccacccct ccctgagctg 1080

gtgcagagac ttgtcaccat caagcagctg cacgagcaag ccatgcagtt tggtcagctc 1140

ctgacacact tggataccac ccagcagatg attgctaatt ccttgaagga caataccacc 1200

ctcttgaccc aggtgcagac aaccatgcgt gaaaacctgg ccacagttga ggggaacttt 1260

gccagcattg atgaacggat gaagaagctg ggaaagtgag cacatttggg agctggagaa 1320

caggggttat ccctacccct gtgaactctg ttaacagctt acatagggtt tccccttttac 1380

tataactcta gcatccccat cccatttgac actgggggca agggttcttc ttgcatgtgg 1440

ggtttatacc cctcccctga tgaatacaga gtggtagcta ggggttggtt atcatcagaa 1500

ggtggtctcc cctcaggcct gggggataag gacgtgggcc cagccacatg ccaactcatg 1560

tccaatactg ctttgcctgg tgtggggaag gattgggtct tgtcccccaa cacagcttct 1620

gtggctgact gtaatactgt acaactgttt ctgaccatta aatgctgttg tactctgaaa 1680

aaaaaaaaaa aaaaaaaaaa aaattcctgc ggccgcaagc t 1721

SEQ ID NO: 92
<211> 1812
<212> DNA
<213> Homo sapiens

SEQ ID NO: 92

tagctaggca ggaagtcggc gcgggcggcg cggacagtat ctgtgggtac ccggagcacg 60

gagatctcgc cggctttacg ttcacctcgg tgtctgcagc accctccgct tcctctccta 120

ggcgacgaga cccagtggct agaagttcac catgtctatt ctcaagatcc atgccaggga 180

gatctttgac tctcgcggga atcccactgt tgaggttgat ctcttcacct caaaaggtct 240

cttcagagct gctgtgccca gtggtgcttc aactggtatc tatgaggccc tagagctccg 300

ggacaatgat aagactcgct atatggggaa gggtgtctca aaggctgttg agcacatcaa 360

taaaactatt gcgcctgccc tggttagcaa gaaactgaac gtcacagaac aagagaagat 420

tgacaaactg atgatcgaga tggatggaac agaaaataaa tctaagtttg gtgcgaacgc 480

cattctgggg gtgtcccttg ccgtctgcaa agctggtgcc gttgagaagg gggtccccct 540

gtaccgccac atcgctgact tggctggcaa ctctgaagtc atcctgccag tcccggcgtt 600

caatgtcatc aatggcggtt ctcatgctgg caacaagctg gccatgcagg agttcatgat 660

cctcccagtc ggtgcagcaa acttcaggga agccatgcgc attggagcag aggtttacca 720

caacctgaag aatgtcatca aggagaaata tgggaaagat gccaccaatg tgggggatga 780

aggcgggttt gctcccaaca tcctggagaa taaagaaggc ctggagctgc tgaagactgc 840

tattgggaaa gctggctaca ctgataaggt ggtcatcggc atggacgtag cggcctccga 900

gttcttcagg tctgggaagt atgacctgga cttcaagtct cccgatgacc ccagcaggta 960

catctcgcct gaccagctgg ctgacctgta caagtccttc atcaaggact acccagtggt 1020

gtctatcgaa gatccctttg accaggatga ctggggagct tggcagaagt tcacagccag 1080

tgcaggaatc caggtagtgg gggatgatct cacagtgacc aacccaaaga ggatcgccaa 1140

ggccgtgaac gagaagtcct gcaactgcct cctgctcaaa gtcaaccaga ttggctccgt 1200

gaccgagtct cttcaggcgt gcaagctggc ccaggccaat ggttggggcg tcatggtgtc 1260

tcatcgttcg ggggagactg aagatacctt catcgctgac ctggttgtgg ggctgtgcac 1320

tgggcagatc aagactggtg ccccttgccg atctgagcgc ttggccaagt acaaccagct 1380

cctcagaatt gaagaggagc tgggcagcaa ggctaagttt gccggcagga acttcagaaa 1440

ccccttggcc aagtaagctg tgggcaggca agcccttcgg tcacctgttg gctacacaga 1500

cccctcccct cgtgtcagct caggcagctc gaggcccccg accaacactt gcaggggtcc 1560

ctgctagtta gcgccccacc gccgtggagt tcgtaccgct tccttagaac ttctacagaa 1620

gccaagctcc ctggagccct gttggcagct ctagctttgc agtcgtgtaa ttggcccaag 1680

tcattgtttt tctcgcctca ctttccacca agtgtctaga gtcatgtgag cctcgtgtca 1740

tctccggggt ggccacaggc tagatccccg gtggttttgt gctcaaaata aaaagcctca 1800

gtgacccatg ag 1812

SEQ ID NO: 96
<211> 2122
<212> DNA
<213> Homo sapiens

SEQ ID NO: 96

```
ggcacgaggc cgagttgcga tgctgtactt ctctttgttt tgggcggctc ggcctctgca     60
gagatgtggg cagctggtca ggatggccat tcgggctcag cacagcaacg cagcccagac    120
tcagactggg gaagcaaaca ggggctggac aggccaggag agcctgtcgg acagtgatcc    180
tgagatgtgg gagttgctgc agagggagaa ggacaggcag tgtcgtggcc tggagctcat    240
tgcctcagag aacttctgca gccgagctgc gctggaggcc ctggggtcct gtctgaacaa    300
caagtactcg gagggttatc ctggcaagag atactatggg ggagcagagg tggtggatga    360
aattgagctg ctgtgccagc gccgggcctt ggaagccttt gacctggatc ctgcacagtg    420
gggagtcaat gtccagccct actccgggtc cccagccaac ctggccgtct acacagccct    480
tctgcaacct cacgaccgga tcatggggct ggacctgccc gatgggggcc atctcaccca    540
cggctacatg tctgacgtca agcggatatc agccacgtcc atcttcttcg agtctatgcc    600
ctataagctc aaccccaaaa ctggcctcat tgactacaac cagctggcac tgactgctcg    660
acttttccgg ccacggctca tcatagctgg caccagcgcc tatgctcgcc tcattgacta    720
cgcccgcatg agagaggtgt gtgatgaagt caaagcacac ctgctggcag acatggccca    780
catcagtggc ctggtggctg ccaaggtgat tccctcgcct ttcaagcacg cggacatcgt    840
caccaccact actcacaaga ctcttcgagg ggccaggtca gggctcatct tctaccggaa    900
aggggtgaag gctgtggacc ccaagactgg ccgggagatc ccttacacat ttgaggaccg    960
aatcaacttt gccgtgttcc catccctgca gggggccccc cacaatcatg ccattgctgc   1020
agtagctgtg gccctaaagc aggcctgcac ccccatgttc cgggagtact ccctgcaggt   1080
tctgaagaat gctcgggcca tggcagatgc cctgctagag cgaggctact cactggtatc   1140
aggtggtact gacaaccacc tggtgctggt ggacctgcgg cccaagggcc tggatggagc   1200
tcgggctgag cgggtgctag agcttgtatc catcactgcc aacaagaaca cctgtcctgg   1260
agaccgaagt gccatcacac cgggcggcct gcggcttggg gccccagcct taacttctcg   1320
acagttccgt gaggatgact tccggagagt tgtggacttt atagatgaag ggtcaacat    1380
```

tggcttagag gtgaagagca agactgccaa gctccaggat ttcaaatcct tcctgcttaa 1440

ggactcagaa acaagtcagc gtctggccaa cctcaggcaa cgggtggagc agtttgccag 1500

ggccttcccc atgcctggtt ttgatgagca ttgaaggcac ctgggaaatg aggcccacag 1560

actcaaagtt actctccttc cccctacctg ggccagtgaa atagaaagcc tttctatttt 1620

ttggtgcggg agggaagacc tctcacttag ggcaagagcc aggtatagtc tcccttccca 1680

gaatttgtaa ctgagaagat cttttctttt tccttttttt ggtaacaaga cttagaagga 1740

gggcccaggc actttctgtt tgaacccctg tcatgatcac agtgtcagag acgcgtcctc 1800

tttcttgggg aagttgagga gtgcccttca gagccagtag caggcagggg tgggtaggca 1860

ccctccttcc tgtttttatc taataaaatg ctaacctgcc ctgagtttcc attactgtgg 1920

gtggggttcc cctgggccaa acagtgattt gtctccctca atgtgtacac cgctccgctc 1980

ccaccaccgc taccacaagg acccccgggg ctgcagcctc ctctttctgt ctctgatcag 2040

agccgacacc agacgtgatt agcaggcgca gcaaattcaa tttgttaaat gaaattgtat 2100

tttgaaaaaa aaaaaaaaaa aa 2122

SEQ ID NO: 97
<211> 1510
<212> DNA
<213> Homo sapiens

SEQ ID NO: 97

atcctgctgc tgccgccacc gctgctgctg ctctgcaaaa ttcagctgct gcctctgtct 60

tgaggacccc agcgcctttc ccccggggcc atgctgcctg cagccacagc ctccctcctg 120

gggcccctcc tcactgcctg cgccctgctg ccttttgccc agggccagac ccccaactac 180

accagacccg tgttcctgtg cggaggggat gtgaaggggg aatcaggtta cgtggcaagt 240

gaggggttcc ccaactccta ccccccctaat aaggagtgca tctggaccat aacggtcccc 300

gagggccaga ctgtgtccct ctcattccga gtcttcgacc tggagctgca ccccgcctgc 360

cgctacgatg ctctggaggt cttcgctggg tctgggactt ccggccagcg gctcggacgc 420

ttttgtggga ccttccggcc tgcgccccta gtcgcccccg gcaaccaggt gaccctgagg 480

atgacgacgg atgagggcac aggaggacga ggcttcctgc tctggtacag cgggcgggcc 540

acctcgggct ctgagcacca attttgcggg gggcggctgg agaaggccca gggaaccctg 600

accacgccca actggcccga gtccgattac cccccgggca tcagctgttc ctggcacatc 660

atcgcgcccc cggaccaggt catcgcgctg accttcgaga agtttgacct ggagccggac 720

acctactgcc gctatgactc ggtcagcgtc ttcaacggag ccgtgagcga cgactcccgg 780

aggctgggga agttctgcgg cgacgcagtc ccgggctcca tctcctccga agggaatgaa 840

ctcctcgtcc agttcgtctc agatctcagt gtcaccgctg atggcttctc agcctcctac 900

aagaccctgc cgcggggcac tgccaaagaa gggcaagggc ccggccccaa acggggaact 960

gagcctaaag tcaagctgcc ccccaagtcc caacctccgg agaaaacaga ggaatctcct 1020

tcagcccctg atgcacccac ctgcccaaag cagtgccgcc ggacaggcac cttgcagagc 1080

aacttctgtg ccagcagcct tgtggtgact gcgacagtga agtccatggt tcgggagcca 1140

ggggagggcc ttgccgtgac tgtcagtctt attggtgctt ataaaactgg aggactggac 1200

ctgccaactc cacccactgg tgcctccctg aagttttacg tgccttgcaa gcagtgcccc 1260

cccatgaaga aaggagtcag ttatctgctg atgggccagg tagaagagaa cagaggcccc 1320

gtccttcctc cagagagctt tgtggttctc caccggccca accaggacca gatcctcacc 1380

aacctaagca agaggaagtg cccctctcaa cctgtgcggg ctgctgcgtc ccaggactga 1440

gacgcaggcc agccccggcc cctagccctc aggcctctct tcttatccaa ataaatgttt 1500

cttaatgaaa 1510

SEQ ID NO: 105
<211> 6773
<212> DNA
<213> Homo sapiens

SEQ ID NO: 105

gcggctggtt gcgggccggc ggcgggctgg cggagatgga ggatcttgtt caagatgggg 60

tggcttcacc agctacccct gggaccggga aatctaagaa ttggagaaag aaattgaaga 120

actcagatca aaacctgtta ctgaaggaac tggtgatatt attaaggcat taactgaacg 180

tctggatgct cttcttctgg aaaaagcaga gactgagcaa cagtgtcttt ctctgaaaaa 240

```
ggaaaatata aaaatgaagc aagaggttga ggattctgta acaaagatgg gagatgcaca   300
taaggagttg gaacaatcac atataaacta tgtgaaagaa attgaaaatt tgaaaaatga   360
gttgatggca gtacgttcca aatacagtga agacaaagct aacttacaaa agcagctgga   420
agaagcaatg aatacgcaat tagaactttc agaacaactt aaatttcaga acaactctga   480
agataatgtt aaaaaactac aagaagagat tgagaaaatt aggccaggct ttgaggagca   540
aatttatat ctgcaaaagc aattagacgc taccactgat gaaaagaagg aaacagttac   600
tcaactccaa aatatcattg aggctaattc tcagcattac caaaaaaata ttaatagttt   660
gcaggaagag cttttacagt tgaaagctat acaccaagaa gaggtgaaag agttgatgtg   720
ccagattgaa gcatcagcta aggaacatga agcagagata aataagttga acgagctaaa   780
agagaactta gtaaaacaat gtgaggcaag tgaaaagaac atccagaaga aatatgaatg   840
tgagttagaa aatttaagga aagccacctc aaatgcaaac caagacaatc agatatgttc   900
tattctcttg caagaaaata catttgtaga acaagtagta aatgaaaaag tcaaacactt   960
agaagatacc ttaaaagaac ttgaatctca acacagtatc ttaaaagatg aggtaactta   1020
tatgaataat cttaagttaa aacttgaaat ggatgctcaa catataaagg atgagttttt   1080
tcatgaacgg gaagacttag agtttaaaat taatgaatta ttactagcta aagaagaaca   1140
gggctgtgta attgaaaaat taaaatctga gctagcaggt ttaaataaac agttttgcta   1200
tactgtagaa cagcataaca gagaagtaca gagtcttaag gaacaacatc aaaaagaaat   1260
atcagaacta aatgagacat ttttgtcaga ttcagaaaaa gaaaaattaa cattaatgtt   1320
tgaaatacag ggtcttaagg aacagtgtga aaacctacag caagaaaagc aagaagcaat   1380
tttaaattat gagagtttac gagagattat ggaaatttta caaacagaac tgggggaatc   1440
tgctggaaaa ataagtcaag agttcgaatc aatgaagcaa cagcaagcat ctgatgttca   1500
tgaactgcag cagaagctca gaactgcttt tactgaaaaa gatgcccttc tcgaaactgt   1560
gaatcgcctc cagggagaaa atgaaaagtt actatctcaa caagaattgg taccagaact   1620
tgaaaatacc ataaagaacc ttcaagaaaa gaatggagta tacttactta gtctcagtca   1680
aagagatacc atgttaaaag aattagaagg aaagataaat tctcttactg aggaaaaaga   1740
```

tgattttata aataaactga aaaattccca tgaagaaatg gataatttcc ataagaaatg 1800

tgaaagggaa gaaagattga ttcttgaact tgggaagaaa gtagagcaaa caatccagta 1860

caacagtgaa ctagaacaaa aggtaaatga attaacagga ggactagagg agactttaaa 1920

agaaaaggat caaaatgacc aaaaactaga aaaacttatg gttcaaatga aagttctctc 1980

tgaagacaaa gaagtattgt cagctgaagt gaagtctctt tatgaggaaa acaataaact 2040

cagttcagaa aaaaaacagt tgagtaggga tttggaggtt tttttgtctc aaaaagaaga 2100

tgttatcctt aaagaacata ttactcaatt agaaaagaaa cttcagttaa tggttgaaga 2160

gcaagataat ttaaataaac tgcttgaaaa tgagcaagtt cagaagttat ttgttaaaac 2220

tcagttgtat ggttttctta aagaaatggg atcagaagtt tcagaagaca gtgaagagaa 2280

agatgttgtt aatgtcctac aggcagtcgg tgaatccttg gcaaaaataa atgaggaaaa 2340

atgcaacctg gcttttcagc gtgatgaaaa agtattagag ttagaaaaag agattaagtg 2400

ccttcaagaa gagagtgtag ttcagtgtga agaacttaag tctttattga gagactatga 2460

gcaagagaaa gttctcttaa ggaaagagtt agaagaaata cagtcagaaa aagaggccct 2520

gcagtctgat cttctagaaa tgaagaatgc taatgaaaaa acaaggcttg aaaatcagaa 2580

tcttttaatt caagttgaag aagtatctca aacatgtagc aaaagtgaaa tccataatga 2640

aaaagaaaaa tgttttataa aggaacatga aaacctaaag ccactactag aacaaaaaga 2700

attacgagat aggagagcag agttgatact attaaaggat tccttagcaa aatcaccttc 2760

tgtaaaaaat gatcctctgt cttcagtaaa agagttggaa gaaaaaatag aaaatctgga 2820

aaaagaatgc aaagaaaagg aggagaaaat aaataagata aaattagttg ccgtaaaggc 2880

aaagaaagaa ctagattcca gcagaaaaga gacccagact gtgaaggaag aacttgaatc 2940

tcttcgatca gaaaaggacc agttatctgc ttccatgaga gatctcattc aaggagcaga 3000

aagctataag aatcttttat tagaatatga aaagcagtca gagcaactgg atgtggaaaa 3060

agaacgtgct aataattttg agcatcgtat tgaagacctt acaagacaat taagaaattc 3120

gactttgcag tgtgaaacaa taaattctga taatgaagat ctcctggctc gtattgagac 3180

attacagtct aatgccaaat tattagaagt acagatttta gaagtccaga gagccaaagc 3240

aatggtagac aaagaattag aagctgaaaa acttcagaaa gaacagaaga taaaggaaca 3300
tgccactact gtaaatgaac ttgaagaact tcaggtacaa cttcaaaagg aaaagaaaca 3360
gcttcagaaa accatgcaag aattagagct ggttaaaaag gatgcccaac aaaccacatt 3420
gatgaatatg gaaatagctg attatgaacg tttgatgaaa gaactaaatc aaaagttaac 3480
taataaaaac aacaagatag aagatttgga gcaagaaata aaaattcaaa aacagaaaca 3540
agaaacccta caagaagaaa taacttcatt acagtcttca gtacaacaat atgaagaaaa 3600
aaacaccaaa atcaagcaat tgcttgtgaa aaccaaaaag gaactggcag attcaaagca 3660
agcagaaact gatcacttaa tacttcaagc atctttaaaa ggtgagctgg aggcaagcca 3720
gcagcaagta gaagtctata aaatacagct ggctgaaata acatcagaga agcacaaaat 3780
ccacgagcac ctgaaaacct ctgcggaaca gcaccagcgt acgctaagtg cataccagca 3840
gagagtgaca gcactacagg aagagtgccg tgctgccaag gcagaacaag ctactgtaac 3900
ctctgaattc gagagctaca aagtccgagt tcataatgtt ctaaaacaac agaaaaataa 3960
atctatgtct caggctgaaa ctgagggcgc taaacaagaa agggaacatc tggaaatgct 4020
gattgaccag ctaaaaatca aattacaaga tagccaaaat aacttacaga ttaatgtatc 4080
tgaacttcaa acattgcagt ctgaacatga tacactgcta gaaaggcaca acaagatgct 4140
gcaggaaact gtgtccaaag aggcggaact ccgggaaaaa ttgtgttcaa tacagtcaga 4200
gaacatgatg atgaaatctg aacatacaca gactgtgagt cagctaacat cccagaacga 4260
ggtccttcga aatagcttcc gagatcaagt gcgacatttg caggaagaac acagaaagac 4320
agtggagaca ttacagcagc agctctccaa gatggaagca cagctcttcc agcttaagaa 4380
tgaaccgacc acaagaagcc cagtttcctc tcaacaatct ttgaagaacc ttcgagaaag 4440
gagaaacaca gacctcccgc ttctagacat gcacactgta acccgggaag agggagaagg 4500
catggagaca actgatacgg agtctgtgtc ttccgccagc acatacacac agtctttaga 4560
gcagctgctt aactctcccg aaactaaact tgagcctcca ttatggcatg ctgaatttac 4620
caaagaagaa ttggttcaga agctcagttc caccacaaaa agtgcagatc acttaaacgg 4680
cctgcttcgg gaaacagaag caaccaatgc aattcttatg gagcaaatta agcttctcaa 4740

aagtgaaata agaagattgg aaaggaatca agagcgagag aagtctgcag ctaacctgga 4800

atacttgaag aacgtcttgc tgcagttcat tttcttgaaa ccaggtagtg aaagagagag 4860

acttcttcct gttataaata cgatgttgca gctcagccct gaagaaaagg gaaaacttgc 4920

tgcggttgct caaggtgagg aagaaaatgc ttcccgttct tctggatggg catcctatct 4980

tcatagttgg tctggacttc gataggttga tggaaggaat atttttatta accaaataga 5040

atctatttac aaaaatggtt cacgtatatt accacaattc ttttgtcaaa aagtgtgtat 5100

atatgtttgc atctacatat atttgtacat ctatatgaca gatgtatttt aaaagtttca 5160

tcttgaagta aaagtacaac agcttgaagt gttgatagca ggccacagcc ctctaactca 5220

tgtgatttcc catgcatgct gccagaataa aaccaccagg aatgaattca ctccccactt 5280

ctctggaacc tcaggacccg cccatttctc ggcagtactg tgaattttga agttaaacta 5340

aattttggta ccataccaac tggaatttag gctttaaaaa taatgtttca aggccaggtg 5400

tggtgattca tgcctgaaat cccactactt tgggaggctg aggctggaga attgcttgag 5460

gctagtgagc tgtgactccc actgcactcc agctcgggga acagagcgag accttgtctc 5520

taaaaataat agtaataaaa taaaaataac gttttatgac tatttattgc aaggtcagag 5580

ttacagattg ttataaattg ttgagaaatt tttgtgatta gaatatgaag gaaaaagctt 5640

tgttggtaaa agtgacatgt taaggggcta tgaagtaaat atgctgcagt taattgtgct 5700

aagttaaaat acagtttagt tatttgcttt aaaataaact cttctttttt tctttaaagt 5760

atactatctc aaaactcatt atgttgtcag agccctagag ctggctagtg taacactgac 5820

tatgagtagg tgggcccacc acttgagttg aggtgatttc atggtgtctt tccaggctct 5880

tgatagggtg tcactgcatg caagccatga atctgttttg agaatcctct ccattttccc 5940

aaataaaaac ctatcacaac agtgactata tcactcagca ttggatctaa atataaaagt 6000

ggtgctttca gtgtttttgg cagatagtgt tccataagct ttccatcaga agggatttta 6060

gacaccttag aggtccgtgc tacatcgtca cagttcctcc gaataacctt aggtggtagt 6120

gttacttgcc tttgacacct ctgcatatgt tttaatgact agatccaaac tgtgttgttc 6180

ttaaatcaaa aattggataa tttgtaatat ttatgtgtta atcacacagt atgctctctg 6240

aagttctctt aagccttcag tttatactct taatttaatt ttctttctga gctggagaac 6300

tggctttgca ctttggttac acagaacatt ggtttccaat tcagtttaac tgaaatttgc 6360

tgctgatatg ttgagtttgt tctttaaaaa atagctcata tatctcatct ttcctcctgt 6420

cttagaagaa cagacctaac tagtgaatgt attaatgaaa atgcatctat ttcagagctg 6480

acatgaagag tttagttttt ttactttata aactgtgaat atgagtatgc cagctgcata 6540

cgatgtaact aatcatattt aaatatattt cactttctct ttgactttag acctttttgaa 6600

gtctgtataa acttgttttg aaatatagtc tctgcttacg aatgtcataa caaaataatt 6660

ttttgcatga taaaaaatta ctttgattac aaaaggcgta ttctttcatg gtttctgcaa 6720

tgagaggaag tgtaatgatt attttaatat ttctattaaa tatgtttaac tgt 6773

SEQ ID NO: 114
<211> 2989
<212> DNA
<213> Homo sapiens

SEQ ID NO: 114

ggcgcgcggg cgagcggttg tgcttgtgct tgtggcgcgt ggtgcgggtt tcggcggcgg 60

ctgaggaaga agcgcgggcg gcgccttcgg gaggcgagca ggcagcagtt ggccgtgccg 120

tagcagcgtc ccgcgcgcgg cgggcagcgg cccaggaggc gcgtggcggc gctcggcctc 180

gcggcggcgg cggcggcagc ggcccagcag ttggcggcga gcgcgtctgc gcctgcgcgg 240

cgggccccgc gcccctcctc ccccctggg cgcccccggc ggcgtgtgaa tggcggcctc 300

cgcggcggca gcctcggcag cagcggcctc ggccgcctct ggcagcccgg gcccgggcga 360

gggctccgct ggcggcgaaa agcgctccac cgccccttcg gccgcagcct cggcctctgc 420

ctcagccgcg gcgtcgtcgc ccgcgggggg cggcgccgag gcgctggagc tgctggagca 480

ctgcggcgtg tgcagagagc gcctgcgacc cgagagggag ccccgcctgc tgccctgttt 540

gcactcggcc tgtagtgcct gcttagggcc cgcggccccc gccgccgcca acagctcggg 600

ggacggcggg gcggcgggcg acggcaccgt ggtggactgt cccgtgtgca agcaacagtg 660

cttctccaaa gacatcgtgg agaattattt catgcgtgat agtggcagca aggctgccac 720

cgacgcccag gatgcgaacc agtgctgcac tagctgtgag gataatgccc cagccaccag 780

ctactgtgtg gagtgctcgg agcctctgtg tgagacctgt gtagaggcgc accagcgggt 840

gaagtacacc aaggaccata ctgtgcgctc tactgggcca gccaagtctc gggatggtga 900

acgtactgtc tattgcaacg tacacaagca tgaacccctt gtgctgtttt gtgagagctg 960

tgatactctc acctgccgag actgccagct caatgcccac aaggaccacc agtaccagtt 1020

cttagaggat gcagtgagga accagcgcaa gctcctggcc tcactggtga agcgccttgg 1080

ggacaaacat gcaacattgc agaagagcac caaggaggtt cgcagctcaa tccgccaggt 1140

gtctgacgta cagaagcgtg tgcaagtgga tgtcaagatg gccatcctgc agatcatgaa 1200

ggagctgaat aagcggggcc gtgtgctggt caatgatgcc cagaaggtga ctgaggggca 1260

gcaggagcgc ctggagcggc agcactggac catgaccaag atccagaagc accaggagca 1320

cattctgcgc tttgcctctt gggctctgga gagtgacaac aacacagccc ttttgctttc 1380

taagaagttg atctacttcc agctgcaccg ggccctcaag atgattgtgg atcccgtgga 1440

gccacatggc gagatgaagt ttcagtggga cctcaatgcc tggaccaaga gtgccgaggc 1500

ctttggcaag attgtggcag agcgtcctgg cactaactca acaggccctg cacccatggc 1560

ccctccaaga gccccagggc ccctgagcaa gcagggctct ggcagcagcc agcccatgga 1620

ggtgcaggaa ggctatggct ttgggtcagg agatgatccc tactcaagtg cagagcccca 1680

tgtgtcaggt gtgaaacggt cccgctcagg tgagggcgag gtgagcggcc ttatgcgcaa 1740

ggtgccacga gtgagccttg aacgcctgga cctggacctc acagctgaca gccagccacc 1800

cgtcttcaag gtcttcccag gcagtaccac tgaggactac aaccttattg ttattgaacg 1860

tggcgctgcc gctgcagcta ccggccagcc agggactgcg cctgcaggaa cccctggtgc 1920

cccacccctg gctggcatgg ccattgtcaa ggaggaggag acggaggctg ccattggagc 1980

ccctcctact gccactgagg gccctgagac caaacctgtg cttatggctc ttgcggaggg 2040

tcctggtgct gagggtcccc gcctggcctc acctagtggc agcaccagct cagggctgga 2100

ggtggtggct cctgagggta cctcagcccc aggtggtggc ccgggaaccc tggatgacag 2160

tgccaccatt tgccgtgtct gccagaagcc aggcgatctg gttatgtgca accagtgtga 2220

gttttgtttc cacctggact gtcacctgcc ggccctgcag gatgtaccag ggaggagtg 2280

gagctgctca ctctgccatg tgctccctga cctgaaggag gaggatggca gcctcagcct 2340

ggatggtgca gacagcactg gcgtggtggc caagctctca ccagccaacc agcggaaatg 2400

tgagcgtgta ctgctggccc tattctgtca cgaaccctgc cgcccccctgc atcagctggc 2460

taccgactcc accttctccc tggaccagcc cggtggcacc ctggatctga ccctgatccg 2520

tgcccgcctc caggagaagt tgtcacctcc ctacagctcc ccacaggagt ttgcccagga 2580

tgtgggccgc atgttcaagc aattcaacaa gttaactgag gacaaggcag acgtgcagtc 2640

catcatcggc ctgcagcgct tcttcgagac gcgcatgaac gaggccttcg gtgacaccaa 2700

gttctctgct gtgctggtgg agccccgcc gatgagcctg cctggtgctg gcctgagttc 2760

ccaggagctg tctggtggcc ctggtgatgg cccctgaggc tggagccccc atggccagcc 2820

cagcctggct ctgttctctg tcctgtcacc ccatccccac tcccctggtg gcctgactcc 2880

cactccctgg tggccccatc cccagttcc tcacgatatg gtttttactt ctgtggattt 2940

aataaaaact tcaccagtta aaaaaaaaaa aaaaaaaaaa aaaaaaaaa 2989

SEQ ID NO: 124
<211> 2706
<212> DNA
<213> Homo sapiens

SEQ ID NO: 124

ctctgtagct gtgaccctga taccgcgtgg tgtgctccga acacatggtg cccagaacga 60

aggcggcgtc cagaagccct aggtcccaga ggtccgctca gcggcaggcg cataaggcgg 120

ggccggcgcg ggcctttcct tccatcggaa ccgttctccc ggggctgagt ccctgcccgg 180

actccgaacg ccgaagacca ggggccggaa gcgcgcgccg ccactgccac gccgtgtcag 240

tcgggaggga gggagcgagc aggcgaagcc gcggaggacg gggtgaagat ggcggccttc 300

tccgagatgg gtgtaatgcc tgagattgca caagctgtgg aagagatgga ttggctcctc 360

ccaactgata tccaggctga atctatccca ttgatcttag gaggaggtga tgtacttatg 420

gctgcagaaa caggaagtgg caaaactggt gcttttagta ttccagttat ccagatagtt 480

tatgaaactc tgaaagacca acaggaaggc aaaaaaggaa aaacaacaat taaaactggt 540

```
gcttcagtgc tgaacaaatg gcagatgaac ccatatgaca gaggatctgc ttttgcaatt   600
gggtcagatg gtctttgttg tcaaagcaga gaagtaaagg aatggcatgg gtgtagagct   660
actaaaggat taatgaaagg gaaacactac tatgaagtat cctgtcatga ccaagggtta   720
tgcagggtcg ggtggtctac catgcaggcc tctttggacc taggtactga caagtttgga   780
tttggctttg gtggaacagg aaagaaatcc cataacaaac aatttgataa ttatggagag   840
gaattcacta tgcatgatac cattggatgt tacctggata tagataaggg acatgtcaag   900
ttctccaaaa atggaaaaga tcttggtctg gcatttgaaa taccaccaca tatgaaaaac   960
caagccctct ttcctgcctg tgttttgaag aatgctgaac tgaaatttaa cttcggtgaa  1020
gaggaattta agtttccacc aaaagatggc tttgttgctc tttccaaggc accggatggt  1080
tacattgtca aatcacagca ctcaggtaat gcacaggtga cacaaacaaa gtttctcccc  1140
aatgctccga aagctctcat tgttgaacct tcccgggagt tagctgaaca aactttgaac  1200
aacatcaagc agtttaagaa atacattgat aatcctaaat taagggagct tctgataatt  1260
ggaggtgttg cagcccggga tcagctctct gttttggaaa atggagtaga tatagttgta  1320
ggtactccgg gaagactaga tgacttggtg tcaactggaa agctgaactt atctcaagtt  1380
agattcctgg tcctggatga agctgatggg cttctttctc aaggttattc tgattttata  1440
aataggatgc acaatcagat tcctcaggtt acctctgatg gaaaaagact tcaggtgatt  1500
gtttgctctg ccactttgca ttctttcgat gtaaagaaac tgtccgagaa gataatgcat  1560
tttcctacat gggttgactt aaaaggagaa gactctgttc cagatactgt acaccatgtt  1620
gttgtcccag taaatcccaa aactgacaga ctctgggaaa ggcttggaaa gagccacatt  1680
agaactgatg atgtacatgc aaaagataac acaagacctg gtgctaatag tccagagatg  1740
tggtctgaag ctattaaaat cctgaaaggg gagtatgctg tccgggcaat caaggaacat  1800
aagatggatc aagcaattat cttctgtaga accaaaattg actgtgataa cttggagcag  1860
tactttatac aacaaggagg aggacctgat aaaaaaggac accagttctc atgtgtttgt  1920
cttcatggtg acagaaagcc tcatgagaga aagcaaaact tggaaagatt taagaaagga  1980
gatgtaagat tcttgatttg cacagatgta gctgctagag gaattgatat ccacggtgtt  2040
```

ccttatgtta taaatgtcac tctgcccgat gaaaagcaaa actacgtaca tcgaattggc 2100

agagtaggaa gagctgaaag gatgggtctg gcaatttccc tggtggcaac agaaaaagaa 2160

aaggtttggt accatgtatg tagcagccgt ggaaaagggt gttataacac aagactcaag 2220

gaagatggag gctgtaccat atggtacaac gagatgcagt tactatctga gatagaagaa 2280

cacctgaact gtaccatttc tcaggttgag ccggatataa aggtaccagt ggatgaattt 2340

gatgggaaag ttacctacgg tcagaaaagg gctgctggtg gtggaagcta taaaggccat 2400

gtggatattt tggcacctac tgttcaagag ttggctgccc ttgaaaagga ggcgcagaca 2460

tctttcctgc atcttggcta ccttcctaac cagctgttca gaaccttctg atttttacat 2520

ttactgaata agatttgagt aatgaaagtc tgtagtctta aaactctaaa acagttgtac 2580

tgcttccaag cagcagtatt tatagtaacg taagctatta atgctaactc ttgcatgtca 2640

agaaacatta gtcttaggaa ttcttcaaaa aatggcatcc caatgaaaat aaatttgatg 2700

actata 2706

SEQ ID NO: 125
<211> 2179
<212> DNA
<213> Homo sapiens

SEQ ID NO: 125

accgaccgca ttgcggcttg gttttctcac ccagtgcatg tggcaggagc ggtgagatca 60

ctgcctcacg gcgatcctgg actgacggtc acgactgcct accctctaac cctgttctga 120

gctgcccctt gcccacacac cccaaacctg tgtgcaggat ccgcctccat ggagctacag 180

cctcctgaag cctcgatcgc cgtcgtgtcg attccgcgcc agttgcctgg ctcacattcg 240

gaggctggtg tccagggtct cagcgcgggg gacgactcag agttggggtc tcactgtgtt 300

gcccagactg gtctcgaact cttggcctca ggtgatcctc ttccctcagc ttcccagaat 360

gccgagatga tagagacggg gtctgactgt gttacccagg ctggtcttca actcttggcc 420

tcaagtgatc ctcctgcctt agcttccaag aatgctgagg ttacaggcac catgagccag 480

gacaccgagg tggatatgaa ggaggtggag ctgaatgagt tagagcccga gaagcagccg 540

atgaacgcgg cgtctggggc ggccatgtcc ctggcgggag ccgagaagaa tggtctggtg 600

aagatcaagg tggcggaaga cgaggcggag gcggcagccg cggctaagtt cacgggcctg 660
tccaaggagg agctgctgaa ggtggcaggc agccccggct gggtacgcac ccgctgggca 720
ctgctgctgc tcttctggct cggctggctc ggcatgcttg ctggtgccgt ggtcataatc 780
gtgcgagcgc cgcgttgtcg cgagctaccg gcgcagaagt ggtggcacac gggcgccctc 840
taccgcatcg gcgaccttca ggccttccag ggccacggcg cgggcaacct ggcgggtctg 900
aaggggcgtc tcgattacct gagctctctg aaggtgaagg gccttgtgct gggtccaatt 960
cacaagaacc agaaggatga tgtcgctcag actgacttgc tgcagatcga ccccaatttt 1020
ggctccaagg aagattttga cagtctcttg caatcggcta aaaaaaagag catccgtgtc 1080
attctggacc ttactcccaa ctaccggggt gagaactcgt ggttctccac tcaggttgac 1140
actgtggcca ccaaggtgaa ggatgctctg gagttttggc tgcaagctgg cgtggatggg 1200
ttccaggttc gggacataga gaatctgaag gatgcatcct cattcttggc tgagtggcaa 1260
aatatcacca agggcttcag tgaagacagg ctcttgattg cggggactaa ctcctccgac 1320
cttcagcaga tcctgagcct actcgaatcc aacaaagact tgctgttgac tagctcatac 1380
ctgtctgatt ctggttctac tggggagcat acaaaatccc tagtcacaca gtatttgaat 1440
gccactggca atcgctggtg cagctggagt ttgtctcagg caaggctcct gacttccttc 1500
ttgccggctc aacttctccg actctaccag ctgatgctct tcaccctgcc agggacccct 1560
gttttcagct acggggatga gattggcctg gatgcagctg cccttcctgg acagcctatg 1620
gaggctccag tcatgctgtg ggatgagtcc agcttccctg acatcccagg ggctgtaagt 1680
gccaacatga ctgtgaaggg ccagagtgaa gaccctggct ccctcctttc cttgttccgg 1740
cggctgagtg accagcggag taaggagcgc tccctactgc atggggactt ccacgcgttc 1800
tccgctgggc ctggactctt ctcctatatc cgccactggg accagaatga gcgttttctg 1860
gtagtgctta actttgggga tgtgggcctc tcggctggac tgcaggcctc cgacctgcct 1920
gccagcgcca gcctgccagc caaggctgac ctcctgctca gcacccagcc aggccgtgag 1980
gagggctccc ctcttgagct ggaacgcctg aaactggagc ctcacgaagg gctgctgctc 2040
cgcttcccct acgcggcctg acttcagcct gacatggacc cactaccctt ctcctttcct 2100

tcccaggccc tttggcttct gatttttctc tttttaaaa acaaacaaac aaactgttgc 2160

aaaaaaaaaa aaaaaaaaa 2179

SEQ ID NO: 129
<211> 1657
<212> DNA
<213> Homo sapiens

SEQ ID NO: 129

cctaagagtc gcgctgtaag aagcaacaac ctctcctctt cgtctccgcc atcagctcgg 60

cagtcgcgaa gcagcaacca tgcgtgagtg catctccatc cacgttggcc aggctggtgt 120

ccagattggc aatgcctgct gggagctcta ctgcctggaa cacggcatcc agcccgatgg 180

ccagatgcca agtgacaaga ccattggggg aggagatgat tccttcaaca ccttcttcag 240

tgagacgggg gctggcaagc atgtgccccg ggcagtgttt gtagacttgg aacccacagt 300

cattgatgaa gttcgcactg gcacctaccg ccagctcttc caccctgagc aacttatcac 360

aggcaaagaa gatgctgcca ataactatgc ccgagggcac tacaccattg gcaaggagat 420

cattgacctc gtgttggacc gaattcgcaa gctggccgac cagtgcacgg gtctccaggg 480

cttcttggtt ttccacagct ttggtggggg aactggttct gggttcacct cgctgctcat 540

ggaacgtctc tcagttgatt atggcaagaa gtccaagctg gagttctcta tttacccggc 600

gccccaggtt tccacagctg tagttgagcc ctacaactcc atcctcacca cccacaccac 660

cctggagcac tctgattgtg ccttcatggt agacaatgag gccatctatg acatctgtcg 720

tagaaacctc gatattgagc gtccaaccta tactaacctg aataggttaa taggtcaaat 780

tgtgtcctcc atcactgctt ccctgagatt tgatggagcc ctgaatgttg acctgacaga 840

attccagacc aacctggtgc cctatccccg catccacttc cctctggcca catatgcccc 900

tgtcatctct gctgagaaag cctaccatga acagctttct gtagcagaga tcaccaatgc 960

ttgctttgag ccagccaacc agatggtgaa atgtgaccct cgccatggta aatacatggc 1020

ttgctgcctg ttgtaccgtg gtgacgtggt tcccaaagat gtcaatgctg ccattgccac 1080

catcaagacc aagcgtacca tccagtttgt ggattggtgc cccactggct tcaaggttgg 1140

catcaactac cagcctccca ctgtggtgcc tggtggagac ctggccaagg tacagagagc 1200

tgtgtgcatg ctgagcaaca ccacagccat tgctgaggcc tgggctcgcc tggaccacaa 1260

gtttgacctg atgtatgcca aacgtgcctt tgttcactgg tacgttgggg aggggatgga 1320

ggaaggtgag ttttcagagg cccgtgagga catggctgcc cttgagaagg attatgagga 1380

ggttggtgtg gattctgttg aaggagaggg tgaggaagaa ggagaggaat actaaagtta 1440

aaacgtcaca aaggtgctgc ttttacaggg aagcttattc tgttttaaac attgaaaagt 1500

tgtggtctga tcagttaatt tgtatgtagc agtgtatgct ctcatataca attactgacc 1560

tatgctctaa aacatgaatg ctttgttaca gacccaagct gtccatttct gtgatgggtt 1620

ttgaataaag tattccctgt cttaaaaaaa aaaaaaa 1657

SEQ ID NO: 130
<211> 1810
<212> DNA
<213> Homo sapiens

SEQ ID NO: 130

cggattgagg cgccgccatt tttgctgccc ggacgcggag cgagaggctg agagagtcgg 60

agacactatc cgcttccatc cgtcgcgcag accctgccgg agccgctgcc gctatggatg 120

atcgagagga tctggtgtac caggcgaagc tggccgagca ggctgagcga tacgacgaaa 180

tggtggagtc aatgaagaaa gtagcaggga tggatgtgga gctgacagtt gaagaaagaa 240

acctcctatc tgttgcatat aagaatgtga ttggagctag aagagcctcc tggagaataa 300

tcagcagcat tgaacagaaa gaagaaaaca agggaggaga agacaagcta aaaatgattc 360

gggaatatcg gcaaatggtt gagactgagc taaagttaat ctgttgtgac attctggatg 420

tactggacaa acacctcatt ccagcagcta acactggcga gtccaaggtt ttctattata 480

aaatgaaagg ggactaccac aggtatctgg cagaatttgc cacaggaaac gacaggaagg 540

aggctgcgga gaacagccta gtggcttata aagctgctag tgatattgca atgacagaac 600

ttccaccaac gcatcctatt cgcttaggtc ttgctctcaa tttttccgta ttctactacg 660

aaattcttaa ttcccctgac cgtgcctgca ggttggcaaa agcagctttt gatgatgcaa 720

ttgcagaact ggatacgctg agtgaagaaa gctataagga ctctacactt atcatgcagt 780

tgttacgtga taatctgaca ctatggactt cagacatgca gggtgacggt gaagagcaga 840

ataaagaagc gctgcaggac gtggaagacg aaaatcagtg agacataagc caacaagaga 900

aaccatctct gaccaccccc tcctccccat cccacccttt ggaaactccc cattgtcact 960

gagaaccacc aaatctgact tttacatttg gtctcagaat ttaggttcct gccctgttgg 1020

tttttttttt tttttttttt aaacagtttt caaaagttct taaaggcaag agtgaatttc 1080

tgtggatttt actggtccca gcttttaggt tctttaagac actaacagga ctacatagag 1140

gctttttcag cattactgtg tcgtctccgt gccagatgtg gcaagatcac cattagcaaa 1200

tggaaattac atttgaaagc cattagactt ataggtgatg caagcatcta agagagaggt 1260

taatcacact atagaggcat aagtggtatc agttttcatt tttctaattg tttaaactgt 1320

gttttatacc agtgtttgca agtaattggg tgttagcttg agatggttaa aggtggtttg 1380

gggagggact tcgttgtaat ggttttgctg taaaaaatgt ttccaactcc gctgaaatgt 1440

tgctgaaaag catggtgctg gtaacagttc aacaatccgt ggctgctcat tcttgcctac 1500

tttactctcc cactgaagca ggttagcgtt gaaggtggta tggaaaagcc tgcatgcctg 1560

ttcaattctt ttgtttcttc tccttccccc tccccctacc tccttcccct cactcctccc 1620

ctccttcgct cgctcaacct cttttgttca gtatgtgtaa cttgaagcta atttgtacta 1680

ctggatatct gactggagcc acagatacag aatctgtatt gttcttactg aaacacagca 1740

tggaattaac attaaactta aataaaacaa acctaaatta aaaaaaaaaa aaaaaaaaaa 1800

aaaaaaaaaa 1810

SEQ ID NO: 131
<211> 987
<212> DNA
<213> Homo sapiens

SEQ ID NO: 131

gagcagcgga cgccggtctc tgttccgcag atggggtttg ttaaagttgt taagaataag 60

gcctacttta agagatacca agtgaaattt agaagacgac gagagggtaa aactgattat 120

tatgctcgga aacgcttggt gatacaagat aaaaataaat acaacacacc caaatacagg 180

atgatagttc gtgtgacaaa cagagatatc atttgtcaga ttgcttatgc ccgtatagag 240

ggggatatga tagtctgcgc acgttatgca cacgaactgc caaaatatgg tgtgaaggtt 300

ggcctgacaa attatgctgc agcatattgt actggcctgc tgctggcccg caggcttctc 360

aataggtttg gcatggacaa gatctatgaa ggccaagtgg aggtgactgg tgatgaatac 420

aatgtggaaa gcattgatgg tcagccaggt gccttcacct gctatttgga tgcaggcctt 480

gccagaacta ccactggcaa taaagttttt ggtgccctga agggagctgt ggatggaggc 540

ttgtctatcc ctcacagtac caaacgattc cctggttatg attctgaaag caaggaattt 600

aatgcagaag tacatcggaa gcacatcatg ggccagaatg ttgcagatta catgcgctac 660

ttaatggaag aagatgaaga tgcttacaag aaacagttct ctcaatacat aaagaacagc 720

gtaactccag acatgatgga ggagatgtat aagaaagctc atgctgctat acgagagaat 780

ccagtctatg aaaagaagcc caagaaagaa gttaaaaaga agaggtggaa ccgtcccaaa 840

atgtcccttg ctcagaagaa ggatcgggta gctcaaaaga aggcaagctt cctcagagct 900

caggagcggg ctgctgagag ctaaacccag caattttcta tgatttttc agatatagat 960

aataaactta tgaacagcaa ctaaaaa 987

SEQ ID NO: 132
<211> 1905
<212> DNA
<213> Homo sapiens

SEQ ID NO: 132

gctctgcgaa cggtgactgc ccatccttgg ccgcaatgag ccaccacccg tcgggcctcc 60

gggccggctt cagctccacc tcataccgcc gtaccttcgg tccaccgccc tcactatccc 120

ccggggcctt ctcctactcg tccagctccc gcttctccag cagccgcctg ctgggctccg 180

cgtccccgag ctcctcggtg cgcctgggca gcttccgtag cccccgagcg ggagcgggcg 240

ccctcctgcg cctgccctcg gagcgcctcg acttctccat ggccgaggcc ctcaaccagg 300

agttcctggc cacgcgcagc aacgagaagc aggagctgca ggagctcaac gaccgcttcg 360

ccaacttcat cgagaaggta cgctttctgg agcagcagaa cgcggccctg cgcggggagc 420

tgagccaagc ccggggccag gagccggcgc gcgccgacca gctgtgccag caggagctgc 480

gcgagctgcg gcgagagctg gagctgttgg gccgcgagcg tgaccgggtg caggtggagc 540
gcgacgggct ggcggaggac ctggcggcgc tcaagcagag gttggaggag gagacgcgca 600
agcgggagga cgcggagcac aacctcgtgc tcttccgcaa ggacgtggac gatgccactc 660
tgtcccgcct ggaactagag cgcaagattg agtctctgat ggatgagatt gagttcctca 720
agaagctgca cgaggaggag ctgcgagacc tgcaggtgag tgtggagagc cagcaggtgc 780
agcaggtgga ggtggaagcc acggtgaagc ccgagctgac ggcagcgctg agggacatcc 840
gcgcgcagta cgagagcatc gccgcgaaga acctgcagga ggcggaggag tggtacaagt 900
ccaagtacgc ggacctgtcc gacgctgcca accggaacca cgaggccctg cgccaggcca 960
agcaggagat gaacgagtcc cgacgccaga tccagagtct aacgtgcgag gtggacgggc 1020
tgcgcggcac gaacgaggcg ctgctcaggc agttgagaga gctggaggag cagttcgccc 1080
tggaggcggg gggctaccag gcgggcgctg cgcggctcga ggaggagctg cgacagctaa 1140
aagaggagat ggcgcggcac ctgagggagt accaggagct cctcaacgtc aagatggccc 1200
tggacatcga gatcgccacc taccgcaagc tgctggaggg cgaggagagc cggatctccg 1260
tgcccgtcca ttcttttgcc tccttaaata taaagacgac tgtgcctgag gtggagcctc 1320
cccaggacag ccacagccgg aagacggttc tgatcaagac cattgagacc cggaatgggg 1380
aggtggtgac agagtcccag aaggagcagc gcagtgagct ggacaagtct tctgcccaca 1440
gttactgaac cccttggtcc ggagccttga ctctgcccta ggcctgctca aagcccaaac 1500
cctaagacca ctcctgaatt gtctcctctc cctctgcatg tgtctaaaag gtggtaccag 1560
gcatcccttt cctggcttat ggccaagccc tacccggcca gcagtcgctg ggcctctccc 1620
tgccctgaca cttgatgtga cctatgtgct tccctttca tgtcccgata agaagccaat 1680
gatccccccct caggacaaat ctactccagc cacgatgaga agtgggtgag ccagggtctg 1740
agtttcacat ttgaaccaaa taaaatgctg tcaagagaaa aaaaaaaaaa aaaaaaaaaa 1800
aaaaaaaaaa aaaaaaaaaa aaaaaaaaaa aaaaaaaaaa aaaaaaaaaa aaaaaaaaaa 1860
aaaaaaaaaa aaaaaaaaaa aaaaaaaaaa aaaaaaaaaa aaaaa 1905

SEQ ID NO: 135
<211> 1769

<212> DNA
<213> Homo sapiens

SEQ ID NO: 135

```
gagagggcga aggtaggctg gcagatacgt tcgtcagctt gctcctttct gcccgtggac   60
gccgccgaag aagcatcgtt aaagtctctc ttcaccctgc cgtcatgtct aagtcagagt  120
ctcctaaaga gcccgaacag ctgaggaagc tcttcattgg agggttgagc tttgaaacaa  180
ctgatgagag cctgaggagc cattttgagc aatggggaac gctcacggac tgtgtggtaa  240
tgagagatcc aaacaccaag cgctctaggg gctttgggtt tgtcacatat gccactgtgg  300
aggaggtgga tgcagctatg aatgcaaggc cacacaaggt ggatggaaga gttgtggaac  360
caaagagagc tgtctccaga gaagattctc aaagaccagg tgcccactta actgtgaaaa  420
agatatttgt tggtggcatt aaagaagaca ctgaagaaca tcacctaaga gattattttg  480
aacagtatgg aaaaattgaa gtgattgaaa tcatgactga ccgaggcagt ggcaagaaaa  540
ggggctttgc ctttgtaacc tttgacgacc atgactccgt ggataagatt gtcattcaga  600
aataccatac tgtgaatggc cacaactgtg aagttagaaa agccctgtca aagcaagaga  660
tggctagtgc ttcatccagc caaagaggtc gaagtggttc tggaaacttt ggtggtggtc  720
gtggaggtgg tttcggtggg aatgacaact tcggtcgtgg aggaaacttc agtggtcgtg  780
gtggctttgg tggcagccgt ggtggtggtg gatatggtgg cagtggggat ggctataatg  840
gatttggcaa tgatggaagc aattttggag gtggtggaag ctacaatgat tttgggaatt  900
acaacaatca gtcttcaaat tttggaccca tgaagggagg aaattttgga ggcagaagct  960
ctggccccta tggcggtgga ggccaatact ttgcaaaacc acgaaaccaa ggtggctatg 1020
gcggttccag cagcagcagt agctatggca gtggcagaag attttaatta ggaaacaaag 1080
cttagcagga gaggagagcc agagaagtga cagggaagct acaggttaca acagatttgt 1140
gaactcagcc aagcacagtg gtggcagggc ctagctgcta caaagaagac atgttttaga 1200
caaatactca tgtgtatggg caaaaaactc gaggactgta tttgtgacta attgtataac 1260
aggttatttt agtttctgtt ctgtggaaag tgtaaagcat tccaacaaag ggttttaatg 1320
```

tagattttttt tttttgcacc ccatgctgtt gattgctaaa tgtaacagtc tgatcgtgac 1380

gctgaataaa tgtctttttt ttaatgtgct gtgtaaagtt agtctactct taagccatct 1440

tggtaaattt ccccaacagt gtgaagttag aattccttca gggtgatgcc aggttctatt 1500

tggaatttat atacaacctg cttgggtgga gaagccattg tcttcggaaa ccttggtgta 1560

gttgaactga tagttactgt tgtgacctga agttcaccat taaaagggat tacccaagca 1620

aaatcatgga atggttataa aagtgattgt tggcacatcc tatgcaatat atctaaattg 1680

aataatggta ccagataaaa ttatagatgg gaatgaagct tgtgtatcca ttatcatgtg 1740

taatcaataa acgatttaat tctcttgaa 1769

SEQ ID NO: 136
<211> 2356
<212> DNA
<213> Homo sapiens

SEQ ID NO: 136

gacacacgcg aggcgctgtc ctttcagcac cacaagctcg ggctgaggag ggaggactcc 60

tggccgtcct cctcctcttc aaattggctt gaatcttctc tgacccccca cgagtgcagc 120

acagtctggg aagaaaggcg taaggatggt gaagctgaac agtaacccca gcgagaaggg 180

aaccaagccg ccttcagttg aggatggctt ccagaccgtc cctctcatca ctcccttgga 240

ggttaatcac ttacagctgc ctgctccaga aaaggtgatt gtgaagacaa gaacggaata 300

tcagccggaa cagaagaaca aagggaagtt ccgggtgccg aaaatcgctg aatttacggt 360

caccatcctt gtcagcctgg ccctagcttt ccttgcgtgc atcgtgttcc tggtggttta 420

caaagccttc acctatgatc acagctgccc agagggattc gtctataagc acaaacgctg 480

tatcccagcc tccctggatg cttactactc ctcccaggac cccaattcca gaagccgctt 540

ctacacagtc atcagccact acagcgtggc caagcagagc actgcccggg ccatcgggcc 600

gtggctgtca gcagccgctg tcatccatga gcccaagccg cccaagaccc agggccacta 660

gaggcctgcc ccagccagaa tgggggcgg ggtggagagg aggacccca ttggctaagc 720

caagctccag ttacaagaca acactgtact cctgggatat gggggcgggg gcggggcagg 780

gcagggtggg gggaagaacg caccaaaaac gtggtgtgtg ctggagttgt ctgaaccgat 840

atttcttttt gttccttggt attgttgatt cgtcgccgag tcaggctcat gtacaaaggc 900

atgtttcgtg ttgattgttc ccatgtaaga tatttttaaa gccactgctt attctttgtt 960

aggaaaatgt aacagcagaa aaggaaagaa acaaagaaca tgaacaaaaa gcattaaact 1020

ggctccatcg gaagacgttg aagggcagtg aagagcacag actctgtggg cttcttagat 1080

aagaaaacgt agcttcagtg gggctccag ggttgcagag tatgagtgac acagaccggg 1140

actattccat tagcctgtgg tctgcagggt aggcccgcag gaaatgagga atggccaagc 1200

tggagagaag agctgatttt ggcattacta agcccagaat gcacataacc catagtgaaa 1260

tgtgctggcc tctggtgcat tttgcaagat gagcacaaac tttctgggcc tccatcctag 1320

gacctgggca gacccacatg gcctgggctc tgaatgccca ccctgcgacg gtgggttctg 1380

catcagcaaa cgctgaggag tgggcagatt ttctttgtct tttgcttgca ttttctagat 1440

ccacacctgg atactgccca tgttgacgag acagcagcag gggagaggg agggaaggaa 1500

ggtgcggctg caagaaggaa ggcacgggac aggcatgtga cactaggcca caagcgataa 1560

gcacaggcac ctgactttta agtttttgtt tgtttgttgt ttcccaaagt gctgataaca 1620

ataacaacaa caataggatt ccaaccagga gcctcaagtg acagccagga agagacctga 1680

aggttggggc caccacaatg ccaaatcgtt tctaaaggaa gctgaaaaat gggactgtct 1740

tttgcccact tcgttgtgtt aaaaggggac atttgtccaa actccccaac cgagttctag 1800

aagctcctga caaggaggca gcatccagcc ttgaccaggc ctcccagttc cctggaaccg 1860

tatcaggcat tcccctgcct ctcacaaatg tttcagggag gccagttctg cagggtgtca 1920

gctccaggac ccacagggcc agaaccagct gggagaattg gttatttgag atgtggtact 1980

gcttcctcac aagtctccca caggccatgt aaagggtatt tttttgtggc ttgctgtgtt 2040

gctgagatca tcgtatgcaa cagctgggta ataagactag catagctcaa actatcctgc 2100

caaacgctct catctgattt ttcctccctt ctcccccaac ctccaatcac cctgagtcac 2160

ctgtaaattc atttgtcatt caaagcggaa taacaagttg tccctagcaa aaccgctgag 2220

cgctttataa ttttgtggtg tatttttgtc agtaggtagc agaggcggaa gtattttttg 2280

gtgtaattct tgaaattttc tgacaggaaa caaataaaga tagatgtgtc tgagaaaaaa 2340

aaaaaaaaaa aaaaaa 2356

SEQ ID NO: 141
<211> 2024
<212> DNA
<213> Homo sapiens

SEQ ID NO: 141

gtgcgcgcgc ccggtccgcg cctccgccgc tttttatagc ggccgcgggc ggcggcggca 60

gcggttggag gttgtaggac cggcgaggaa taggaatcat ggcggctgcg ctgttcgtgc 120

tgctgggatt cgcgctgctg ggcacccacg gagcctccgg ggctgccggc ttcgtccagg 180

cgccgctgtc ccagcagagg tgggtggggg gcagtgtgga gctgcactgc gaggccgtgg 240

gcagcccggt gcccgagatc cagtggtggt ttgaagggca gggtcccaac gacacctgct 300

cccagctctg ggacggcgcc cggctggacc gcgtccacat ccacgccacc taccaccagc 360

acgcggccag caccatctcc atcgacacgc tcgtggagga ggacacgggc acttacgagt 420

gccgggccag caacgacccg gatcgcaacc acctgacccg ggcgcccagg gtcaagtggg 480

tccgcgccca ggcagtcgtg ctagtcctgg aacccggcac agtcttcact accgtagaag 540

accttggctc caagatactc ctcacctgct ccttgaatga cagcgccaca gaggtcacag 600

ggcaccgctg gctgaagggg ggcgtggtgc tgaaggagga cgcgctgccc ggccagaaaa 660

cggagttcaa ggtggactcc gacgaccagt ggggagagta ctcctgcgtc ttcctccccg 720

agcccatggg cacggccaac atccagctcc acgggcctcc cagagtgaag gctgtgaagt 780

cgtcagaaca catcaacgag ggggagacgg ccatgctggt ctgcaagtca gagtccgtgc 840

cacctgtcac tgactgggcc tggtacaaga tcactgactc tgaggacaag gccctcatga 900

acggctccga gagcaggttc ttcgtgagtt cctcgcaggg ccggtcagag ctacacattg 960

agaacctgaa catggaggcc gaccccggcc agtaccggtg caacggcacc agctccaagg 1020

gctccgacca ggccatcatc acgctccgcg tgcgcagcca cctggccgcc ctctggccct 1080

tcctgggcat cgtggctgag gtgctggtgc tggtcaccat catcttcatc tacgagaagc 1140

gccggaagcc cgaggacgtc ctggatgatg acgacgccgg ctctgcaccc ctgaagagca 1200

gcgggcagca ccagaatgac aaaggcaaga acgtccgcca gaggaactct tcctgaggca 1260
ggtggcccga ggacgctccc tgctccacgt ctgcgccgcc gccggagtcc actcccagtg 1320
cttgcaagat tccaagttct cacctcttaa agaaaaccca ccccgtagat tcccatcata 1380
cacttccttc tttttaaaa aagttgggtt ttctccattc aggattctgt tccttaggtt 1440
tttttccttc tgaagtgttt cacgagagcc cgggagctgc tgccctgcgg ccccgtctgt 1500
ggctttcagc ctctgggtct gagtcatggc cgggtgggcg gcacagcctt ctccactggc 1560
cggagtcagt gccaggtcct tgccctttgt ggaaagtcac aggtcacacg aggggccccg 1620
tgtcctgcct gtctgaagcc aatgctgtct ggttgcgcca tttttgtgct tttatgttta 1680
attttatgag ggccacgggt ctgtgttcga ctcagcctca gggacgactc tgacctcttg 1740
gccacagagg actcacttgc ccacaccgag ggcgaccccg tcacagcctc aagtcactcc 1800
caagccccct ccttgtctgt gcatccgggg gcagctctgg agggggtttg ctggggaact 1860
ggcgccatcg ccgggactcc agaaccgcag aagcctcccc agctcacccc tggaggacgg 1920
ccggctctct atagcaccag ggctcacgtg ggaacccccc tcccacccac cgccacaata 1980
aagatcgccc ccacctccac cctcaaaaaa aaaaaaaaaa aaaa 2024

SEQ ID NO: 142
<211> 2807
<212> DNA
<213> Homo sapiens

SEQ ID NO: 142

cgctggccaa tcggttgaga gctgagctgg acttggcggt gggagccgga gcctgcttgt 60
tgcagctgtg ggtgaggacg gctctagcta gttccctttt agactatggc gacatacctg 120
gagttcatcc agcagaatga agaacgggat ggtgtgcgtt ttagttggaa cgtgtggcct 180
tccagccggc tggaggctac aagaatggtt gtacccctgg cttgtctcct tactcctttg 240
aaagaacgtc cagacctacc tcctgtacaa tatgaacctg tgctttgcag caggccaact 300
tgtaaagctg ttctcaaccc actttgtcag gttgattatc gagcaaaact ttgggcctgt 360
aatttctgtt ttcaaagaaa tcagtttcct ccagcttatg gaggcatatc tgaggtgaat 420
caacctgccg aattgatgcc ccagttttct acaattgagt acgtgataca gcgaggtgct 480

cagtcccctc tgatctttct ctatgtggtt gacacatgcc tggaggaaga tgaccttcaa 540

gcactcaaag agtccctgca gatgtccctg agtcttcttc ctccagatgc tctggtgggt 600

ctgatcacat ttggaaggat ggtgcaggtt catgagctaa gctgtgaagg aatctccaaa 660

agttatgtct tccgagggac caaggattta actgcaaagc aaatacagga tatgttgggc 720

ctgaccaagc cagccatgcc catgcagcaa gcacgacctg cacaaccaca ggagcacccct 780

tttgcttcaa gcagatttct gcagcctgtt cacaagattg atatgaacct cactgatctt 840

cttggggagc tacagaggga cccatggcca gtaactcagg ggaagagacc tttgcgatcc 900

actggtgtgg ctttgtccat tgctgttggc ttgctggagg gcacttttcc aaacacagga 960

gccaggatca tgctgtttac tggaggtccc cctacccaag ggcctggcat ggtggttgga 1020

gatgaattaa agattcctat tcgttcttgg catgatattg agaaagataa tgcacgattc 1080

atgaaaaagg caaccaagca ctatgagatg cttgctaatc gaacagctgc aaatggtcac 1140

tgcattgata tttatgcttg tgcccttgat caaactggac ttttggagat gaagtgttgt 1200

gcaaatctta ctggaggcta catggtaatg ggagattctt tcaacacttc tctcttcaag 1260

cagacattcc aaagaatctt tactaaagat tttaatggag atttccgaat ggcatttggt 1320

gctactttgg acgtaaagac ctctcgggaa ctgaagattg caggagccat tggtccatgc 1380

gtatctctga atgtgaaagg accgtgtgtg tcagaaaatg agcttggtgt tggtggcacg 1440

agtcagtgga aaatctgtgg cctagatcct acatctacac ttggcatcta ttttgaagtt 1500

gtcaatcagc acaacacccc gatcccccaa ggaggcagag gagccatcca gtttgtcacg 1560

cattatcagc actccagcac ccagagacgc atccgcgtga ccaccatcgc ccgaaattgg 1620

gcagatgtac agagtcagct caggcacata gaagcagcat ttgaccagga ggctgcggca 1680

gtgttgatgg cacggcttgg ggtgttccga gcggagtcag aggaggggcc cgatgtgctc 1740

cggtggctgg accgacaact catccgactg tgtcaaaagt ttggacagta taacaaagaa 1800

gaccccactt cttttaggtt atcagattcc ttttctctat atcctcagtt tatgttccat 1860

ctgagaagat ctccatttct tcaagtgttt aacaacagtc ctgatgagtc gtcatattac 1920

agacatcatt ttgcccggca ggacctgacc cagtccctca tcatgatcca gcccattctc 1980

tactcttact cctttcatgg gccaccagag ccagtactct tggatagcag cagcattcta 2040

gctgacagaa ttttgctgat ggatactttc tttcaaattg tcatttatct tggtgagacc 2100

atagcccagt ggcgtaaagc tggctaccag gacatgcccg agtatgaaaa cttcaagcac 2160

cttctgcagg caccactgga tgatgctcaa gaaattctgc aagcacgctt cccgatgcca 2220

cgttacatca acacggagca tggaggcagt caggctcgat tccttttgtc caaagtgaac 2280

ccatctcaga cacacaataa cctgtatgct tggggacagg aaactggagc acccatccta 2340

actgatgatg ttagcctgca ggtgttcatg gaccatttga agaagctggc tgtctccagt 2400

gcctgttaag ctgaggatac aaccaggaaa tgcaacggtg tcagattgtg ttcaaaatgt 2460

ctagaaaggc ttgataacat tcctgttact tttctagcag attttaacaa ataatcaagg 2520

acatttttata tgtaactctt tagattataa tttatttgta ttcctgtctt tgtccttttt 2580

cttgcactat aaaattataa ggtcataaat gttttggtac ttgtagatgt ttatgtgctt 2640

tttgtatcct aacttttaga atctaaataa aatcagaggt aatgtatttt ggcagcttgt 2700

ttaggtgaga atcttaatga tcataaaagg aaataaatct agatgcagaa agtactggct 2760

aaaatattgc taatacaaat gtgatttcct gaaaaaaaaa aaaaaaa 2807

SEQ ID NO: 145
<211> 3241
<212> DNA
<213> Homo sapiens

SEQ ID NO: 145

agatgcactc tcttgtcctc aggtcccatc tgaggcagac ctgtctcaca ggacagcgct 60

ggagtcccca gcagcagcag cagccccagc cctatggttc ctggcaaacc cagcagacct 120

cagcaacccg gctcctgcag accatccgag ctgcctctgt ccactcctgg gcaggttgtg 180

cttgcattct ggaagctgtt tgacatacaa aggccagata tcagccttct cgctaactct 240

ggagtagggg agggtgtggt gggatgtgtg attgtgtgtg tatgtgtgtg tatgtagtgt 300

gtgtgcgcat agatatacaa agctgtgtgt atgcatctgt ttgcgtgcgc aggtttgtgg 360

acctgttctg gggccttctc tctttcagac tgctggggat gggcactttc tctttcccca 420

tctcttcccc atccagcccc acccatccct ggtacctgca actcatcaat actggaaacc 480

agctcctgcc agtccatcca tctctcagcc ttggcagccg ctgttgcccc tcagcccccct 540

ccccttccta cctcaggaat cactctggtt cccgtaaagc ccatgactga gcagcaattg 600

gtgtggggtt gggggggaggg agaatccagt aaaccaagac agaatacaaa aggcccactt 660

agcccagccc gtggcatggg gcagaccctc ccgagtggga tccagagctg ggtccagcca 720

ccctcagccc cctatgatgt acccacctca ttggggcttg ttgcggggta ggtctaggtt 780

tgggggctgt gtctttaagg ctgaaacatc agcagacaac ctctgctggg ccgtcttcag 840

gggaggggga ggggggctgg tcagcccatt agcagcagag ctggcgccag gcaccagccc 900

ttcacagggc cctggggatt agggagagta gctcttcccc agcccgatct gcctctgcgc 960

ccctgtctcc tattcctctc tgggtgtggt gaaggagggg tagctgacag caggtgaaaa 1020

tagagcctgg ccttcagggg gcaagtcaag gcactagcta tggggcctaa gccaggtcaa 1080

ctctccctcc agctggcctc ctccctactc ctgtgccaga agcctgccct ttaacttcag 1140

gtccttctac ctcgcaattt tcagggactc tagttcaagt caggccttcc taccctaggt 1200

tctagctttg ggtgcttgtt gggaggaagt gaccccccacc ccagcggcaa caggggggcaa 1260

tcaagtgggt agaggcctgc aaccagggaa gggaggggaa ggcgctcctg ctgttccgca 1320

ggggtggggc atccccctcc ccatacaacc cccctccagc gggccatcag gccagtggga 1380

ggagctgccc gtgccccccc tgagaccgca gggctataaa gccgcctcgc agcggtctgc 1440

ggctccttcc cagcccccgg cctagctctg cgaacggtga ctgcccatcc ttggccgcaa 1500

tgagtcacca cccgtcgggc ctccgggccg gcttcagctc cacctcatac cgccgtacct 1560

ttggtccacc gccctcacta tcccccgggg ccttctccta ctcgtccagc tcccgcttct 1620

ccagcagccg cctgctgggc tccgcgtccc cgagctcctc ggtgcgcctg ggcagcttcc 1680

gtagcccccg agcgggagcg ggcgccctcc tgcgcctgcc ctcggagcgc ctcgacttct 1740

ccatggccga ggccctcaac caggagttcc tggccacgcg cagcaacgag aagcaggagc 1800

tgcaggagct caacgaccgc ttcgccaact tcatcgagaa ggtacgcttt ctggagcagc 1860

agaacgcggc cctgcgcggg gagctgagcc aagcccgggg ccaggagccg gcgcgcgccg 1920

accagctgtg ccagcaggag ctgcgcgagc tgcggcgaga gctggagctg ttgggccgcg 1980

agcgtgaccg ggtgcaggtg gagcgcgacg ggctggcgga ggacctggcg gcgctcaagc 2040

agaggttgga ggaggagacg cgcaagcggg aggacgcgga gcacaacctc gtgctcttcc 2100

gcaaggacgt ggacgatgcc actctgtccc gcctggaact agagcgcaag attgagtctc 2160

tgatggatga gattgagttc ctcaagaagc tgcacgagga ggagctgcga gacctgcagg 2220

tgagtgtgga gagccagcag gtgcagcagg tggaggtgga agccacggtg aagcccgagc 2280

tgacggcagc gctgagggac atccgcgcgc agtacgagag catcgccacg aagaacctgc 2340

aggaggcgga ggagtggtac aagtccaagt acgcggacct gtccgacgct gccaaccgga 2400

accacgaggc cctgcgccag gccaagcagg agatgaacga gtcccgacgc cagatccaga 2460

gtctaacgtg cgaggtggac gggctgcgcg gcacgaacga ggcgctgctc aggcagttga 2520

gagagctgga ggagcagttc gccctggagg cgggggggcta ccaggcgggc gctgcgcggc 2580

tcgaggagga gctgcgacag ctaaaggagg agatggcgcg gcacctgagg gagtaccagg 2640

agctcctcaa cgtcaagatg gccctggaca tcgagatcgc cacctaccgc aagctgctgg 2700

agggcgagga gagccggatc tccgtgcccg tccattcttt tgcctcctta aatataaaga 2760

cgactgtgcc tgaggtggag cctccccagg acagccacag ccggaagacg gttctgatca 2820

agaccattga gacccggaat ggggaggtgg tgacagagtc ccagaaggag cagcgcagtg 2880

agctggacaa gtcttctgcc cacagttact gaaccccttg gtccggagcc ttgactctgc 2940

cctaggcctg ctcaaagccc aaaccctaag accactcctg aattgtctcc tctccctctg 3000

catgtgtcta aaaggtggta ccaggcatcc ctttcctggc ttatggccaa gccctacccg 3060

gccagcagtc gctgggcctc tccctgccct gacacttgat gtgacctatg tgcttccctt 3120

ttcatgtccc gataagaagc caatgatccc ccctcaggac aaatctactc cagccacgat 3180

gagaagtggg tgggccaggg tctgagtttc acatttgaac caaataaaat gctgtcaaga 3240

g 3241

SEQ ID NO: 152
<211> 2194
<212> DNA
<213> Homo sapiens

SEQ ID NO: 152

cctcgtgccg agcgacccgg gcaagcgacc cgggcgcggc gcggggaggc tgaagggacg 60

ctcgggtagg caagcgtttg cctcagcatg gagggcgggg cctcggcagc cacccccaca 120

gcactgcctt actacgtggc cttctcccag ctgctgggcc tgaccttggt ggccatgacc 180

ggcgcgtggc tcgggctgta ccgaggcggc attgcctggg agagcgacct gcagttcaac 240

gcgcaccccc tctgcatggt cataggcctg atcttcctgc agggaaatgc cctgctggtt 300

taccgtgtct tcaggaacga agctaaacgc accaccaagg tcctgcacgg gctgctgcac 360

atctttgcgc tcgtcatcgc cctggttggc ttggtggcgg tgttcgacta ccacaggaag 420

aagggctacg ctgacctgta cagcctacac agctggtgcg ggatccttgt ctttgtcctg 480

tactttgtgc agtggctggt gggcttcagc ttcttcctgt tccccggagc ttcattctcc 540

ctgcggagcc gctaccgccc acagcacatc ttctttggtg ctaccatctt cctcctttcc 600

gtgggcaccg ccctgctggg cctgaaggag gcactgctgt tcaacctcgg gggcaagtat 660

agcgcatttg agcccgaggg tgtcctggcc aacgtgctgg gcctgctgct ggcctgcttc 720

ggtggggcgg tgctctacat cttgacccgg gccgactgga agcggccttc ccaggcggaa 780

gagcaggccc tctccatgga cttcaagacg ctgacggagg gagatagccc cggctcccag 840

tgatgcgccc ggccggccct gggggttcgc ggggtgtctt cttgcctgcc cctgctgagg 900

cgtcttcagg actgcaggct ccggagagtg gctctggcag caggcgggcg cgtgggtgca 960

gctgcatctg tttgagtgct gctttctggg gtcaggtctc cgcctcctct gcttctcctt 1020

tctccgctgc tatagaccag ttcattgtgt gtggctcccg tgtctctgtt gccccccttca 1080

gtgcagaagg ctttgggtag gacttcgggt gttcggtcct ggtcgcagag cacagatctt 1140

taaagaagcg agagaggagg ccccaccctc ctggcagcag atgcctgggg caaggccagg 1200

ggaaactggg ggggcctcag ggacaggcct ggaaaggcca cgatggctgc tgaattcaaa 1260

caaggagtcc ctccagcctg aataacacgt ggcacaaatg ggcccggcct ttggcagagg 1320

agcaagtgat atgatgtgta aagtatgttg gtggtgaaag caaggttccc caggagaggg 1380

gagggactgg cccctgggaa gctctgagat gaggctgtgg cccagctgta gtcctgacct 1440

tcctcttctt taacccttta gccctaggat ggctttggtg ggagagggga tagaagccca 1500

tgacttcaga cagactttct cttggcagat gcaggcgggc ctcctcccag gctgctccag 1560

acatgggggt tggggatggg gggcaccttg cagccccttc ctgctggggc tccctccttg 1620

tagcaccccc cttgcggctc agctctggtt tcctctccca ggctcaccca ggctctgctc 1680

aggctgggag gcagagggca caaaccttat aatttttaa atgaaaaacc gctgctgctg 1740

gctgtggcta gagcccctg gggctgctgg agctgctgcc tctgttctgg aggacgagcc 1800

ttctccttat ctgctgccca tctttccagg aagtcaggat ggagtcagaa caactacagt 1860

catccccgt ggtgtctgca catcactcca gccccataaa gagtgtcatg ttagctgagt 1920

caccatttgg cttcggcctg gaaatagtgt gattagaaca ctgatcgtgt gcgaggccag 1980

gagatcaaga ccatcctgac taacaaacac agtgaaaccc cgtctctact aaaaatacaa 2040

aaaaattagc caggcgtggt ggtgggcgcc tgtagtccca gctacttggg aggctgaggc 2100

aggagaatgg tgtgaacccg ggagatggcg cttgcagtga gctgagattg cactccagcc 2160

tgggcgacag gctcaaaaaa aaaaaaaaaa aaaa 2194

SEQ ID NO: 154
<211> 2075
<212> DNA
<213> Homo sapiens

SEQ ID NO: 154

gccataaagg ccgccgcgcg cccacgcgcc tcgcttgctg cgcgctgccg gcgctccttc 60

ctcctcggct cgcgtctcac tcagtgtacc ttctagtccc gccatggccg ctctcacccg 120

ggacccccag ttccagaagc tgcagcaatg gtaccgcgag caccgctccg agctgaacct 180

gcgccgcctc ttcgatgcca acaaggaccg cttcaaccac ttcagcttga ccctcaacac 240

caaccatggg catatcctgg tggattactc caagaacctg gtgacggagg acgtgatgcg 300

gatgctggtg gacttggcca agtccagggg cgtggaggcc gcccgggagc ggatgttcaa 360

tggtgagaag atcaactaca ccgagggtcg agccgtgctg cacgtggctc tgcggaaccg 420

gtcaaacaca cccatcctgg tagacggcaa ggatgtgatg ccagaggtca acaaggttct 480

ggacaagatg aagtctttct gccagcgtgt ccggagcggt gactggaagg ggtacacagg 540
caagaccatc acggacgtca tcaacattgg cattggcggc tccgacctgg gacccctcat 600
ggtgactgaa gcccttaagc catactcttc aggaggtccc cgcgtctggt atgtctccaa 660
cattgatgga actcacattg ccaaaaccct ggcccagctg aaccccgagt cctccctgtt 720
catcattgcc tccaagacct ttactaccca ggagaccatc acgaatgcag agacggcgaa 780
ggagtggttt ctccaggcgg ccaaggatcc ttctgcagtg gcgaagcact ttgttgccct 840
gtctactaac acaaccaaag tgaaggagtt tggaattgac cctcaaaaca tgttcgagtt 900
ctgggattgg gtgggaggac gctactcgct gtggtcggcc atcggactct ccattgccct 960
gcacgtgggt tttgacaact tcgagcagct gctctcgggg gctcactgga tggaccagca 1020
cttccgcacg acgcccctgg agaagaacgc ccccgtcttg ctggccctgc tgggtatctg 1080
gtacatcaac tgctttgggt gtgagacaca cgccatgctg ccctatgacc agtacctgca 1140
ccgctttgct gcgtacttcc agcagggcga catggagtcc aatgggaaat acatcaccaa 1200
atctggaacc cgtgtggacc accagacagg ccccattgtg tgggggggagc cagggaccaa 1260
tggccagcat gctttttacc agctcatcca ccaaggcacc aagatgatac cctgtgactt 1320
cctcatcccg gtccagaccc agcaccccat acggaagggt ctgcatcaca agatcctcct 1380
ggccaacttc ttggcccaga cagaggccct gatgagggga aaatcgacgg aggaggcccg 1440
aaaggagctc caggctgcgg gcaagagtcc agaggacctt gagaggctgc tgccacataa 1500
ggtctttgaa ggaaatcgcc caaccaactc tattgtgttc accaagctca caccattcat 1560
gcttggagcc ttggtcgcca tgtatgagca caagatcttc gttcagggca tcatctggga 1620
catcaacagc tttgaccagt ggggagtgga gctgggaaag cagctggcta agaaaataga 1680
gcctgagctt gatggcagtg ctcaagtgac ctctcacgac gcttctacca atgggctcat 1740
caacttcatc aagcagcagc gcgaggccag agtccaataa actcgtgctc atctgcagcc 1800
tcctctgtga ctcccctttc tcttctcgtc cctcctcccc ggagccggca ctgcatgttc 1860
ctggacacca cccagagcac cctctggttg tgggcttgga ccacgagccc ttagcaggga 1920
aggctggtct cccccagcct aacccccagc ccctccatgt ctatgctccc tctgtgttag 1980

aattggctga agtgtttttg tgcagctgac ttttctgacc catgttcacg ttgttcacat 2040

cccatgtaga aaaataaaga tgccacggag gaggt 2075

SEQ ID NO: 160
<211> 2055
<212> DNA
<213> Homo sapiens

SEQ ID NO: 160

ggagggagag gctgaatgtt ggctcggtaa ttgagaggag cggccgctcc agagcttcct 60

cccggggcgc ccccctcagt ccgtccgcgc ttctcagccg ccagtctcct ggccgcgcag 120

tccccgcgga cggccgggcc gcggagaccc tcgcagaaga ggcgctgccg gcggcgagga 180

ctggccggcg gcatccgagg cgcgcgcccc accgccaccc ccacccgcct gccagccggt 240

ccctccggcc gccgccatgt cctcctcctc ttcctccccc agggagacgt acgaggagga 300

ccgggagtac gagagccagg ccaagcgtct caagaccgag gagggggaga tcgactactc 360

ggccgaggaa ggcgagaacc gccgggaagc gacgccccgg ggcgggggcg atggcggcgg 420

cggcggccgg agcttctctc agccggaggc aggtggaagt catcataaag tttctgtttc 480

acccgtcgtc catgttcgag gactctgtga atctgtggtg gaagcagacc tcgtggaagc 540

gctggaaaaa tttgggacaa tatgctatgt gatgatgatg ccatttaaac gacaggctct 600

agtggaattt gaaaacatag atagtgccaa agaatgtgtg acatttgctg cagatgaacc 660

cgtgtacatt gctggtcaac aggctttttt caactattct acaagcaaaa ggatcactcg 720

gccaggaaat actgatgatc catcaggagg caacaaagtt cttctgctct caattcagaa 780

tccgctttat ccaattacag tggatgtttt atatactgta tgcaaccctg ttggcaaagt 840

gcaacgtatt gttatattca agagaaatgg gatacaagca atggttgagt ttgaatcagt 900

cctttgtgcc cagaaagcta aagcagcact caatggagct gatatatatg ctggatgttg 960

cacactaaaa attgaatatg cacggccaac tcgtctaaat gttattagga atgacaatga 1020

cagttgggac tacactaaac catatttggg aagacgagat agaggaaagg gtcgccagag 1080

acaagccatt ttgggagaac acccttcttc gtttagacat gatggctatg gatcccatgg 1140

tccattattg cctttaccaa gtcgttacag aatgggctct cgagatacac ctgaacttgt 1200

tgcttatcca ttaccacagg cttcttcctc ttacatgcat ggaggaaatc cctctggttc 1260

agttgtaatg gttagtggat tacatcaact aaaaatgaat tgttcaagag tcttcaacct 1320

gttctgctta tatggaaata ttgagaaggt aaaatttatg aagaccattc ctggtacagc 1380

actggtagaa atgggtgatg agtatgctgt agaaagagct gtcacacacc ttaataatgt 1440

caaattattt gggaaaagac ttaatgtttg cgtgtctaaa caacattcag ttgttccaag 1500

tcaaatattt gagctggagg atggtaccag cagctacaaa gattttgcaa tgagcaaaaa 1560

taatcgcttt acaagtgctg gccaagcatc taagaatata atccagccac cctcctgtgt 1620

tttgcattat tataatgttc cattgtgtgt cacagaagag accttcacaa agttgtgtaa 1680

tgaccatgaa gttcttacat tcatcaaata taaagtgttt gatgcaaaac cttcagccaa 1740

aacactttct gggctattag aatgggagtg caaaactgat gcagtagaag cccttacggc 1800

actgaatcac tatcagataa gagtgccgaa tggttccaat ccctatacat tgaagctttg 1860

cttttctaca tcatcccatt tataagaaga gaagagcatg ttagaattta tgttcacctt 1920

tattacaatt tcaaagctac acttcattaa aaaaaaatct aaaatggttg atctcatgtt 1980

gccttgctta ctttaagatc ctgttctgta ataaacatat tttgccttga gtaaaaaaaa 2040

aaaaaaaaaa aaaaa 2055

SEQ ID NO: 163
<211> 4914
<212> DNA
<213> Homo sapiens

SEQ ID NO: 163

ctgtcaggtg actctcccgt ggcgccatgg cggaagcagt ggagcaggaa actgggtccc 60

ttgaagaatc tacagatgag tctgaggaag aagagagcga agaggaaccc aagctgaagt 120

atgaaaggct ttccaatggg gtaactgaaa tacttcagaa ggatgcagct agctgcatga 180

cagtccatga caagtttttg gcattgggca cacattatgg caaggtttat ttacttgatg 240

tccaggggaa catcactcag aagtttgatg taagtcctgt gaagataaat cagattagct 300

tggatgaaag tggagagcac atgggtgtgt gttcagagga tggcaaggtg caggtatttg 360

gactgtattc tggagaagaa tttcacgaga cttttgactg tcccattaaa attattgctg 420

tgcacccaca tttcgtgaga tccagttgca agcagtttgt gaccggaggg aagaagctgc 480

tactgtttga acggtcttgg atgaacagat ggaagtctgc tgttctgcat gaaggggaag 540

ggaacataag gagtgtgaag tggagaggcc atctgattgc ttgggccaat aatatgggtg 600

tgaagatttt tgacatcatc tcaaagcaaa gaatcaccaa tgtgccccgg gatgatataa 660

gtcttcgccc agacatgtat ccctgcagcc tctgctggaa ggacaatgtg acactgatta 720

ttggctgggg gacttctgtc aaggtgtgct cagtgaagga acggcatgcc agtgaaatga 780

gggatttgcc aagtcgatat gttgaaatag tgtctcagtt tgaaactgaa ttctacatca 840

gtggacttgc acctctctgt gatcagcttg ttgtactttc gtatgtaaag gagatttcag 900

aaaaaacgga aagagaatac tgtgccaggc ctagactgga catcatccag ccactttctg 960

agacttgtga agagatctct tctgatgctt tgacagtcag aggctttcag gagaatgaat 1020

gtagagatta tcatttagaa tactctgaag ggaatcact tttttacatc gtgagtccga 1080

gagatgttgt agtggccaag gaacgagacc aagatgatca cattgactgg ctccttgaaa 1140

agaagaaata tgaagaagca ttgatggcag ctgaaattag ccaaaaaaat attaaaagac 1200

ataagattct ggatattggc ttggcatata taaatcacct ggtggagaga ggagactatg 1260

acatagcagc acgcaaatgc cagaaaattc ttgggaaaaa tgcagcactc tgggaatatg 1320

aagtttataa atttaaagaa attggacagc ttaaggctat tagtccttat ttgccaagag 1380

gtgatccagt tctgaaacca ctcatctatg aaatgatctt acatgaattt ttggagagtg 1440

attatgaggg ttttgccaca ttgatccgag aatggcctgg agatctgtat aataattcag 1500

tcatagttca agcagttcgg gatcatttga agaaagatag tcagaacaag actttactta 1560

aaaccctggc agaattgtac acctatgaca agaactatgg caatgctctg gaaatatact 1620

taacattaag acataaagac gtttttcagt tgatccacaa gcataatctt ttcagttcta 1680

tcaaggataa aattgtttta ttaatggatt ttgattcaga gaaagctgtt gacatgcttt 1740

tggacaatga agataaaatt tcaattaaaa aggtagtgga agaattggaa gacagaccag 1800

agctacagca tgtgtatttg cataagcttt tcaagagaga ccaccataag ggcagcgtt 1860

accatgaaaa acagatcagt ctttatgctg aatatgatcg accaaactta cttccctttc 1920

tccgagacag tacccattgc ccacttgaaa aggctcttga gatctgtcaa cagagaaact 1980

ttgtagaaga gacagtttat cttctgagcc gaatgggtaa tagccgaagt gccctgaaga 2040

tgattatgga ggaattacat gatgttgata aagcaatcga atttgccaag gagcaagatg 2100

atggagagct gtgggaagat ttgattttat attccattga caaaccacca tttattactg 2160

gcttgttaaa caacattggc acacatgttg acccaattct actgattcac cgtattaagg 2220

aaggaatgga gatccccaat ttgagagatt ccttggttaa aattctgcaa gactacaatt 2280

tgcaaattct gcttcgtgaa ggctgcaaga agattctcgt agctgactct ttgtccttac 2340

tgaagaaaat gcaccgaact caaatgaaag gtgttcttgt tgatgaggag aacatctgtg 2400

agtcgtgcct ttcccctatt cttccatcag atgcagctaa gcccttcagc gtggtggtct 2460

tccattgccg gcacatgttc cacaaggagt gcctgcccat gcccagcatg aactctgctg 2520

cacagttctg caacatctgc agtgctaaga accgtggacc aggaagtgca attttggaga 2580

tgaaaaaata gctcatttct ccttgtcagt ctccttgtca ccactctttt tgagactgtt 2640

tttgcaacaa caaaagcatt tgttgacact cgtgctgtta agagatttgt ttatgtttat 2700

attatactca aaaacaattt cttcatctat tcctgtacta atggtttctc tttgcagttc 2760

acagagaatt tggggctctc ttcatgcctt gaaattttgg ggtccatagt gaatattttg 2820

ttatttattt gtttggctca ttctttatat agtaatggaa acataagtct aggagttaga 2880

aatgaatttt ttagacctta gtaaaaccat ttaaccataa aatggacaac tgagaattct 2940

cccagctgcc tgaaagcgtc gccaactgtg gttatcctgc aagctgctac ctgcaacttg 3000

gacgttgttt ccacgtgctc tgctggctac gattcttgca ttctgggttt ggcttttttc 3060

tgtgtcatca actatggtta tcctctaaat aggcatttaa tgaaacattg tacaaattgt 3120

cactcatttg atgacacctg ggaataacat tagcaggctg atgtcctgca ccattatgtt 3180

tactaatcac atgttctgtg tgctgtgacg actgtcaaag agtatctggc catggcggac 3240

actcagcatt tgttgattga ataaatgtta gctcttctca ttgtgaagga ctcactttta 3300

ctgggataaa caaatgcagt taagaattct ggcacccttg taaggaagaa aagagagttc 3360

aacaccttcg agtctgagcg cttgtggcta gagtttgcca ggagggagga aaccagtgac 3420

cctgaaaact gagggtgcct caggagcagt gggaccacct gatgctgaag gacggactaa 3480

tgatgtttcc tcttgccttc tctggtgcct ccattgccct catggaacag agcatatcat 3540

agagggagaa aagtcaaact tgtaattgtg tcttacagtt actggcttca tcttccttgg 3600

gatatatggt catcctctaa tgagtgtaaa agtgcgcaaa acacatcctt attgttcctg 3660

atctcttagt cccataaatg ggaacaaata cagctttctg cttctttctt tttggggaaa 3720

ggacagggtg ctagtgagta ctgacagcat gccagctacc gaagtcaccc agccattccc 3780

atgagcagca gttcatttaa ttgtcacagc gtcgccagga agaagatctg ataaacctag 3840

gtttacagat aaagaaagca aaatgtagag atgttgttga ggtcacagag gtgactgcct 3900

aacttcagag cagggcttct gatcccttta agaaattaca gggccagccg ggcatggtgg 3960

ctcacgcccg taatcccagg gctttgggag gccttggcag gtggatcacc tgagatcgca 4020

cgttcgagac cagcctgacc aacatggaga aaccccatct ctactaaaaa cacaaattag 4080

ccaggcgtgg tggtacatgc ctgtaatccc agctactcag gaggctgagg caggagaatc 4140

acttgacccc aggagacgta ggttgtggtg agctgagatc gcgccattgc actccagcct 4200

gggcaacaag agcaaaactc cgtctcaaaa aagaaaagaa aagaaaagaa atcatagggc 4260

caagttcaaa ggaaatgcac agaacatatc ttcacattag agttaagaat tctctagcaa 4320

acaacagatt tttttgttgt tgttagtcac aaatacttag aactggaagg ctctttgtta 4380

ttattgaatg tacccctcag ccttctcagc atttccttat cccaagacta gtgtgctttc 4440

tgctacactg ctagttttca gttttgttct tacccaattg ttttttcttt tcaacattac 4500

caatttacag attcagttta ttacatttac attaatcctc acttatgatt tgagcaagct 4560

catttccaga aaagtttact ttaagatcat caataggatt tgctaatttc agtgaagtca 4620

ttttgcttca ggggtaaatt atcctagtta ccaagtccta tttggacata aagaaaatcc 4680

tacttataga aaaggagaaa ataattaaac agtcttcatt tttaagtaac tgatttaaaa 4740

ggaaaataat aaaatatgtt cgtttatcat ttcagaaatt gctgtaacac actggaaaat 4800

tcctgaacaa tatagatttt atcgttaata aaaaacacta gctttcgttc cttagaatgt 4860

cttttctttt gaataaacag tattgggtga tttaaaaaaa aaaaaaaaaa aaaa 4914

SEQ ID NO: 171
<211> 1225
<212> DNA
<213> Homo sapiens

SEQ ID NO: 171

cagaaaagag gtggagaggg ggggaataag aaagagagag aaggaaagga gagaaggcag 60

gaagaaggca agggacgaga caaccatgct gtgctgtatg agaagaacca aacaggttga 120

aaaaaatgat gacgaccaaa agattgaaca agatggtatc aaaccagaag ataaagctca 180

taaggccgca accaaaattc aggctagctt ccgtggacac ataacaagga aaaagctcaa 240

aggagagaag aaggatgatg tccaagctgc tgaggctgaa gctaataaga aggatgaagc 300

ccctgttgcc gatggggtgg agaagaaggg agaaggcacc actactgccg aagcagcccc 360

agccactggc tccaagcctg atgagcccgg caaagcagga gaaactcctt ccgaggagaa 420

gaagggggag ggtgatgctg ccacagagca ggcagccccc caggctcctg catcctcaga 480

ggagaaggcc ggctcagctg agacagaaag tgccactaaa gcttccactg ataactcgcc 540

gtcctccaag gctgaagatg ccccagccaa ggaggagcct aaacaagccg atgtgcctgc 600

tgctgtcact gctgctgctg ccaccacccc tgccgcagag gatgctgctg ccaaggcaac 660

agcccagcct ccaacggaga ctggggagag cagccaagct gaagagaaca tagaagctgt 720

agatgaaacc aaacctaagg aaagtgcccg gcaggacgag ggtaaagaag aggaacctga 780

ggctgaccaa gaacatgcct gaactctaag aaatggcttt ccacatcccc accctcccct 840

ctcctgagcc tgtctctccc taccctcttc tcagctccac tctgaagtcc cttcctgtcc 900

tgctcacgtc tgtgagtctg tcctttccca cccactagcc ctctttctct ctgtgtggca 960

aacatttaaa aaaaaaaaaa aaaagcagga aagatcccaa gtcaaacagt gtggcttaaa 1020

catttttgt ttcttggtgt tgttatggca agtttttggt aatgatgatt caatcatttt 1080

gggaaattct tgcactgtat ccaagttatt tgatctggtg cgtgtggccc tgtgggagtc 1140

cactttcctc tctctctctc tctctgttcc aagtgtgtgt gcaatgttcc gttcatctga 1200

ggagtccaaa atatcgagtg aattc 1225

SEQ ID NO: 172
<211> 2110
<212> DNA
<213> Homo sapiens

SEQ ID NO: 172

ctgaggccca gcccccttcg cccgtttcca tcacgagtgc cgccagcatg tctgacaaac 60
tgccctacaa agtcgccgac atcggcctgg ctgcctgggg acgcaaggcc ctggacattg 120
ctgagaacga gatgccgggc ctgatgcgta tgcgggagcg gtactcggcc tccaagccac 180
tgaagggcgc ccgcatcgct ggctgcctgc acatgaccgt ggagacggcc gtcctcattg 240
agaccctcgt caccctgggt gctgaggtgc agtggtccag ctgcaacatc ttctccaccc 300
aggaccatgc ggcggctgcc attgccaagg ctggcattcc ggtgtatgcc tggaagggcg 360
aaacggacga ggagtacctg tggtgcattg agcagaccct gtacttcaag gacgggcccc 420
tcaacatgat tctggacgac gggggcgacc tcaccaacct catccacacc aagtacccgc 480
agcttctgcc aggcatccga ggcatctctg aggagaccac gactggggtc cacaacctct 540
acaagatgat ggccaatggg atcctcaagg tgcctgccat caatgtcaat gactccgtca 600
ccaagagcaa gtttgacaac ctctatggct gccgggagtc cctcatagat ggcatcaagc 660
gggccacaga tgtgatgatt gccggcaagg tagcggtggt agcaggctat ggtgatgtgg 720
gcaagggctg tgcccaggcc ctgcggggtt tcggagcccg cgtcatcatc accgagattg 780
accccatcaa cgcactgcag gctgccatgg agggctatga ggtgaccacc atggatgagg 840
cctgtcagga gggcaacatc tttgtcacca ccacaggctg tattgacatc atccttggcc 900
ggcactttga gcagatgaag gatgatgcca ttgtgtgtaa cattggacac tttgacgtgg 960
agatcgatgt caagtggctc aacgagaacg ccgtggagaa ggtgaacatc aagccgcagg 1020
tggaccggta tcggttgaag aatgggcgcc gcatcatcct gctggccgag ggtcggctgg 1080
tcaacctggg ttgtgccatg ggccacccca gcttcgtgat gagtaactcc ttcaccaacc 1140
aggtgatggc gcagatcgag ctgtggaccc atccagacaa gtaccccgtt ggggttcatt 1200
tcctgcccaa gaagctggat gaggcagtgg ctgaagccca cctgggcaag ctgaatgtga 1260

agttgaccaa gctaactgag aagcaagccc agtacctggg catgtcctgt gatggcccct 1320
tcaagccgga tcactaccgc tactgagagc caggtctgcg tttcaccctc cagctgctgt 1380
ccttgcccag gccccacctc tcctccctaa gagctaatgg caccaacttt gtgactggtt 1440
tgtcagtgtc ccccatcgac tctctggggc tgatcactta gtttttggcc tctgctgcag 1500
ccgtcatact gttccaaatg tggcagcggg aacagagtac cctcttcaag ccccggtcat 1560
gatggaggtc ccagccacag ggaaccatga gctcagtggt cttggaacag ctcactaagt 1620
cagtccttcc ttagcctgga agccagtagt ggagtcacaa agcccatgtg ttttgccatc 1680
taggccttca cctggtctgt ggacttatac ctgtgtgctt ggtttacagg tccagtggtt 1740
cttcagccca tgacagatga gaaggggcta tattgaaggg caaagaggaa ctgttgtttg 1800
aattttcctg agagcctggc ttagtgctgg gccttctctt aaacctcatt acaatgaggt 1860
tagtactttt agtccctgtt ttacaggggt tagaatagac tgttaagggg caactgagaa 1920
agaacagaga agtgacagct aggggttgag aggggccaga aaaacatgaa tgcaggcaga 1980
tttcgtgaaa tctgccacca ctttataacc agatggttcc tttcacaacc ctgggtcaaa 2040
aagagaataa tttggcctat aatgttaaaa gaaagcagga aggtgggtaa ataaaaatct 2100
tggtgcctgg 2110

SEQ ID NO: 173
<211> 1953
<212> DNA
<213> Homo sapiens

SEQ ID NO: 173

ggagagagag aggacagaga gcaagtcact cccggctgcc tttttcacct ctgacagagc 60
ccagacacca tgaacgcaag tgaattccga aggagaggga aggagatggt ggattacgtg 120
gccaactaca tggaaggcat tgagggacgc caggtctacc ctgacgtgga gcccgggtac 180
ctgcggccgc tgatccctgc cgctgcccct caggagccag acacgtttga ggacatcatc 240
aacgacgttg agaagataat catgcctggg gtgacgcact ggcacagccc ctacttcttc 300
gcctacttcc ccactgccag ctcgtacccg gccatgcttg cggacatgct gtgcggggcc 360
attggctgca tcggcttctc ctgggcggca agcccagcat gcacagagct ggagactgtg 420

atgatggact ggctcgggaa gatgctggaa ctaccaaagg catttttgaa tgagaaagct 480

ggagaagggg gaggagtgat ccagggaagt gccagtgaag ccaccctggt ggccctgctg 540

gccgctcgga ccaaagtgat ccatcggctg caggcagcgt ccccagagct cacacaggcc 600

gctatcatgg agaagctggt ggcttactca tccgatcagg cacactcctc agtggaaaga 660

gctgggttaa ttggtggagt gaaattaaaa gccatcccct cagatggcaa cttcgccatg 720

cgtgcgtctg ccctgcagga agccctggag agagacaaag cggctggcct gattcctttc 780

tttatggttg ccaccctggg gaccacaaca tgctgctcct ttgacaatct cttagaagtc 840

ggtcctatct gcaacaagga agacatatgg ctgcacgttg atgcagccta cgcaggcagt 900

gcattcatct gccctgagtt ccggcacctt ctgaatggag tggagtttgc agattcattc 960

aactttaatc cccacaaatg gctattggtg aattttgact gttctgccat gtgggtgaaa 1020

aagagaacag acttaacggg agcctttaga ctggacccca cttacctgaa gcacagccat 1080

caggattcag ggcttatcac tgactaccgg cattggcaga taccactggg cagaagattt 1140

cgctctttga aaatgtggtt tgtatttagg atgtatggag tcaaaggact gcaggcttat 1200

atccgcaagc atgtccagct gtcccatgag tttgagtcac tggtgcgcca ggatccccgc 1260

tttgaaatct gtgtggaagt cattctgggg cttgtctgct ttcggctaaa gggttccaac 1320

aaagtgaatg aagctcttct gcaaagaata aacagtgcca aaaaaatcca cttggttcca 1380

tgtcacctca gggacaagtt tgtcctgcgc tttgccatct gttctcgcac ggtggaatct 1440

gcccatgtgc agcgggcctg ggaacacatc aaagagctgg cggccgacgt gctgcgagca 1500

gagagggagt aggagtgaag ccagctgcag gaatcaaaaa ttgaagagag atatatctga 1560

aaactggaat aagaagcaaa taaatatcat cctgccttca tggaactcag ctgtctgtgg 1620

cttcccatgt ctttctccaa agttatccag agggttgtga ttttgtctgc ttagtatctc 1680

atcaacaaag aaatattatt tgctaattaa aaagttaatc ttcatggcca tagcttttat 1740

tcattagctg tgatttttgt tgattaaaac attatagatt ttcatgttct tgcagtcatc 1800

agaagtggta ggaaagcctc actgatatat tttccagggc aatcaatgtt cacgcaactt 1860

gaaattatat ctgtggtctt caaattgtct tttgtcatgt ggctaaatgc ctaataaaca 1920

attcaagtga aatactaaaa aaaaaaaaaa aaa 1953

SEQ ID NO: 175
<211> 4285
<212> DNA
<213> Homo sapiens

SEQ ID NO: 175

gagtccgggg tcgccgcagc ccgggaggag tgtctggtct ccggcctgcc tgtgctgtcc 60

ccgcgccctg tccactggac tcccgagacc cttggaaccc aggacaccat tggagaaact 120

gggcatttta ccaaggattt gactggaatg gcatgcttcc tttaaagatg aaagttgact 180

tttagagcca attaaagccc tttggggaat ctggcctcat accttgtcca cacagagttc 240

ctgtacaagg ttcctggcct gtgggaagcg gcacagcacc agctaggcag agacgcccca 300

ggccatgtta gagctttgag tgaggcctgg taacagggag gcgctgtcac ctactggcct 360

tgccaatcca gctccaagat gctgagcctg aagctcccca ggctgtttag catagaccag 420

ataccccagg tgttccatga gcaaggcacc ctgttcggct accgccatcc acagagttct 480

gccactgcct gcatcctcag ccttttccaa atgaccaatg agactctcaa catttggact 540

cacttgctgc ccttctggtt ctttgcatgg aggtttgtga ctgcactgta tatgacagac 600

atcaagaatg acagctactc ctggcccatg cttgtgtaca tgtgcaccag ctgcgtgtac 660

ccacttgtgt ccagctgtgc gcacaccttc agctctatgt ccaagaatgc ccggcacatt 720

tgctacttcc tggactatgg tgccgtcaac ctcttcagcc tgggctcagc cattgcctac 780

tctgcataca cgttcccgga tgcgctcatg tgcaccactt tccatgacta ctacgtggcc 840

ctggctgtac tgaacaccat cctcagcaca ggcctctcct gctactccag gtttcttgaa 900

atccagaagc ccagactctg taaggtgatt cgtgtcctcg cctttgctta tccgtacacc 960

tgggactccc tccccatctt ctacaggcta ttcctgttcc caggggagag tgcacaaaat 1020

gaagccacct cgtaccacca gaagcacatg atcatgaccc tcctggcctc tttcttgtac 1080

tctgcacatc tgccagaacg cctagcccct ggacgctttg actacatcgg tcacagtcac 1140

cagctgtttc acgtgtgtgt gatcctggcc acgcacatgc agatggaagc catacttctg 1200

gacaagactc tgaggaagga atggctcctg gccacctcca agcccttctc tttctctcag 1260

atagctggag ccatacttct gtgcatcatc ttcagcctca gcaacataat ttatttctca 1320

gctgctctgt atcggattcc caagccagaa ttacataaaa aagaaacatg actcagacca 1380

taagcttttc atgccagatg tcaacattaa gctgcaacat cctaaccacc ataagccgga 1440

ggtggttaca gcttatcacg gcctaaaata ttcataatgg ttggtgtctt ttgaatgaat 1500

tcatgtcaaa aatgttattc agctggggaa atttctctaa atgtacactg attctgtgtg 1560

tgtgatttta aaaggagaac atggttcaag caagtccttg ttaaggcaaa ctattgatat 1620

ttcattaatt ttaaatttac ttaaaatgtg gttttaaatt ctatttaaac atttggatta 1680

agcatattac tctggagctt tgtattattc tttaaaaatg aatcttgctt catttgatga 1740

atttcatgtc acaaagcttc tctcaagagc tctcgtagtg gcctgtcaac cactttcgga 1800

gaatgagcca ggtcagtcac tgggaggacc tgtgaaagaa agttggtcac tgagtgcctt 1860

ggggactggc aaaacattcc agaatgaaga gagctgtctg ggagagcccc ttcttccctt 1920

ctccacctag tgaagggaga acagaagggg agtcctctct tcccacaagt ccattctgcc 1980

ttcaaagtgt aagttctcat gtcattatgg atttggagtt tgtcagtttt tttcttaaaa 2040

gtccacagcg gggtgtcatg tgttctgtta tgttctgttt tctggttgcc agtttgatct 2100

gcaaagtatt tttcagtgca ggtaaaatgt gttaaaccat aagagttgac tgagttaatc 2160

ccaattggtt gtgctcatat gccacctttt taaaccaatg gaaattgcta tatttgggga 2220

tgtcatacat ttgatattgc ttcagcattt caaatatgtt gcaaaattta gtatccatat 2280

gcataagaag ttaatcacct tgccagtggg gtggtcacat atagtgttct caagctttac 2340

tgtgaatcag aaacatccag taagcttgtg acaaatgcag actcctaggg aaaaccaggg 2400

ggttctgctt cactaggtta aatgtaggtt ttgtagagga gatctgcact ggagcaagtc 2460

tcccagatga ttctaataca ggagggccag aggccaaact tgaagaactg ctggtgttac 2520

atgttccaaa aggcattaga cctatcagct ttgtttcgcc tgatctcaag ttttagcaac 2580

tcaccaacta aggcgtaacc tcattttatg ttccagcgtt ttctactctg aatagttcta 2640

aaacactgag cattttctca tttgttgcat tactgattaa agttcagtat ttcatggtgt 2700

tttcagggat cacagaaatg cggatgcaaa acaacttttg acagaggccc atcaaaccca 2760

gatctgcact tggtaatgac ttagggggc ttcctcacaa tgcaggatcc tctttgctga 2820

ctcaggaata ctccatattt tagactactg cctctaccat taactagccc agacatttga 2880

atggccttgt aaacagctca ctgtctaata ctcgaaggac aagtaacaat ttatattgga 2940

tgagagtggt caagaaatat gatttcatta gttttattat ctgtttactt cctctgggga 3000

aggcagactg ataaaattat atgggctggt aaatttttgg tacattaatt gcaagctgca 3060

tgacaaaatt tgtttttctg gcaacaatct gacattatta gtggaattcc acaccaacag 3120

ggcttgtgag cttccatttc atcttactgt aacttcaatt ttatggcaaa aaaaggtaca 3180

aagtttaaat acccctttct agtaatgcca aagacataca gtgttttaca ttctctcctt 3240

tataggtaac aaaataatgt ttagctcagt aagtactgaa ataatgagtg accaatttga 3300

aggattttta gattatttaa tctttgctta tgttgctttt tcacatgctt aatgatggaa 3360

atgaaatgtt aaatcaacaa aataagatcc agtgtttcat aacattttta taagtttggt 3420

aaactttagt cccattatat acttttgggg acagtgttat aaatcagaat tttacgacag 3480

tttgcagaac actgatttga aagcttccta tgcaaaatga gaaggggttc aaatatatta 3540

attatcatta agtattaaat aataggcatt agatgtctaa tgtgagtata atttcatcca 3600

agccatctca gaaagtctaa aaggttggca gggggtcagc tgaagacctc actggagtgg 3660

gtcttaattt ttaaaaagtg tctcactaca cttaagacat gtgacacatt cccattggta 3720

acaattgctc accatggcat tgtctcaaaa aagactatgg tggggagggt ggcacattcc 3780

taatgggatg tacctctccc cactcccctt gcttgagaag cactactcaa aaatttacca 3840

gaccttacaa ctgtgataaa acttgaatgt gtatgatgaa taaggccatt cagttaactt 3900

tacctccctt tacccattga aggggttcct tttccattgc tcagttttta gaaaataatt 3960

ctcatctttt ttctaaagaa atgaatttgt ggctggtctg aaggtagtga gttagctcaa 4020

ttgattgttc gcagtcagtt atagatcaag ctccttgttc tactcttccc ctcttctcac 4080

tactgcactt gactagtctt aaaacaaaga aagaacgaat gaatgaatga atctgtggct 4140

ggatgcagtg gctcgtgcct gtaatcccag cactttggga ggctgaagtg ggaggactgc 4200

ttgggcccag gaattcagga ccagtctggg cagcagggcc agacactgtc tctataaaaa 4260

aaaaaaaaaa aaaaaaaaaa aaaaa 4285

SEQ ID NO: 179
<211> 2641
<212> DNA
<213> Homo sapiens

SEQ ID NO: 179

gagccagttc tgggaggcgg ggggaaggag gttggtggcg actccctcgc tcgccctcac 60

tgccggcggt cccaactcca ggcaccatgt tccccgcggg cccccccagc cacagcctcc 120

tccggctccc cctgctgcag ttgctgctac tggtggtgca ggccgtgggg agggggctgg 180

gccgcgccag cccggccggg ggcccccctgg aagatgtggt catcgagagg taccacatcc 240

ccagggcctg tccccgggaa gtgcagatgg gggattttgt gcgctaccac tacaacggca 300

cttttgaaga tggcaagaag tttgattcaa gctatgatcg caacaccttg gtggccatcg 360

tggtgggtgt ggggcgcctc atcactggca tggaccgagg cctcatgggc atgtgtgtca 420

acgagcggcg acgcctcatt gtgcctcccc acctgggcta tgggagcatc ggcctggcgg 480

ggctcattcc accggatgcc accctctact tcgatgtggt tctgctggat gtgtggaaca 540

aggaagacac cgtgcaggtg agcacattgc tgcgcccgcc ccactgcccc cgcatggtcc 600

aggacggcga ctttgtccgc taccactaca atggcaccct gctggacggc accttcttcg 660

acaccagcta cagtaagggc ggcacttatg acacctacgt cggctctggt tggctgatca 720

agggcatgga ccaggggctg ctgggcatgt gtcctggaga gagaaggaag attatcatcc 780

ctccattcct ggcctatggc gagaaaggct atgggacagt gatcccccca caggcctcgc 840

tggtctttca cgtcctcctg attgacgtgc acaacccgaa ggacgctgtc cagctagaga 900

cgctggagct ccccccggc tgtgtccgca gagccggggc cggggacttc atgcgctacc 960

actacaatgg ctccttgatg gacggcaccc tcttcgattc cagctactcc cgcaaccaca 1020

cctacaatac ctatatcggg cagggttaca tcatccccgg gatggaccag gggctgcagg 1080

gtgcctgcat gggggaacgc cggagaatta ccatcccccc gcacctcgcc tatggggaga 1140

atggaactgg agacaagatc cctggctctg ccgtgctaat cttcaacgtc catgtcattg 1200

acttccacaa ccctgcggat gtggtggaaa tcaggacact gtcccggcca tctgagacct 1260

gcaatgagac caccaagctt ggggactttg ttcgatacca ttacaactgt tctttgctgg 1320

acggcaccca gctgttcacc tcgcatgact acggggcccc ccaggaggcg actctcgggg 1380

ccaacaaggt gatcgaaggc ctggacacgg gcctgcaggg catgtgtgtg ggagagaggc 1440

ggcagctcat cgtgcccccg cacctggccc acggggagag tggagcccgg ggagtcccag 1500

gcagtgctgt gctgctgttt gaggtggagc tggtgtcccg ggaggatggg ctgcccacag 1560

gctacctgtt tgtgtggcac aaggaccctc ctgccaacct gtttgaagac atggacctca 1620

acaaggatgg cgaggtccct ccggaggagt tctccacctt catcaaggct caagtgagtg 1680

agggcaaagg acgcctcatg cctgggcagg accctgagaa aaccatagga gacatgttcc 1740

agaaccagga ccgcaaccag gacggcaaga tcacagtcga cgagctcaag ctgaagtcag 1800

atgaggacga ggagcgggtc cacgaggagc tctgaggggc agggagcctg gccaggcctg 1860

agacacagag gcccactgcg agggggacag tggcggtggg actgacctgc tgacagtcac 1920

cctccctctg ctgggatgag gtccaggagc caactaaaac aatggcagag gagacatctc 1980

tggtgttccc accaccctag atgaaaatcc acagcacaga cctctaccgt gtttctcttc 2040

catccctaaa ccacttcctt aaaatgtttg gatttgcaaa gccaatttgg ggcctgtgga 2100

gcctggggtt ggatagggcc atggctggtc cccaccata cctcccctcc acatcactga 2160

cacagctgag cttgttatcc atctccccaa actttctctt tctttgtact tcttgtcatc 2220

cccactccca gcccctattc ctctatgtga cagctggcta ggacccctct gccttcctcc 2280

ccaatcctga ctggctccta gggaagggga aggctcctgg agggcagccc tacctctccc 2340

atgccctttg ccctcctccc tcgcctccag tggaggctga gctgaccctg ggctgctgga 2400

ggccagactg ggctgtagtt agcttttcat ccctaaagaa ggctttccct aaggaaccat 2460

agaagagagg aagaaaacaa agggcatgtg tgagggaagc tgcatgggtg ggtgttaggg 2520

ctatgaaatc ttggatttgg ggctgagggg tgggagggag ggcagagctc tgcacactca 2580

aaggctaaac tggtgtcagt cctttttcc tttgttccaa ataaaagatt aaaccaatgg 2640

c 2641

SEQ ID NO: 183
<211> 1231
<212> DNA
<213> Homo sapiens

SEQ ID NO: 183

```
gacaagatgg ccacaccggc ggtaccagta agtgctcctc cggccacgcc aaccccagtc    60
ccggcggcgg ccccagcctc agttccagcg ccaacgccag caccggctgc ggctccggtt   120
cccgctgcgg ctccagcctc atcctcagac cctgcggcag cagcggctgc aactgcggct   180
cctggccaga ccccggcctc agcgcaagct ccagcgcaga ccccagcgcc cgctctgcct   240
ggtcctgctc ttccagggcc cttccccggc ggccgcgtgg tcaggctgca cccagtcatt   300
ttggcctcca ttgtggacag ctacgagaga cgcaacgagg gtgctgcccg agttatcggg   360
accctgttgg gaactgtcga caaacactca gtggaggtca ccaattgctt ttcagtgccg   420
cacaatgagt cagaagatga agtggctgtt gacatggaat ttgctaagaa tatgtatgaa   480
ctgcataaaa aagtttctcc aaatgagctc atcctgggct ggtacgctac gggccatgac   540
atcacagagc actctgtgct gatccatgag tactacagcc gagaggcccc caaccccatc   600
cacctcactg tggacacaag tctccagaac ggccgcatga gcatcaaagc ctacgtcagc   660
actttaatgg gagtccctgg gaggaccatg ggagtgatgt tcacgcctct gacagtgaaa   720
tacgcgtact acgacactga acgcatcgga gttgacctga tcatgaagac ctgctttagc   780
cccaacagag tgattggact ctcaagtgac ttgcagcaag taggaggggc atcagctcgc   840
atccaggatg ccctgagtac agtgttgcaa tatgcagagg atgtactgtc tggaaaggtg   900
tcagctgaca atactgtggg ccgcttcctg atgagcctgg ttaaccaagt accgaaaata   960
gttcccgatg actttgagac catgctcaac agcaacatca atgacctttt gatggtgacc  1020
tacctggcca acctcacaca gtcacagatt gcactcaatg aaaaacttgt aaacctgtga  1080
atggacccca agcagtacac ttgctggtct aggtattaac cccaggactc agaagtgaag  1140
gagaaatggg ttttttgtgg tcttgagtca cactgagata gtcagttgtg tgtgactcta  1200
ataaacggag cctacctttt gtaaaaaaaa a                                 1231
```

SEQ ID NO: 188
<211> 1429

<212> DNA
<213> Homo sapiens

SEQ ID NO: 188

```
cgccgagaac cccacccccct ttctttgcgg aatcaccatg gcggctggga ccctgtacac 60
gtatcctgaa aactggaggg ccttcaaggc tctcatcgct gctcagtaca gcggggctca 120
ggtccgcgtg ctctccgcac caccccactt ccattttggc caaaccaacc gcacccctga 180
atttctccgc aaatttcctg ccggcaaggt cccagcattt gagggtgatg atggattctg 240
tgtgtttgag agcaacgcca ttgcctacta tgtgagcaat gaggagctgc ggggaagtac 300
tccagaggca gcagcccagg tggtgcagtg ggtgagcttt gctgattccg atatagtgcc 360
cccagccagt acctgggtgt tccccacctt gggcatcatg caccacaaca aacaggccac 420
tgagaatgca aaggaggaag tgaggcgaat tctggggctg ctggatgctt acttgaagac 480
gaggactttt ctggtgggcg aacgagtgac attggctgac atcacagttg tctgcaccct 540
gttgtggctc tataagcagg ttctagagcc ttctttccgc caggcctttc ccaataccaa 600
ccgctggttc ctcacctgca ttaaccagcc ccagttccgg gctgtcttgg gcgaagtgaa 660
actgtgtgag aagatggccc agtttgatgc taaaaagttt gcagagaccc aacctaaaaa 720
ggacacacca cggaaagaga agggttcacg ggaagagaag cagaagcccc aggctgagcg 780
gaaggaggag aaaaaggcgg ctgcccctgc tcctgaggag gagatggatg aatgtgagca 840
ggcgctggct gctgagccca aggccaagga ccccttcgct cacctgccca agagtacctt 900
tgtgttggat gaatttaagc gcaagtactc caatgaggac acactctctg tggcactgcc 960
atatttctgg gagcactttg ataaggacgg ctggtccctg tggtactcag agtatcgctt 1020
ccctgaagaa ctcactcaga ccttcatgag ctgcaatctc atcactggaa tgttccagcg 1080
actggacaag ctgaggaaga atgccttcgc cagtgtcatc ctttttggaa ccaacaatag 1140
cagctccatt tctggagtct gggtcttccg aggccaggag cttgcctttc cgctgagtcc 1200
agattggcag gtggactacg agtcatacac atggcggaaa ctggatcctg gcagcgagga 1260
gacccagacg ctggttcgag agtacttttc ctgggagggg gccttccagc atgtgggcaa 1320
```

agccttcaat cagggcaaga tcttcaagtg aacatctctt gccatcacct agctgcctgc 1380

acctgccctt cagggagatg gggtcatta aaggaaactg aacattgaa 1429

SEQ ID NO: 191
<211> 3051
<212> DNA
<213> Homo sapiens

SEQ ID NO: 191

gagtgcaaga aagaaagaga tggagaacga gagcgagctg gagatgaacc acatgcgatg 60

agtaggcctt gtttggatcc tgaatcgaac acaccaactg taaaaatatg tttaagacgc 120

atcgggaaaa ttgggacacg gattcgatat ttgatgtttt ttagggaatt gatgtcagtt 180

tttttaggcg tcacagtagt attgtgatta tgttttcaaa ttgtcctttt ttgtagagac 240

atacgaaaat atttacggat taaataatgt ctgggattgg cttctaaata cattaatgac 300

taggatttgc ttcaaaataa tctcagcggt agggggaaat ggggaggggt atagatgaaa 360

caaaattggc cctaaattaa taatattttt ttgctgagtg ataggcagtg ggttgcgtat 420

attaatctgc tttccttggt atacgtttaa atttttctat aataaaatac agaagtcaga 480

tattccggtg agcttgaaaa agtcgggggt ggggggggagc agtgggtggg gttatagatg 540

aaacaagatg gccttcagtt ggtaattgtt gaaagctgga tgatggattc gtgtaggttt 600

ataatactat ttcttttta tttcatccat ttgaaattat atttaaggaa agttaaaaaa 660

caaatttgtc agaaattata caaatgtaca ataaattaaa tttgaaaatg tggccacaag 720

aaaggaaaaa gaaacacttg tacattattt atcagctttg gtgtcctttg tgtgtgatga 780

aattgcattg gctgatgtag aagaaagcca tatctcatat ctttttattt tatgttcttt 840

cttgtccttt tgtttgacct tctaggtcac catcagaaaa gctaagtttg ctgtatagtg 900

aggatcagga gatctgatcc tgattgcaga accttccctg attacagaat cttgggtaag 960

tgcctccctt ctgtcctcag ttctcaaaca ggataatacc acataacctt cctaactgtc 1020

caggaatatt ttgaaaatta ataagctcct atctgggaaa gtagtctaaa ttctgagaag 1080

ggaagggtgg agctaagtcc attgatagtt ccagtataga aagtgcataa gcaacagagg 1140

gctttgtaat cttacatccc ttgataaaag atactacagt caatctcctg tagtagttcc 1200

acagttccat agattacatt tttccttgga gcatcctata tgcagcatag tttagtggtt 1260

aagagcaaat acttcctgaa tttaaatcct ggctctgcca cttaactatg tgatcttggg 1320

caagatattt actcacttta tacctaagtt cctcgtatat gaaacagagg tgataataat 1380

agttcctact tcataggatt gttgagagga ttacatgtaa agtaccagga çagtgcatgg 1440

cacatgtaag tatttgcttt aataataatt atggttctgt tagtcctgat aatctcatgt 1500

tttatctaca catttacacc tacttctaaa agcagtggat atatttcttt ttggaattgt 1560

gtaaaaaaaa aaataataat taataccgtt ggtttctctc ctcattttcc agaagcagca 1620

gttaccacta gaactgaatt ccgaaattat gacttctggc ttgtcttaac aatctagaaa 1680

ggtttcaaat atattgatca tatttattta tgagggaatt tccaggagct ataattttag 1740

ctagcagttc aaaccaaatt tataaataag caaatctttt cactgaatat tcagtctgct 1800

aacagctttt gtatcattcc tcctttgtct cagacttaaa aaaaaaaatc tattacatgt 1860

aatggaaatg tttcaagatc cagaagagaa aattgatatg aatgtaactg atatggtata 1920

tgtgcattta aaggtttatt ccaacaaaat gttactcaca ctcttttctg acacatgcat 1980

acacaaaaga tgttagcatt tgcagtctga ttcccaaatg gcccattgct tgtgtggaga 2040

gatgaagtag gatttgaagg tttttcagcc ttttgacagc tacatatgga gtcactgtaa 2100

tttccttttt tttctctctt ctgaaaagta ggtttcggag gagggcactg ccctcctgga 2160

agagatgcat tagatcggta ggcacagaat acctttacat gagaccattt agagaatgat 2220

taggggccaa aggtaggggg tggactgtta agccaacagg gactcagaga aagcaagggt 2280

cagggtgacc agaaatagag aaaaaaaagc ctcacagagg aagaggacct ggacctgagc 2340

cacagaggat gggtagaact tagaaggagg gaatgagccc agtctgaatg atatgtctac 2400

aaagtataca atatgcaatg atgattaact gaaaaattgg gctggccatt tgtgagatcc 2460

atgaataaaa gtagatcaga aagtcagtgt gagttttaaa tatatatatt ttattataaa 2520

acgttaatca cataatatgt gaaatcacta ttgataacct tttctattgg tcctagtgtt 2580

ttgctattta agataatact gcatcaaata ttcttagtgt agccatccat ttacaagtgt 2640

```
tattttcaag agcaaaattt atgcctttaa actgtgataa ttaaattaat actggtgaga   2700
actatcagat ataccactgt gacagaacag atggcctaga gacagtgata cctttaaaag   2760
ttaaataatt gctgagcgcg gtggcttacg cctgtaatcc cagcactttg ggaggccgag   2820
gtgtgtggat cacgaggtca ggagttcaag accagcctgg ccaagatggt gaaaccccgt   2880
ctctactaaa aatacaggtg ggcgcctata atcccagcta ctcaggaggc tgaggcagag   2940
aattgcttga acccaggagg cggaggttgc agtgagccaa gatcgtgcca ctgcactcca   3000
gcctaggtga cagcgagact ccgtctcaaa aaaagaaaaa aaaaaaaaaa a            3051
```

## Claims

1. A microarray for predicting prognosis of neuroblastoma, having 24 probes related to good prognosis which respectively hybridize to the gene transcripts of genes having the nucleotide sequences of SEQ ID NOs: 5, 152, 132, 145, 142, 160, 87, 16, 191, 51, 173, 163, 129, 37, 136, 171, 67, 78, 64, 130, 91, 28 and 22 and Accession No. NM_003617.1, and
30 probes related to poor prognosis which respectively hybridize to gene transcripts of genes having the nucleotide sequences of SEQ ID NOs: 183, 135, 11, 131, 105, 188, 18, 46, 7, 32, 13, 2, 3, 25, 124, 26, 175, 97, 96, 20, 172, 92, 83, 34, 179, 154, 125, 141 and 114 and Accession No. BC003629.1.

2. A method for predicting prognosis of neuroblastoma, comprising:

   labeling a gene transcript obtained from a tumor cell of a patient diagnosed as having neuroblastoma;
   bringing the labeled gene transcript into contact with the microarray of claim 1;
   measuring labeling signals of each of the gene transcripts hybridized to the probes related to good prognosis and the probes related to poor prognosis on the microarray; and
   determining a predicted prognosis based on the measured labeling signals, wherein detection of significant labeling signals for 24 or more of the probes related to good prognosis indicates a good prognosis and detection of significant labeling signals for 25 or more of the probes related to poor prognosis indicates a poor prognosis.

## Patentansprüche

1. Mikroarray zur Vorhersage der Prognose eines Neuroblastoms, umfassend 24 Sonden in Verbindung mit einer guten Prognose, die jeweils an die Gentranskripte von Genen mit den Nucleotidsequenzen der Seq.-ID Nr. 5, 152, 132, 145, 142, 160, 87, 16, 191, 51, 173, 163, 129, 37, 136, 171, 67, 78, 64, 130, 91, 28 und 22 und der Zugangsnummer NM_003617.1 hybridisieren, und
30 Sonden in Verbindung mit einer schlechten Prognose, die jeweils an Gentranskripte von Genen mit den Nucleotidsequenzen der Seq.-ID Nr. 183, 135, 11, 131, 105, 188, 18, 46, 7, 32, 13, 2, 3, 25, 124, 26, 175, 97, 96, 20, 172, 92, 83, 34, 179, 154, 125, 141 und 114 und der Zugangsnummer BC003629.1 hybridisieren.

2. Verfahren zur Vorhersage der Prognose eines Neuroblastoms, umfassend:

   das Markieren eines Gentranskripts, das aus einer Tumorzelle eines Patienten erhalten wurde, bei dem ein Neuroblastom diagnostiziert wurde;
   das In-Kontakt-Bringen des markierten Gentranskripts mit einem Mikroarray nach Anspruch 1;
   das Messen der Markierungssignale der einzelnen Gentranskripte, die an die Sonden in Verbindung mit einer guten Prognose und die Sonden in Verbindung mit einer schlechten Prognose hybridisiert sind, im Mikroarray; und
   das Bestimmen einer vorhergesagten Prognose basierend auf den gemessenen Markierungssignalen, wobei die Detektion von signifikanten Markierungssignalen für 24 oder mehr der Sonden in Verbindung mit einer guten Prognose eine gute Prognose anzeigt und die Detektion von signifikanten Markierungssignalen für 25 oder mehr der Sonden in Verbindung mit schlechter Prognose eine schlechte Prognose anzeigt.

**Revendications**

1. Microréseau pour prédire le pronostic de neuroblastome, ayant 24 sondes liées à un bon pronostic qui s'hybrident respectivement à des transcripts de gènes ayant la séquence de nucléotides des SEQ ID NOs: 5, 152, 132, 145, 142, 160, 87, 16, 191, 51, 173, 163, 129, 37, 136, 171, 67, 78, 64, 130, 91, 28 et 22 et le No. d'Accès NM_003617.1, et 30 sondes liées à un pronostic médiocre qui s'hybrident respectivement à des transcripts de gènes ayant les séquences de nucléotides de SEQ ID NOs: 183, 135, 11, 131, 105, 188, 18, 46, 7, 32, 13, 2, 3, 25, 124, 26, 175, 97, 96, 20, 172, 92, 83, 34, 179, 154, 125, 141 et 114 et le No. d'Accès BC003629.1.

2. Méthode pour prédire le pronostic de neuroblastome, comprenant:

   marquer un transcript de gène obtenu d'une cellule tumorale d'un patient chez lequel on a diagnostiqué un neuroblastome;
   amener le transcript de gène marqué en contact avec le microréseau de la revendication 1;
   mesurer des signaux de marquage de chacun des transcripts de gènes qui se sont hybridés aux sondes liées à un bon pronostic et aux sondes liées à un pronostic médiocre sur le microréseau; et
   déterminer un pronostic prédit sur la base des signaux de marquage mesurés, où la détection de signaux de marquage significatifs pour 24 sondes ou plus liées à un bon pronostic indique un bon pronostic et la détection de signaux de marquage significatifs pour 25 sondes ou plus liées à un pronostic médiocre indique un pronostic médiocre.

Fig. 1

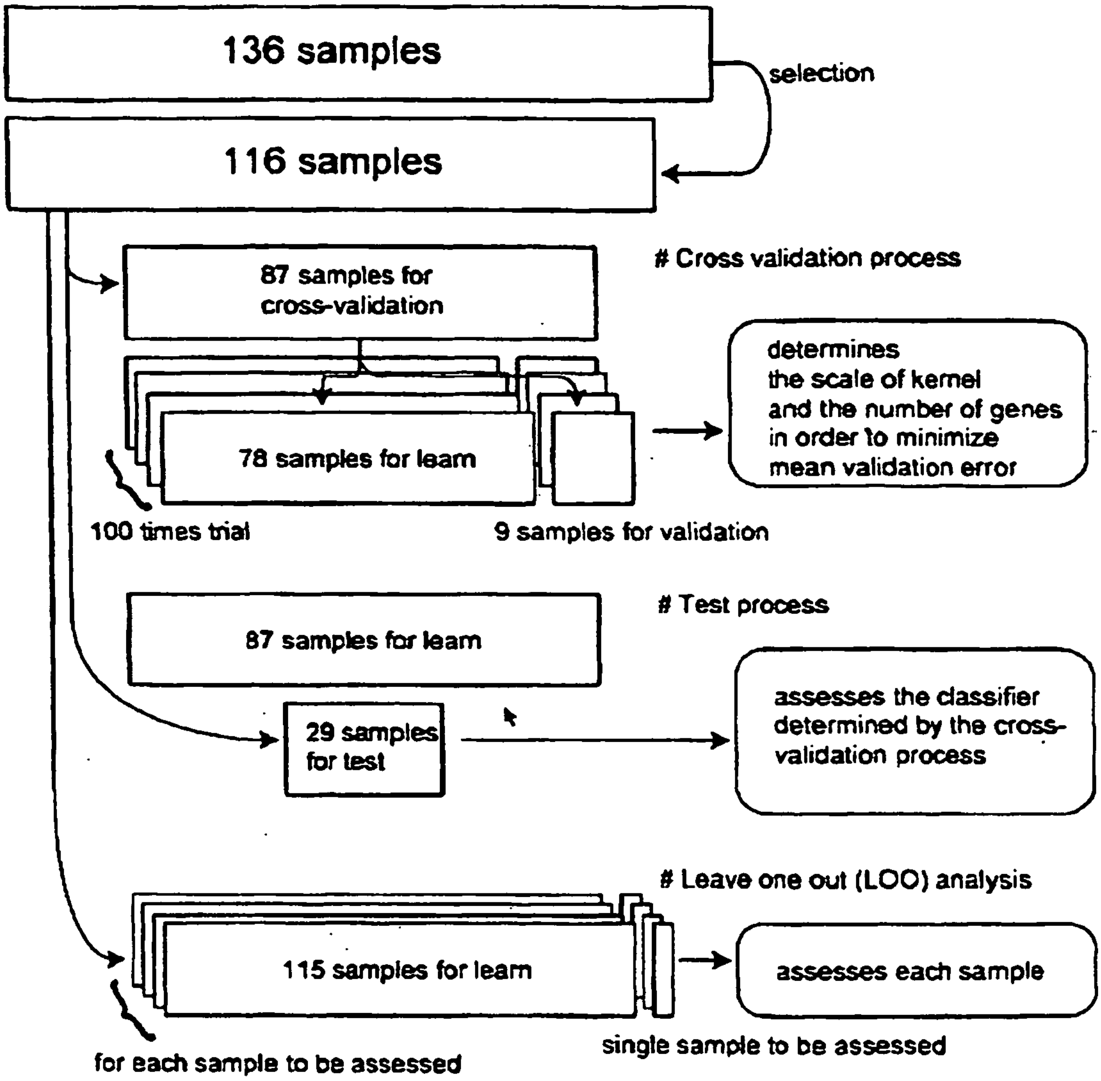
136 samples
selection
116 samples
87 samples for cross-validation
# Cross validation process
78 samples for learn
determines the scale of kernel and the number of genes in order to minimize mean validation error
100 times trial
9 samples for validation
# Test process
87 samples for learn
29 samples for test
assesses the classifier determined by the cross-validation process
# Leave one out (LOO) analysis
115 samples for learn
assesses each sample
for each sample to be assessed
single sample to be assessed

Fig. 2

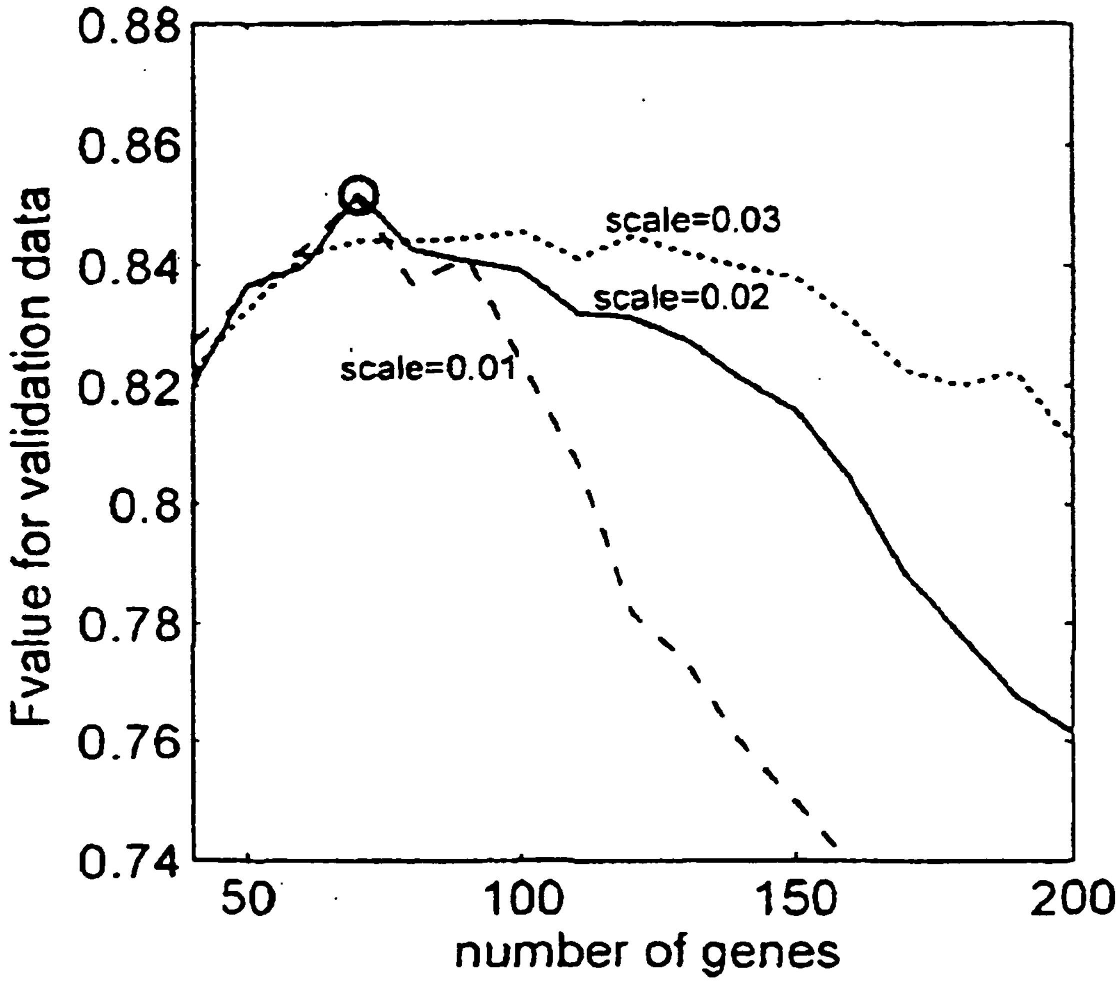
0.88
0.86
0.84
0.82
0.8
0.78
0.76
0.74
Fvalue for validation data
scale=0.03
scale=0.02
scale=0.01
50
100
150
200
number of genes

Fig. 3

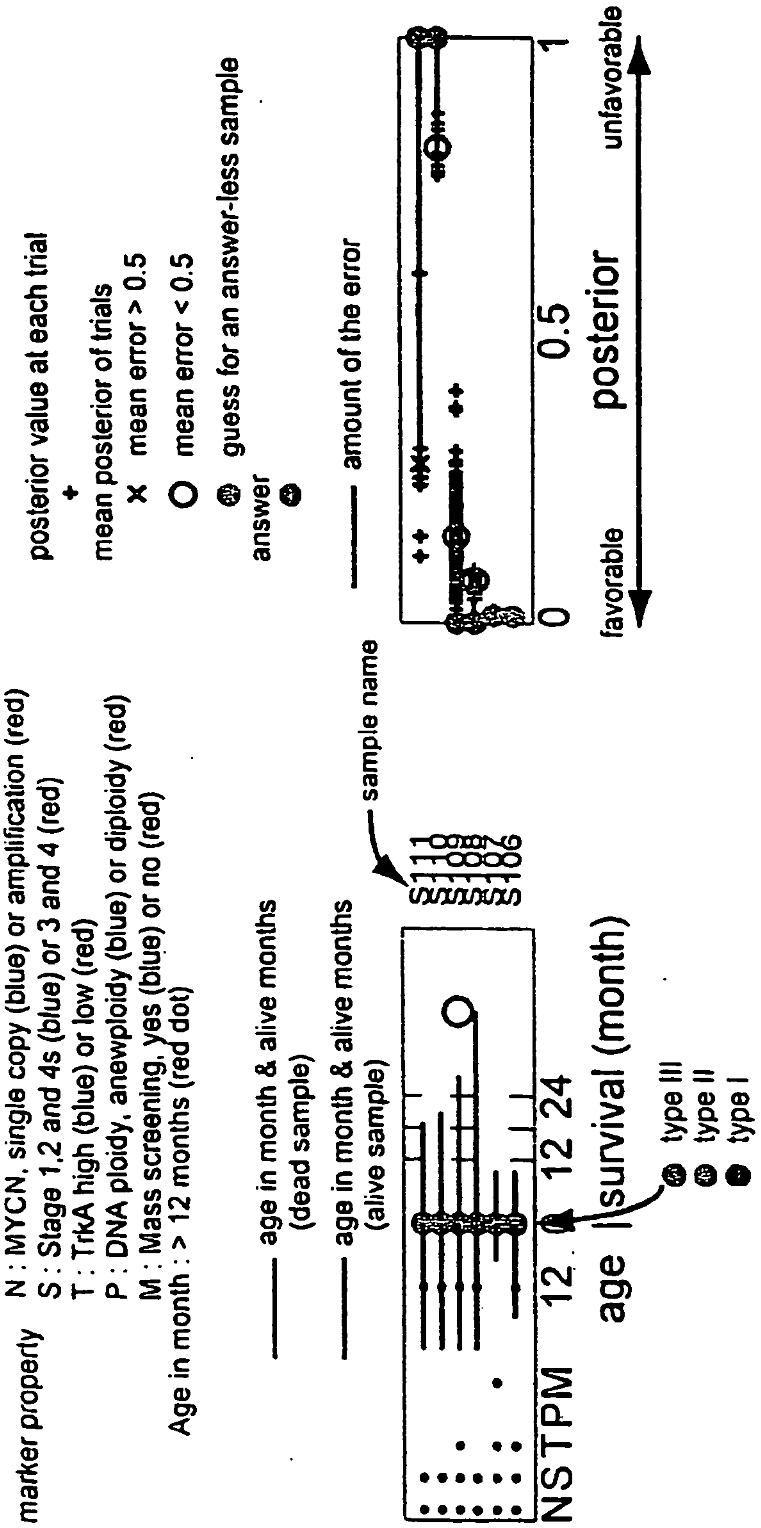

marker property
N : MYCN, single copy (blue) or amplification (red)
S : Stage 1,2 and 4s (blue) or 3 and 4 (red)
T : TrkA high (blue) or low (red)
P : DNA ploidy, anewploidy (blue) or diploidy (red)
M : Mass screening, yes (blue) or no (red)
Age in month : > 12 months (red dot)
posterior value at each trial
mean posterior of trials
mean error > 0.5
mean error < 0.5
guess for an answer-less sample
answer
age in month & alive months (dead sample)
age in month & alive months (alive sample)
sample name
amount of the error
S111
S109
S108
S107
S106
NSTPM
12
0
12
24
age
survival (month)
type III
type II
type I
0
0.5
1
posterior
favorable
unfavorable

Fig. 3/1

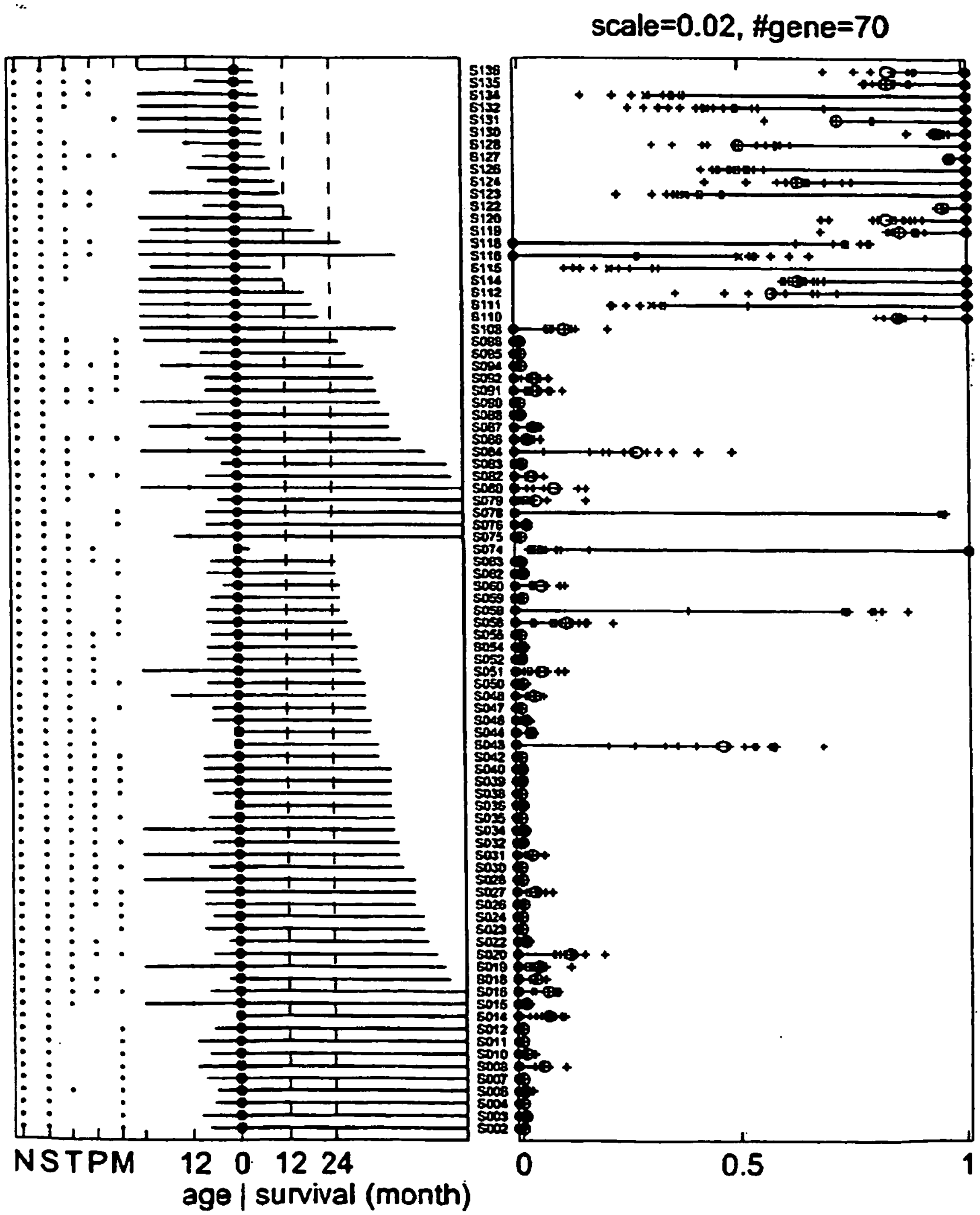

Fig. 4

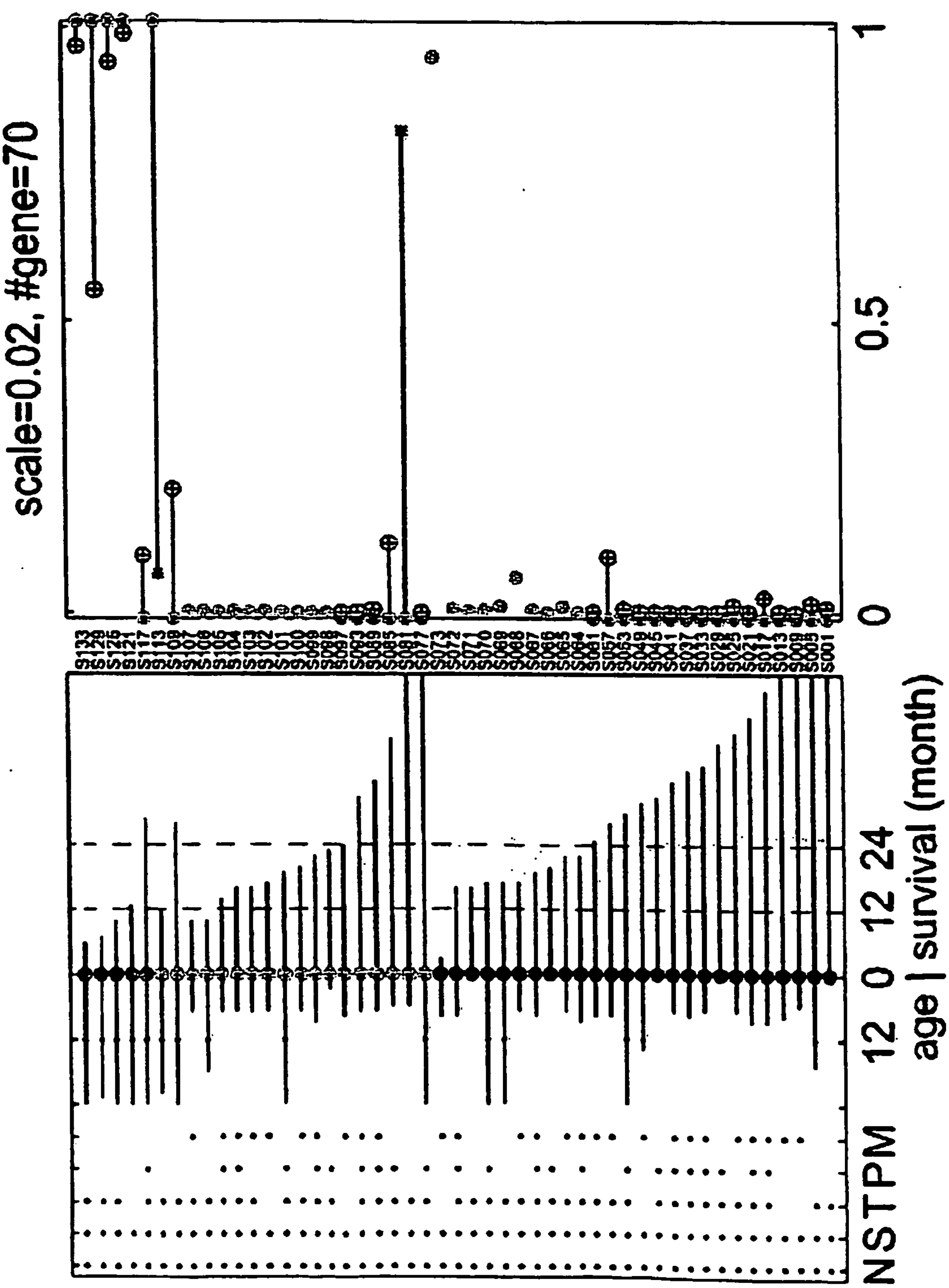

Fig. 5

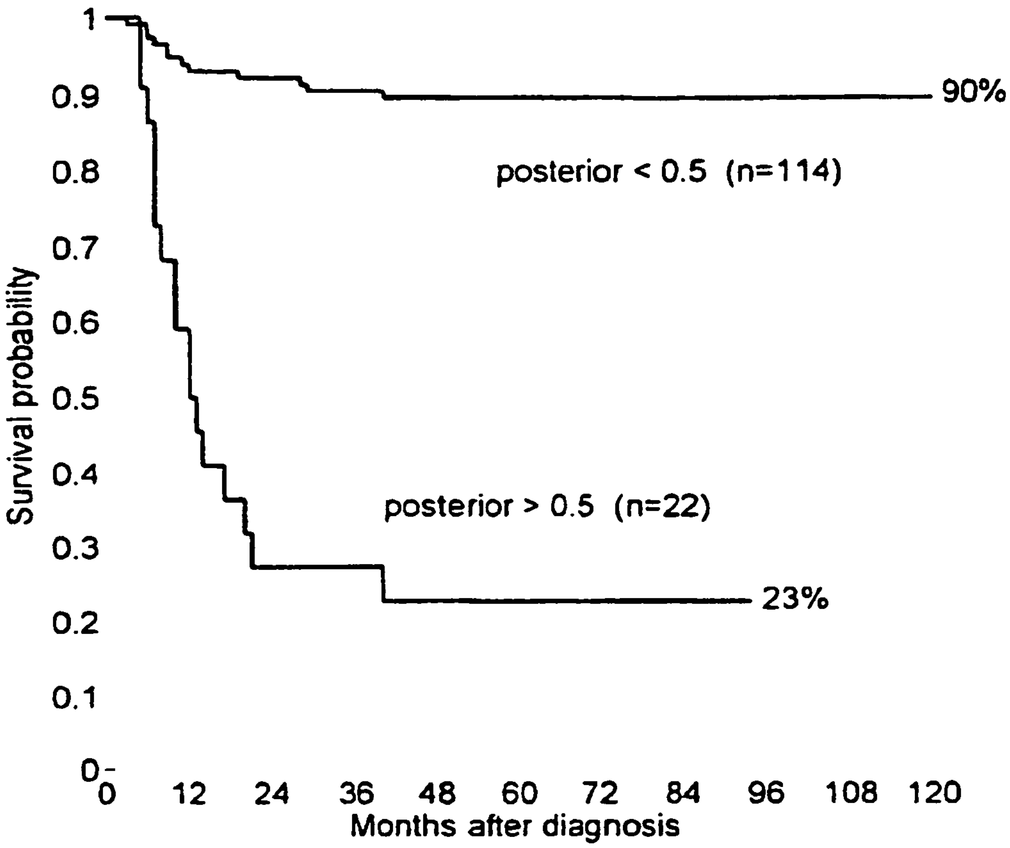
1
0.9
0.8
0.7
0.6
0.5
0.4
0.3
0.2
0.1
0
Survival probability
90%
posterior < 0.5 (n=114)
posterior > 0.5 (n=22)
23%
0
12
24
36
48
60
72
84
96
108
120
Months after diagnosis

Fig. 6

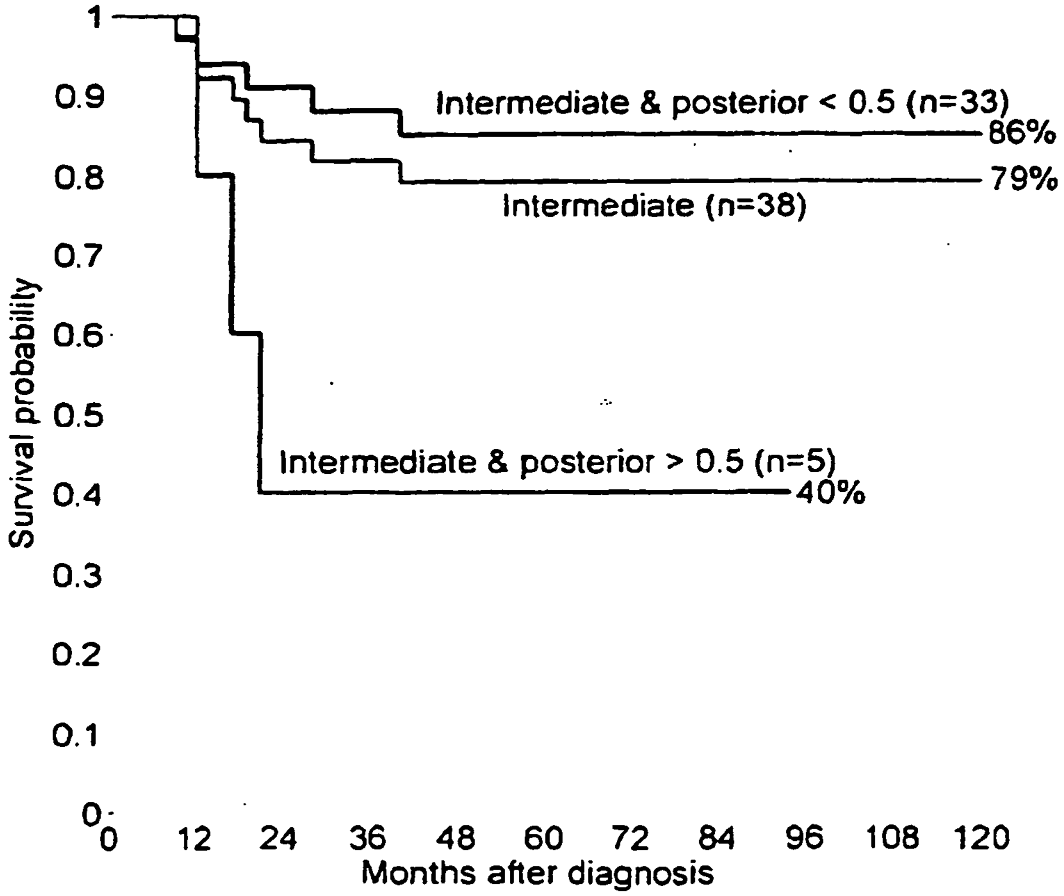
1
0.9
0.8
0.7
0.6
0.5
0.4
0.3
0.2
0.1
0
Survival probability
Intermediate & posterior < 0.5 (n=33)
86%
79%
Intermediate (n=38)
Intermediate & posterior > 0.5 (n=5)
40%
0
12
24
36
48
60
72
84
96
108
120
Months after diagnosis

Fig. 7

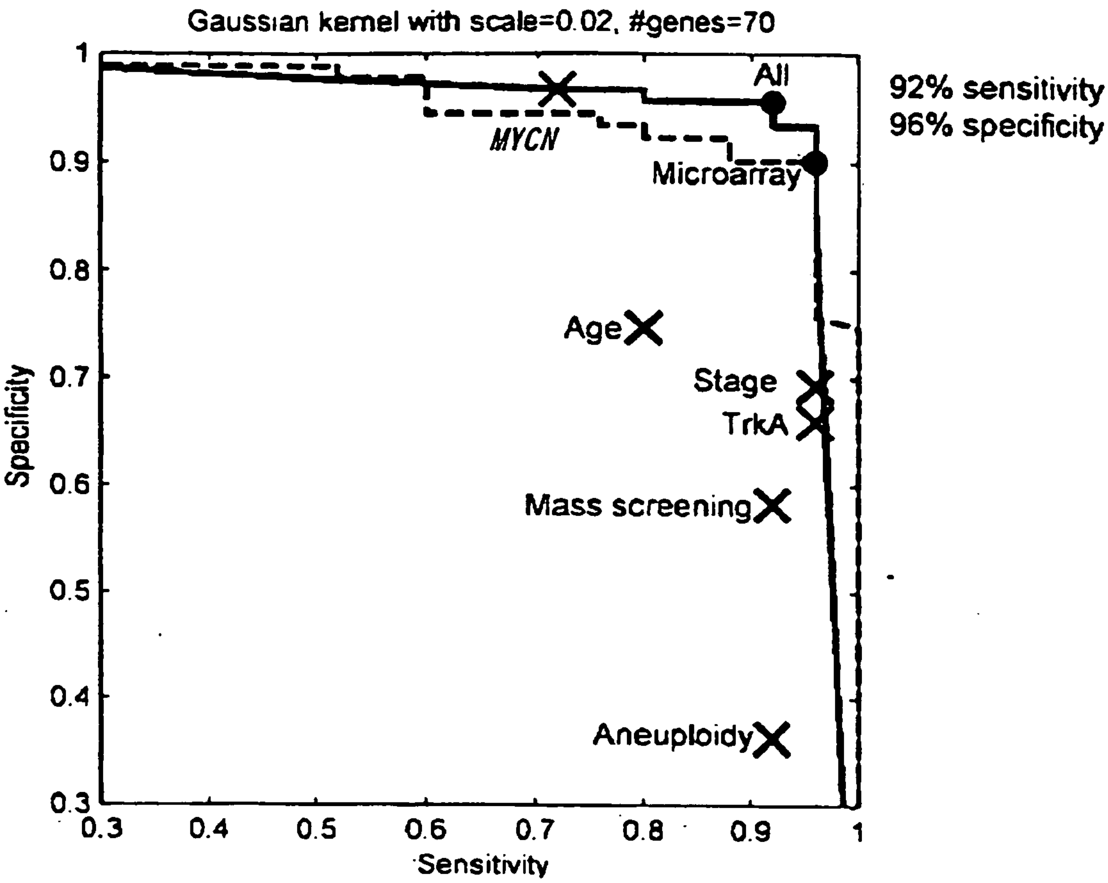
Gaussian kernel with scale=0.02, #genes=70
All
MYCN
Microarray
Age
Stage
TrkA
Mass screening
Aneuploidy
Specificity
Sensitivity
1
0.9
0.8
0.7
0.6
0.5
0.4
0.3
0.3
0.4
0.5
0.6
0.7
0.8
0.9
1
92% sensitivity
96% specificity

Fig. 8

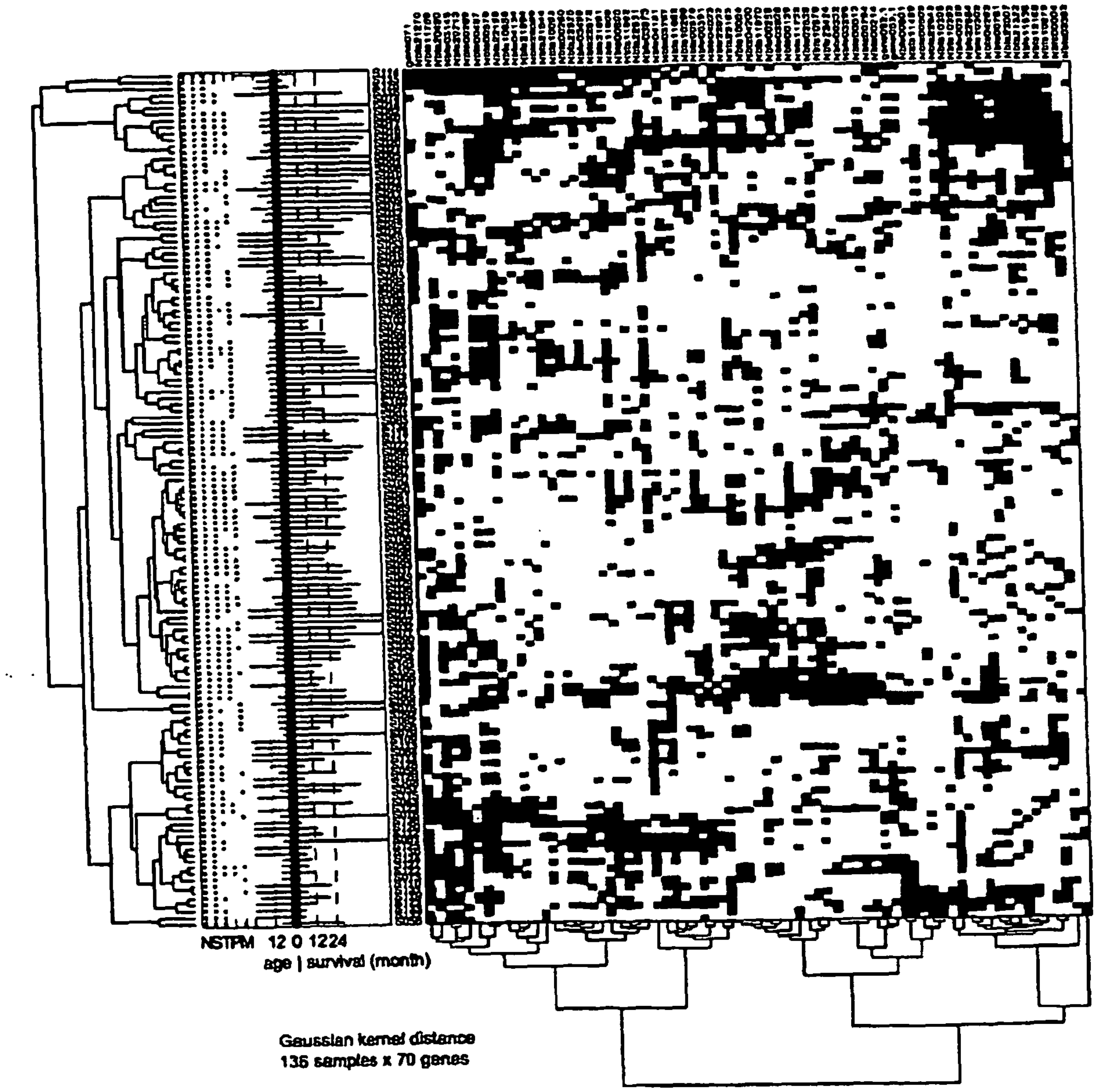
NSTPM 12 0 1224
age | survival (month)
Gaussian kernel distance
135 samples x 70 genes

Fig. 9

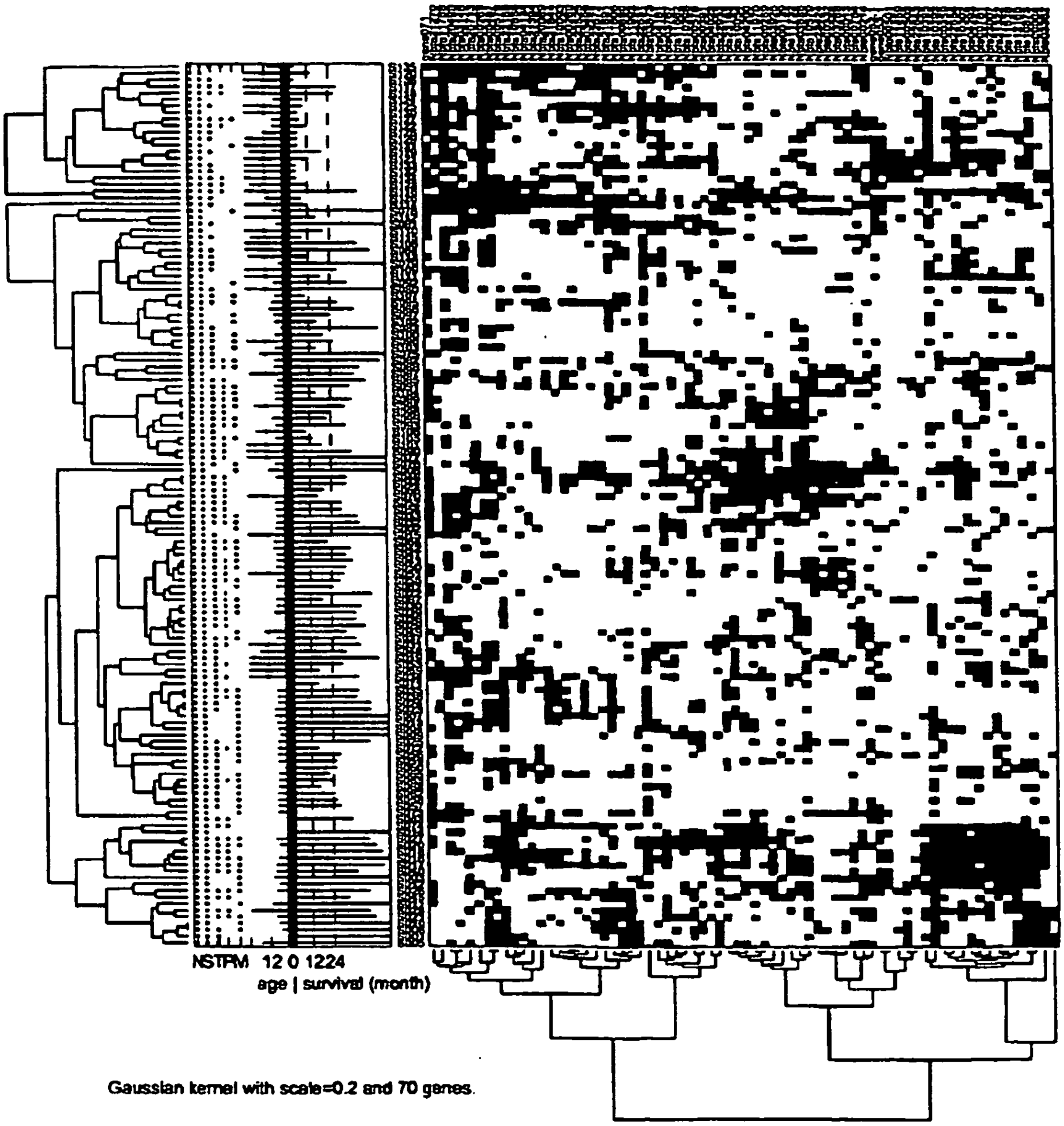


Gaussian kernel with scale=0.2 and 70 genes.

Fig. 1 0

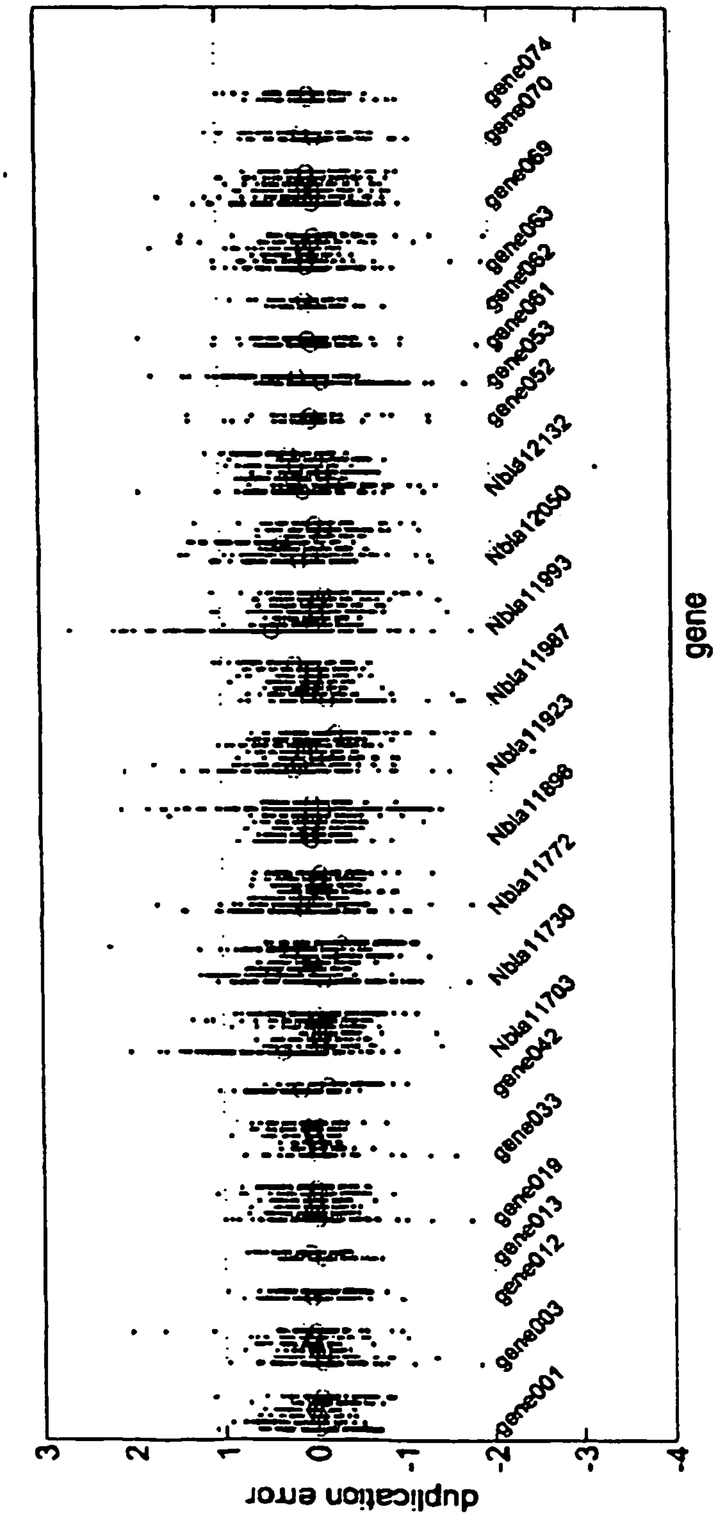
gene
duplication error
3
2
1
0
-1
-2
-3
-4
gene001
gene003
gene012
gene013
gene019
gene033
gene042
Nbla11703
Nbla11730
Nbla11772
Nbla11898
Nbla11923
Nbla11987
Nbla11993
Nbla12050
Nbla12132
gene052
gene053
gene061
gene062
gene063
gene069
gene070
gene074

Fig. 11

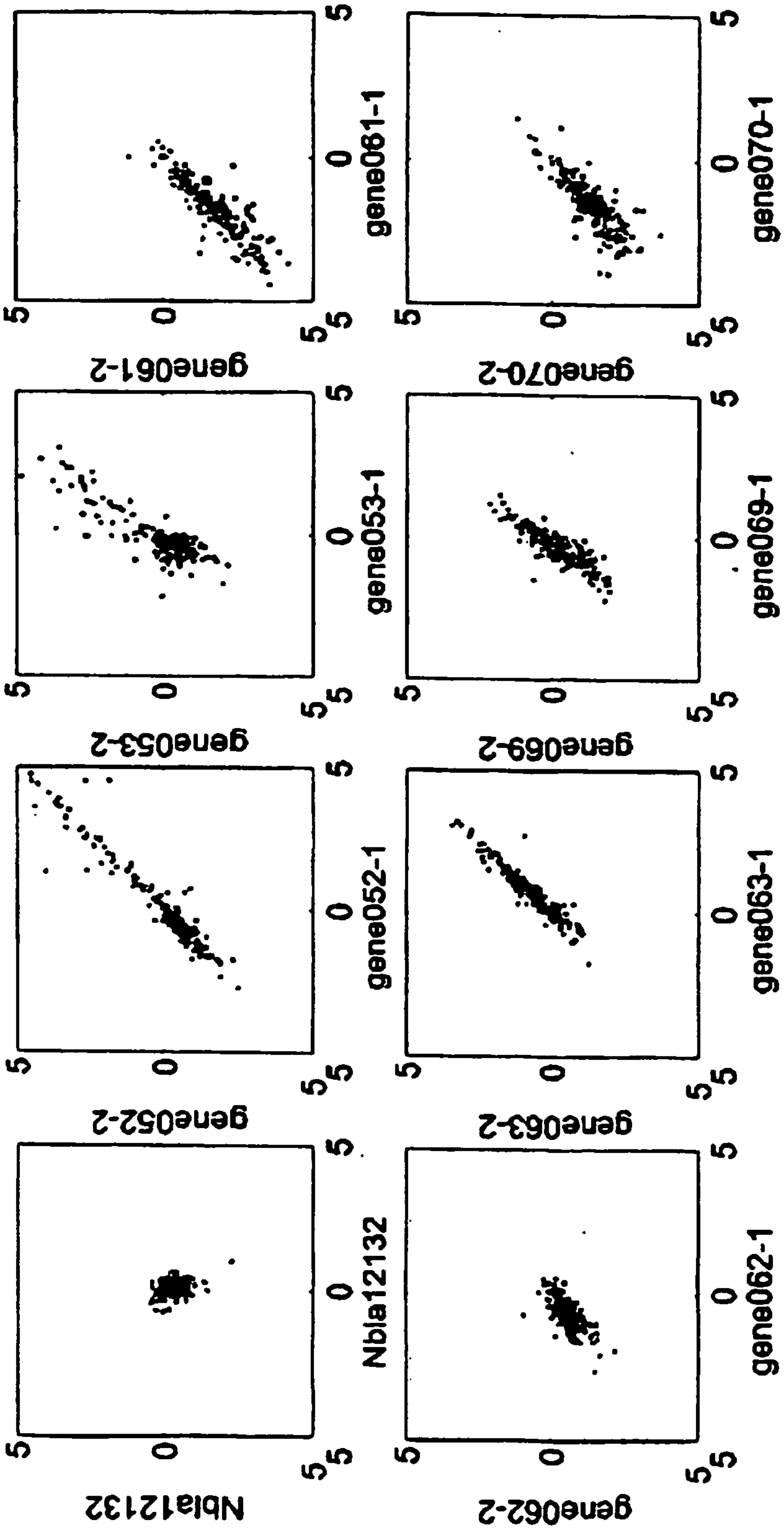
gene061-1
gene061-2
gene070-1
gene070-2
gene053-1
gene053-2
gene069-1
gene069-2
gene052-1
gene052-2
gene063-1
gene063-2
Nbla12132
Nbla12132
gene062-1
gene062-2

Fig. 1 2

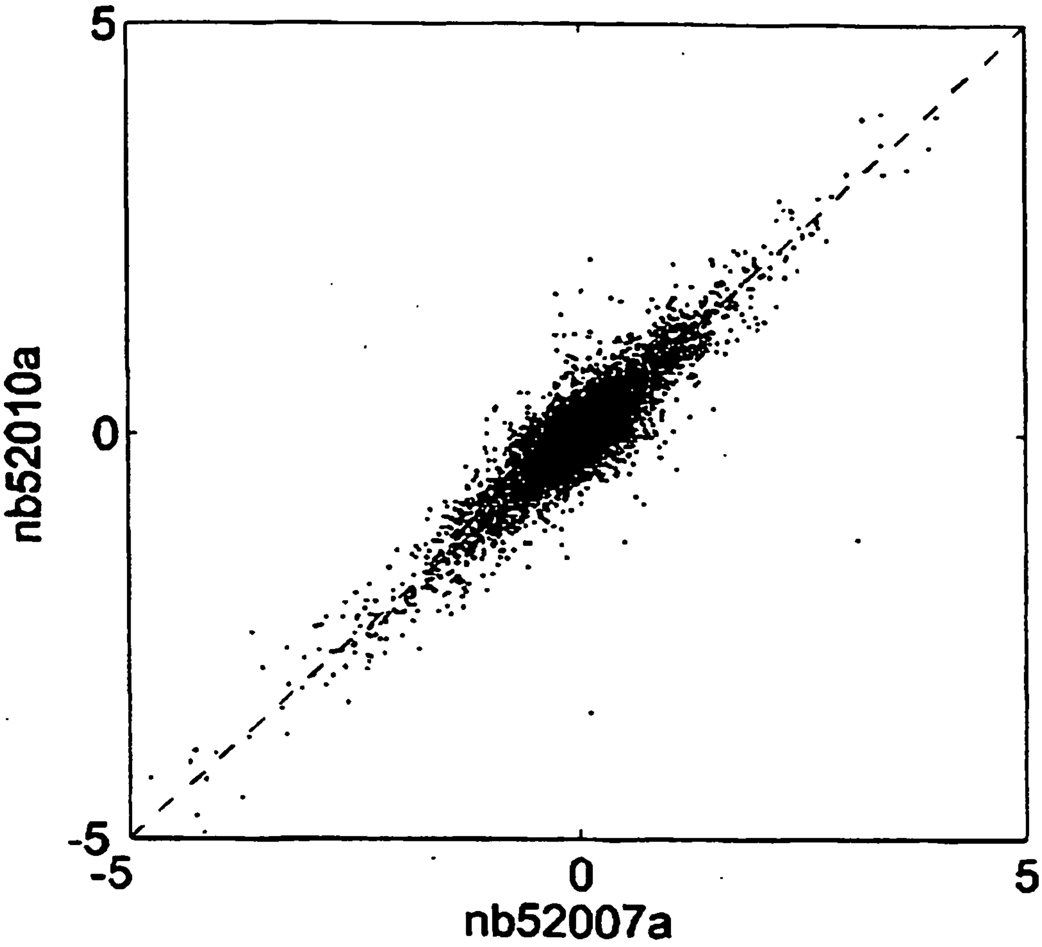

Fig. 1 3

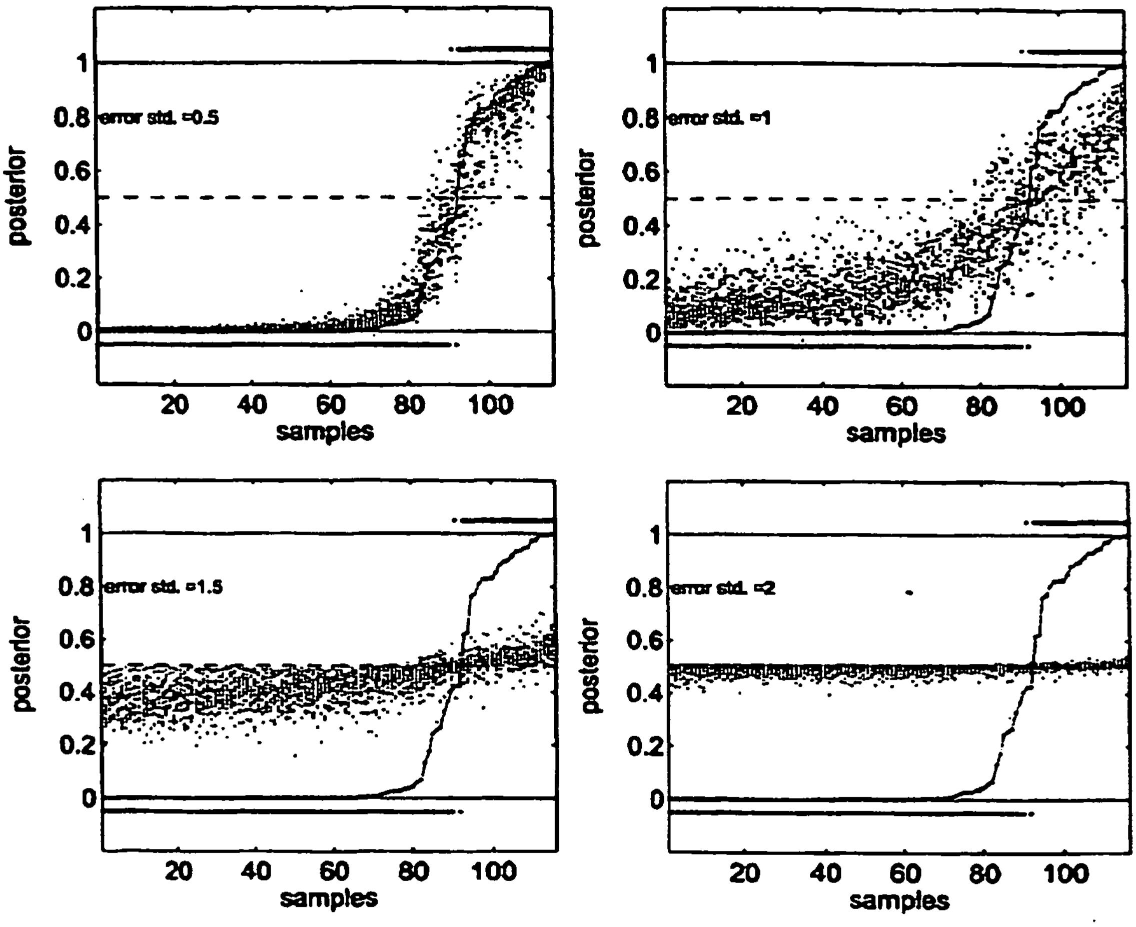
error std. =0.5
error std. =1
error std. =1.5
error std. =2
posterior
samples
0
0.2
0.4
0.6
0.8
1
20
40
60
80
100

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2001245671 A
- JP 2001321175 A
- JP 2004147563 A
- US 4458066 A
- JP 2001186880 A
- US 58075223 B
- JP 2001116750 A
- JP 2001186881 A
- WO 03038089 A1


### Non-patent literature cited in the description


- **Bolande, R. P.** *Hum Pathol,* 1974, vol. 5, 409-429
- **Evans, A. E. et al.** *Cancer,* 1971, vol. 27, 374-8
- **Brodeur, G. M. et al.** *J Clin Oncol,* 1993, vol. 11, 1466-77
- **Schwab, M. et al.** *Nature,* 1983, vol. 305, 245-8
- **Brodeur, G. M. et al.** *Science,* 1984, vol. 224, 1121-4
- **Look, A. T. et al.** *N Engl J Med,* 1984, vol. 311, 231-5
- **Look, A. T. et al.** *J Clin Oncol,* 1991, vol. 9, 581-91
- **Brodeur, G. M. et al.** *Prog Clin Biol Res,* 1988, vol. 271, 3-15
- **Nakagawara, A. et al.** *Cancer Res,* 1992, vol. 52, 1364-8
- **Nakagawara, A. et al.** *N Engl J Med,* 1993, vol. 328, 847-54
- **Hiyama, E. et al.** *Nat Med,* 1995, vol. 1, 249-55
- **Favrot, M. C. et al.** *N Engl J Med,* 1993, 329
- **Nakagawara, A. et al.** *Cancer Res,* 1995, vol. 55, 1792-7
- **Shimono, R. et al.** *Anticancer Res,* 2000, vol. 20, 917-23
- **Nagata, T. et al.** *J Surg Res,* 2000, vol. 92, 267-75
- **Berwanger, B. et al.** *Cancer Cell,* 2002, vol. 2, 377-86
- **Yamanaka, Y. et al.** *Int Oncol,* 2002, vol. 21, 803-7
- **Ueda, K.** *Kurume Med J,* 2001, vol. 48, 159-64
- **Ohira, M. et al.** *Oncogene,* 2003, vol. 22, 5526-36
- **Ohira, M. et al.** *Cancer Lett,* 2003, vol. 197, 63-8
- **J. Sambrook ; E. F. Fritsch ; T. Maniatis.** Molecular Cloning: A Laboratory Manual. Cold Spring Harbor Laboratory Press, 1989
- DNA Cloning. The Practical Approach Series. IRL Press, 1995, vol. 1-4
- **Ausubel, F. M. et al.** Current Protocols in Molecular Biology. John Wiley & Sons, 1995
- Zoku Seikagaku Jikken Koza 1, Idenshi Kenkyuho II. Tokyo Kagaku Dozin, 1986
- Shin Seikagaku Jikken Koza 2, Kakusan III (Kumikae DNA Gijutsu). Tokyo Kagaku Dozin, 1992
- Methods in Enzymology. Recombinant DNA. Academic Press, 1980, vol. 68
- Methods in Enzymology. Recombinant DNA. 1983, vol. 100
- Recombinant DNA. Recombinant DNA. Academic Press, vol. 101
- Methods in Enzymology. Recombinant DNA. 1987, vol. 153
- Recombinant DNA. vol. 154
- Recombinant DNA. Academic Press, 1987, vol. 155
- *Mol. Cell. Biol.,* 1982, vol. 2, 167-170
- *J. Gene,* 1983, vol. 25, 263-269
- *Gene,* 1994, vol. 150, 243-250
- **Carruthers.** *Cold Spring Harbor Symp. Quant. Biol.,* 1982, vol. 47, 411-418
- **Adams.** *J. Am. Chem. Soc.,* 1983, vol. 105, 661
- **Belousov.** *Nucleic Acid Res.,* 1997, vol. 25, 3440-3444
- **Frenkel.** *Free Radic. Biol. Med.,* 1995, vol. 19, 373-380
- **Blommers.** *Biochemistry,* 1994, vol. 33, 7886-7896
- **Narang.** *Meth. Enzymol.,* 1979, vol. 68, 90
- **Brown.** *Meth. Enzymol.,* 1979, vol. 68, 109
- **Beaucage.** *Tetra. Lett.,* 1981, vol. 22, 1859
- **Lamture, J.B. et al.** *Nucl. Acids Res.,* 1994, vol. 22, 2121-2125

- **Guo, Z. et al.** *Nucl. Acids Res.,* 1994, vol. 22, 5456-5465
- **Yershov, G. et al.** *Proc. Natl. Acad. Sci. USA,* 1996, vol. 94, 4913
- **Sosnowski, R.G. et al.** *Proc. Natl. Acad. Sci. USA,* 1997, vol. 94, 1119-1123
- **Kaneko, M. et al.** *Med. Pediatr Oncol,* 1998, vol. 31, 1-7
- **Sawada et al.** *Lancet,* 1984, vol. 2, 271-3
- **Chomczynski ; Sacchi.** *Anal Biochem,* 1987, vol. 162, 156-9
- **Takahashi, M. et al.** *Cancer Res,* 2002, vol. 62, 2203-9
- **Yoshikawa, T. et al.** *Biochem Biophys Res Commun,* 2000, vol. 275, 532-7
- **Quackenbush.** *J. Nat Genet,* 2002, vol. 32, 496-501
- **Oba, S. et al.** *Bioinformatics,* 2003
- **Bo, T ; Jonassen, I.** *Genome Biol,* 2002, vol. 3
- **MacKay. D. J. C.** *Neural Network and Machine Learning,* 1998, 133-165
- **van't Veer et al.** *Nature,* 2002, vol. 415, 530-6
- **Singh et al.** *Cancer Cell,* 2002, vol. 1, 203-9
- **Ye et al.** *Nat Med,* 2003, vol. 9, 416-23
- **Ambros, I. M. ; Ambros, P. F.** *Eur J Cancer,* 1995, vol. 4, 429-34
- **Ambros, I. M. ; Ambros, P. F.** *Neuroblastoma,* 2000, 229-243
- **Knoops, B. ; Octave, J. N.** *Neuroreport,* 1997, vol. 8, 795-8
- **Hall, J. L. ; Cowan, N. J.** *Nucleic Acid Res,* 1985, vol. 13, 207-23
- **Godbout, R. ; Squire, J.** *Proc Natl Acad Sci USA,* 1993, vol. 90, 7578-82
- **Noguchi, T. et al.** *Genes Chromosomes Cancer,* 1996, vol. 15, 129-33
- **Slavc, I. et al.** *Cancer Res,* 1990, vol. 50, 1459-63